# EUROPEAN PATENT APPLICATION

(11) **EP 3 305 919 A1**
(43) Date of publication of application: **11.04.2018**
(21) Application number: 17185133.0
(22) Date of filing: 09.06.2004
(51) Int. Cl.: C12Q 1/6886

(54) **DETECTION METHODS FOR DISORDERS OF THE LUNG**

(30) Priority: 10.06.2003 US 477218 P
(62) Divisional of application: 10184732.5
(71) Applicant: The Trustees of Boston University, Boston, MA 02215 (US)
(72) Inventor: BRODY, Jerome S., Boston, Massachusetts 02118 (US); SPIRA, Avrum, Newton, Massachusetts 02465 (US)
(74) Representative: Haseltine Lake LLP

(57) **Abstract**

The present invention is directed to prognostic and diagnostic methods to assess lung disease risk caused by airway pollutants by analyzing expression of one or more genes belonging to the airway transcriptome provided herein. Based on the finding of a so-called "field defect" affecting the airways, the invention further provides a minimally invasive sample procurement method in combination with the gene expression-based tools for the diagnosis and prognosis of diseases of the lung, particularly diagnosis and prognosis of lung cancer

## Description

### CROSS-REFERENCE TO RELATED APPLICATIONS

The present application claims the benefit under 35 U.S.C. §119(e) of U.S. provisional application No. 60/477,218, filed June 10, 2003, which application is herewith incorporated by reference in its entirety.

### GOVERNMENT SUPPORT

The invention was supported, in whole or in part, by grant ES00354 from the NIEHS , the Doris Duke Charitable foundation and by grant HL07035 from the National Institute of Health. The United States Government has certain rights in the invention.

### BACKGROUND OF THE INVENTION

Lung disorders represent a serious health problem in the modern society. For example, lung cancer claims more than 150,000 lives every year in the United States, exceeding the combined mortality from breast, prostate and colorectal cancers. Cigarette smoking is the most predominant cause of lung cancer. Presently, 25% of the U.S. population smokes, but only 10% to 15% of heavy smokers develop lung cancer. There are also other disorders associated with smoking such as emphysema. There are also health questions arising from people exposed to smokers, for example, second hand smoke. Former smokers remain at risk for developing such disorders including cancer and now constitute a large reservoir of new lung cancer cases. In addition to cigarette smoke, exposure to other air pollutants such as asbestos, and smog, pose a serious lung disease risk to individuals who have been exposed to such pollutants.

Approximately 85% of all subjects with lung cancer die within three years of diagnosis. Unfortunately survival rates have not changed substantially of the past several decades. This is largely because there are no effective methods for identifying smokers who are at highest risk for developing lung cancer and no effective tools for early diagnosis.

One major hurdle in developing an early detection screen for lung diseases, such as lung cancer, is that present methods for diagnosis are invasive and require removal of tissue from inside the lung. Moreover, while it appears that a subset of smokers are more susceptible to, for example, the carcinogenic effects of cigarette smoke and are more likely to develop lung cancer, the particular risk factors, and particularly genetic risk factors, for individuals have gone largely unidentified. Same applies to lung cancer associated with, for example, asbestos exposure.

### SUMMARY OF THE INVENTION

The present invention provides prognostic and diagnostic methods to assess lung disease risk caused by airway pollutants. The methods according to the present invention use a novel minimally invasive sample procurement method and gene expression-based tools for the diagnosis and prognosis of diseases of the lung, particularly diagnosis and prognosis of lung cancer.

We have shown that exposure of airways to pollutants such as cigarette smoke, causes a so-called "field defect", which refers to gene expression changes in all the epithelial cells lining the airways from mouth mucosal epithelial lining through the bronchial epithelial cell lining to the lungs. Because of this field defect, it is now possible to detect changes, for example, pre-malignant and malignant changes resulting in diseases of the lung using cell samples isolated from epithelial cells obtained not only from the lung biopsies but also from other, more accessible, parts of the airways including bronchial or mouth epithelial cell samples.

The invention is based on the finding that that there are different patterns of gene expression between, smokers and non-smokers. The genes involved can be grouped into clusters of related genes that are reacting to the irritants or pollutants. We have found unique sets of expressed genes or gene expression patterns associated with pre-malignancy in the lung and lung cancer in smokers and non-smokers. All of these expression patterns constitute expression signatures that indicate operability and pathways of cellular function that can be used to guide decisions regarding prognosis, diagnosis and possible therapy. Epithelial cell gene expression profiles obtained from relatively accessible sites can thus provide important prognostic, diagnostic, and therapeutic information which can be applied to diagnose and treat lung disorders.

We have found that cigarette smoking induces xenobiotic and redox regulating genes as well as several oncogenes, and decreases expression of several tumor suppressor genes and genes that regulate airway inflammation. We have identified a subset of smokers, who respond differently to cigarette smoke and appear thus to be predisposed, for example, to its carcinogenic effects, which permits us to screen for individuals at risks of developing lung diseases.

The invention is based on characterization of "airway transcriptomes" or a signature gene expression profiles of the airways and identification of changes in this transcriptome that are associated with epithelial exposure to pollutants, such as direct or indirect exposure to cigarette smoke, asbestos, and smog. These airway transcriptome gene expression profiles provide information on lung tissue function upon cessation from smoking, predisposition to lung cancer in non-smokers and smokers, and predisposition to other lung diseases. The airway transcriptome expression pattern can be obtained from a non-smoker, wherein deviations in the normal expression pattern are indicative of increased risk of lung diseases. The airway transcriptome expression pattern can also be obtained from a non-smoking subject exposed to air pollutants, wherein deviation in the expression pattern associated with normal response to the air pollutants is indicative of increased risk of developing lung disease.

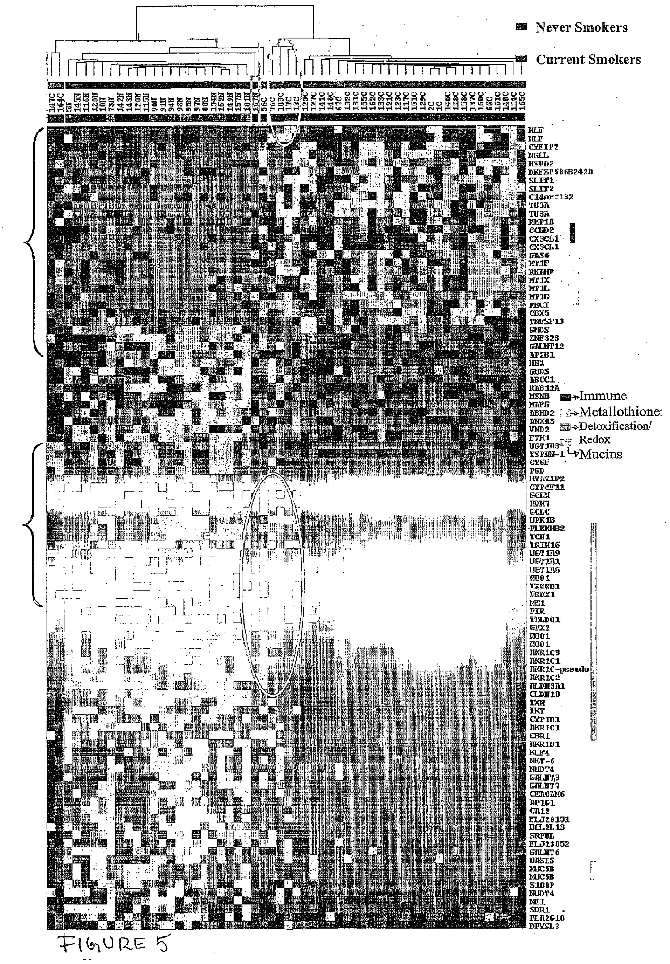

Accordingly, in one embodiment, the invention provides an "airway transcriptome" the expression pattern of which is useful in prognostic, diagnostic and therapeutic applications as described herein. We have discovered the expression of 85 genes, corresponding to 97 probesets on the affymetrix U133A Genechip array, having expression patterns that differs significantly between healthy smokers and healthy non-smokers. Examples of these expression patterns are shown in Figure 5. The expression patterns of the airway transcriptome are useful in prognosis of lung disease, diagnosis of lung disease and a periodical screening of the same individual to see if that individual has been exposed to risky airway pollutants such as cigarette smoke that change his/her expression pattern.

In one embodiment, the invention provides distinct airway "expression clusters", i.e., sub-transcriptomes, comprised of related genes among the 85 genes that can be quickly screened for diagnosis, prognosis or treatment purposes.

In one embodiment, the invention provides an airway sub-transcriptome comprising mucin genes of the airway transcriptome. Examples of mucin genes include muc 5 subtypes A, B, and C.

In another embodiment, the invention provides a sub-transcriptome comprising cell adhesion molecules of the airway trasncriptome, such as carcinoembryonic antigen-related adhesion molecule 6 and claudin 10 encoding genes.

In another embodiment, the invention provides a sub-transcriptome comprising detoxification related genes of the airway transcriptome. Examples of these genes include cytochrome P450 subfamily I (dioxin-inducible) encoding genes, NADPH dehydrogenase encoding genes. For example, upregulation of transcripts of cytochrome P450 subfamily I (dioxin-inducible) encoding genes

In yet another embodiment, the invention provides a sub-trasncriptome comprising immune system regulation associated genes of the airway transcriptome. Examples of immunoregulatory genes include small inducible cytokine subfamily D encoding genes.

In another embodiment, the invention provides a sub-transcriptome comprising metallothionein genes of the airway transcriptome. Examples of metallothionein genes include MTX G, X, and L encoding genes.

In another embodiment, the subtranscriptome comprises protooncogenes and oncogenes such as RAB11A and CEACAM6.
In another embodiment, the subtranscriptome includes tumor suppressor genes such as SLIT1, and SLIT2.

In one embodiment, the invention provides a lung cancer "diagnostic airway transcriptome" comprising 208 genes selected from the group consisting of group consisting of 208238_x_at-probeset; 216384_x_at-probeset; 217679_x_at-probeset; 216859_x_at-probeset; 211200_s_at- probeset; PDPK1; ADAM28; ACACB; ASMTL; ACVR2B; ADAT1; ALMS1; ANK3; ANK3; DARS; AFURS1; ATP8B1; ABCC1; BTF3; BRD4; CELSR2; CALM31 CAPZB; CAPZB1 CFLAR; CTSS; CD24; CBX3; C21orf106; C6orf111; C6orf62; CHC1; DCLRE1C; EML2; EMS1; EPHB6; EEF2; FGFR3; FLJ20288; FVT1; GGTLA4; GRP; GLUL; HDGF; Homo sapiens cDNA FLJ11452 fis, clone HEMBA1001435; Homo sapiens cDNA FLJ12005 fis, clone HEMBB1001565; Homo sapiens cDNA FLJ13721 fis, clone PLACE2000450; Homo sapiens cDNA FLJ14090 fis, clone MAMMA1000264; Homo sapiens cDNA FLJ14253 fis, clone OVARC1001376; Homo sapiens fetal thymus prothymosin alpha mRNA, complete cds Homo sapiens fetal thymus prothymosin alpha mRNA; Homo sapiens transcribed sequence with strong similarity to protein ref:NP_004726.1 (H.sapiens) leucine rich repeat (in FLII) interacting protein 1; Homo sapiens transcribed sequence with weak similarity to protein ref:NP_060312.1 (H.sapiens) hypothetical protein FLJ20489; Homo sapiens transcribed sequence with weak similarity to protein ref:NP_060312.1 (H.sapiens) hypothetical protein FLJ20489; 222282_at -probeset corresponding to Homo sapiens transcribed sequences; 215032_at - probeset corresponding to Homo sapiens transcribed sequences; 81811_at-probeset corresponding to Homo sapiens transcribed sequences; DKFZp547K1113; ET; FLJ10534; FLJ10743; FLJ13171; FLJ14639; FLJ14675; FLJ20195; FLJ20686; FLJ20700; CG005; CG005; MGC5384; IMP-2; INADL; INHBC; KIAA0379; KIAA0676; KIAA0779; KIAA1193; KTN1; KLF5; LRRFIP1; MKRN4; MAN1C1; MVK; MUC20; MPZL1; MYO1A; MRLC2; NFATC3; ODAG; PARVA; PASK; PIK3C2B; PGF; PKP4; PRKX; PRKY; PTPRF; PTMA; PTMA; PHTF2; RAB14; ARHGEF6; RIPX; REC8L1; RIOK3; SEMA3F; SRRM21 MGC709071 SMT3H2; SLC28A3; SAT; SFRS111 SOX2; THOC2; TRIM51 USP7; USP9X; USH1C; AF020591; ZNF131; ZNF160; ZNF264; 217414_x_at-probeset;; 217232_x_at-probeset;; ATF3; ASXL2; ARF4L; APG5L; ATP6V0B; BAG1; BTG2; COMT; CTSZ; CGI-128; C14orf87; CLDN3; CYR61; CKAP1; DAF; DAF; DSIPI; DKFZP564G2022; DNAJB9; DDOST; DUSP1; DUSP6; DKC1; EGR1; EIF4EL3; EXT2; GMPPB; GSN; GUK1; HSPA8; Homo sapiens PRO2275 mRNA, complete cds; Homo sapiens transcribed sequence with strong similarity to protein ref:NP_006442.2, polyadenylate binding protein-interacting protein 1; HAX1; DKFZP434K046; IMAGE3455200; HYOU1; IDN3; JUNB; KRT8; KIAA0100; KIAA0102; APH-1A; LSM4; MAGED2; MRPS7; MOCS2; MNDA; NDUFA8; NNT; NFIL3; PWP1; NR4A2; NUDT4; ORMDL2; PDAP2; PPIH; PBX3; P4HA2; PPP1R15A; PRG11 P2RX4; SUI1; SUI1; SUI1; RAB5C; ARHB; RNASE4; RNH; RNPC4; SEC23B; SERPINA1; SH3GLB1; SLC35B1; SOX9; SOX9; STCH; SDHC; TINF2; TCF8; E2-EPF; FOS; JUN; ZFP36; ZNF500; and ZDHHC4.

Accordingly, the invention provides methods of diagnosing lung cancer in an individual comprising taking a biological sample from the airways of the individual and analyzing the expression of at least 10 genes, preferably at least 50 genes, still more preferably at least 100 genes, still more preferably at least 150 genes, still more preferably at least 200 genes selected from genes of the diagnostic airway transcriptome, wherein deviation in the expression of at least one, preferably at least 5, 10, 20, 50, 100, 150, 200 genes as compared to a control group is indicative of lung cancer in the individual.

Deviation is preferably decrease of the transcription of at least one gene selected from the group consisting of of 208238_x_at -probeset; 216384_x_at--probeset; 217679_x_at-probeset; 216859_x_at-probeset; 211200_s_at- probeset;. PDPK1; ADAM28; ACACB; ASMTL; ACVR2B; ADAT1; ALMS1; ANK3; ANK3; DARS; AFURS1; ATP8B1; ABCC1; BTF3; BRD4; CELSR2; CALM31 CAPZB; CAPZB1 CFLAR; CTSS; CD24; CBX3; C21orf106; C6orf111; C6orf62; CHC1; DCLRE1C; EML2; EMS1; EPHB6; EEF2; FGFR3; FLJ20288; FVT1; GGTLA4; GRP; GLUL; HDGF; Homo sapiens cDNA FLJ11452 fis, clone HEMBA1001435; Homo sapiens cDNA FLJ12005 fis, clone HEMBB1001565; Homo sapiens cDNA FLJ13721 fis, clone PLACE2000450; Homo sapiens cDNA FLJ14090 fis, clone MAMMA1000264; Homo sapiens cDNA FLJ14253 fis, clone OVARC1001376; Homo sapiens fetal thymus prothymosin alpha mRNA, complete cds; Homo sapiens transcribed sequence with strong similarity to protein ref:NP_004726.1 (H.sapiens) leucine rich repeat (in FLII) interacting protein 1; Homo sapiens transcribed sequence with weak similarity to protein ref:NP_060312.1 (H.sapiens) hypothetical protein FLJ20489; Homo sapiens transcribed sequence with weak similarity to protein ref:NP_060312.1 (H.sapiens) hypothetical protein FLJ20489; 222282_at-probeset corresponding to Homo sapiens transcribed sequences; 215032_at-probeset corresponding to Homo sapiens transcribed sequences; 81811_at-probeset corresponding to Homo sapiens transcribed sequences; DKFZp547K1113; ET; FLJ10534; FLJ10743; FLJ13171; FLJ14639; FLJ14675; FLJ20195; FLJ20686; FLJ20700; CG005; CG005; MGC5384; IMP-2; INADL; INHBC; KIAA0379; KIAA0676; KIAA0779; KIAA1193; KTN1; KLF5; LRRFIP1; MKRN4; MAN1C1; MVK; MUC20; MPZL1; MYO1A; MRLC2; NFATC3; ODAG; PARVA; PASK; PIK3C2B; PGF; PKP4; PRKX; PRKY; PTPRF; PTMA; PTMA; PHTF2; RAB14; ARHGEF6; RIPX; REC8L1; RIOK3; SEMA3F; SRRM21 MGC709071 SMT3H2; SLC28A3; SAT; SFRS111 SOX2; THOC2; TRIM51 USP7; USP9X; USH1C; AF020591; ZNF131; ZNF160; andZNF264 genes.

Deviation is preferably increase of the expression of at least one gene selected from the group consisting of of 217414_x_at-probeset;; 217232_x_at- probeset;; ATF3; ASXL2; ARF4L; APG5L; ATP6V0B; BAG1; BTG2; COMT; CTSZ; CGI-128; C14orf87; CLDN3; CYR61; CKAP1; DAF; DAF; DSIPI; DKFZP564G2022; DNAJB9; DDOST; DUSP1; DUSP6; DKC1; EGR1; EIF4EL3; EXT2; GMPPB; GSN; GUK1; HSPA8; Homo sapiens PRO2275 mRNA, complete cds; Homo sapiens transcribed sequence with strong similarity to protein ref:NP_006442.2, polyadenylate binding protein-interacting protein 1; HAX 1; DKFZP434K046; IMAGE3455200; HYOU1; IDN3; JUNB; KRT8; KIAA0100; KIAA0102; APH-1A; LSM4; MAGED2; MRPS7; MOCS2; MNDA; NDUFA8; NNT; NFIL3; PWP1; NR4A2; NUDT4; ORMDL2; PDAP2; PPIH; PBX3; P4HA2; PPP1R15A; PRG11 P2RX4; SUI1; SUI1; SUI1; RAB5C; ARHB; RNASE4; RNH; RNPC4; SEC23B; SERPINA1; SH3GLB1; SLC35B1; SOX9; SOX9; STCH; SDHC; TINF2; TCF8; E2-EPF; FOS; JUN; ZFP36; ZNF500; and ZDHHC4 genes.

The genes are referred to using their HUGO names or alternatively the probeset number on Affymetrix (Affymetrix, Inc. (U.S.), Santa Clara, CA) probesets.

In one embodiment, the invention provides methods of prognosis and diagnosis of lung diseases comprising obtaining a biological sample from a subject's airways, analyzing the level of expression of at least one gene of the airway transcriptome, comparing the level of expression of the at least one gene of at least one of the airway transcriptome to the level of expression in a control, wherein deviation in the level of expression in the sample from the control is indicative of increased risk of lung disease.

Preferably the analysis is performed using expression of at least two genes of the airway transcriptome, more preferably at least three genes, still more preferably at least four to 10 genes, still more preferably at least 10-20 genes, still more preferably at least 20-30, still more preferably at least 30-40, still more preferably at least 40-50, still more preferably at least 50-60, still more preferably at least 60-70, still more preferably at least 70-85 genes is analyzed.

In one preferred embodiment, the expression level of the genes of one or more of the sub-transcriptomes is analyzed. Preferably, gene expression of one or more genes belonging to at least two different sub-transcriptome sets is analyzed. Still more preferably, gene expression of at least one gene from at least three sub-transcriptome sets is analyzed. Still more preferably, gene expression of at least one gene from at least four sub-transcriptome sets is analyzed. Still more preferably, gene expression of at least one gene from at least five sub-transcriptome sets is analyzed.

The expression analysis according to the methods of the present invention can be performed using nucleic acids, particularly RNA, DNA or protein analysis.

The, cell samples are preferably obtained from bronchial airways using, for example, endoscopic cytobrush in connection with a fiberoptic bronchoscopy. In one preferred embodiment, the cells are obtained from the individual's mouth buccal cells, using, for example, a scraping of the buccal mucosa.
In one preferred embodiment, the invention provides a prognostic and/or diagnostic immunohistochemical approach, such as a dip-stick analysis, to determine risk of developing lung disease. Antibodies against at least one, preferably more proteins encoded by the genes of the airway transcriptome are either commercially available or can be produced using methods well know to one skilled in the art.

The invention further provides an airway transcriptone expression pattern of genes that correlate with time since cigarette discontinuance in former smokers, i.e., the expression of these genes in a healthy smoker returns to normal, or healthy non-smoker levels, after about two years from quitting smoking. These genes include: MAGF, GCLC, UTG1A10, SLIT2, PECI, SLIT1, and TNFSF13. If the transcription of these genes has not returned to the level of a healthy non-smoker, as measured using the methods of the present invention, within a time period of about 1-5 years, preferably about 1.5-2.5 years, the individual with a remaining abnormal expression is at increased risk of developing a lung disease.

The invention further provides an airway transcriptome expression pattern of genes the expression of which remains abnormal after cessation from smoking. These genes include: CX3CL1, RNAHP, MT1X, MT1L, TU3A, HLF, CYFIP2, PLA2G10, HN1, GMDS. PLEKHB2, CEACAM6, ME1, and DPYSL3.

Accordingly, the invention provides methods for prognosis, diagnosis and therapy designs for lung diseases comprising obtaining an airway sample from an individual who smokes and analyzing expression of at least one, preferably at least two, more preferably at least three, still more preferably at least four, still more preferably at least five, still more preferably at least six, seven, eight, and still more preferably at least nine genes of the normal airway transcriptome, wherein an expression pattern of the gene or genes that deviates from that in a healthy age, race, and gender matched smoker, is indicative of an increased risk of developing a lung disease.

The invention also provides methods for prognosis, diagnosis and therapy designs for lung diseases comprising obtaining an airway sample from a non-smoker individual and analyzing expression of at least one, preferably at least two, more preferably at least three, still more preferably at least four, still more preferably at least five, still more preferably at least six, seven, eight, and still more preferably at least nine genes of the normal airway transcriptome, wherein an expression pattern of the gene or genes that deviates from that in a healthy age, race, and gender matched non-smoker, is indicative of an increased risk of developing a lung disease. Non-smoking individual whose expression pattern begins to resemble that of a smoker and at increased risk of developing a lung disease.

In one embodiment, the analysis is performed from a biological sample obtained from bronchial airways.

In one embodiment, the analysis is performed from a biological sample obtained from buccal mucosa.

In one embodiment, the analysis is performed using nucleic acids, preferably RNA, in the biological sample.

In one embodiment, the analysis is performed analyzing the amount of proteins encoded by the genes of the airway transcriptome present in the sample.

In one embodiment the analysis is performed uning DNA by analyzing the gene expression regulatory regions of the airway transcriptome genes using nucleic acid polymorphisms, such as single nucleic acid polymorphisms or SNPs, wherein polymorphisms known to be associated with increased or decreased expression are used to indicate increased or decreased gene expression in the individual.

In one embodiment, the present invention provides a minimally invasive sample procurement method for obtaining airway epithelial cell RNA that can be analyzed by expression profiling, for example, by array-based gene expression profiling. These methods can be used to determine if airway epithelial cell gene expression profiles are affected by cigarette smoke and if these profiles differ in smokers with and without lung cancer. These methods can also be used to identify patterns of gene expression that are diagnostic of lung disorders/diseases, for example, cancer or emphysema, and to identify subjects at risk for developing lung disorders. All or a subset of the genes identified according to the methods described herein can be used to design an array, for example, a microarray, specifically intended for the diagnosis or prediction of lung disorders or susceptibility to lung disorders. The efficacy of such custom-designed arrays can be further tested, for example, in a large clinical trial of smokers.

In one embodiment, the invention relates to a method of diagnosing a disease or disorder of the lung comprising obtaining a sample, nucleic acid or protein sample, from an individual to be diagnosed; and determining the expression of one or more of the 85 identified genes in said sample, wherein changed expression of such gene compared to the expression pattern of the same gene in a healthy individual with similar life style and environment is indicative of the individual having a disease of the lung.

In one embodiment, the invention relates to a method of diagnosing a disease or disorder of the lung comprising obtaining at least two samples, nucleic acid or protein samples, in at least one time interval from an individual to be diagnosed; and determining the expression of one or more of the 85 identified genes in said samples, wherein changed expression of such gene or genes in the sample taken later in time compared to the sample taken earlier in time is diagnostic of a lung disease.

In one embodiment, the disease of the lung is selected from the group consisting of asthma, chronic bronchitis, emphysema, primary pulmonary hypertension, acute respiratory distress syndrome, hypersensitivity pneumonitis, eosinophilic pneumonia, persistent fungal infection, pulmonary fibrosis, systemic sclerosis, ideopathic pulmonary hemosiderosis, pulmonary alveolar proteinosis, and lung cancer, such as adenocarcinoma, squamous cell carcinoma, small cell carcinoma, large cell carcinoma, and benign neoplasms of the lung (e.g., bronchial adenomas and hamartomas). In a particular embodiment, the nucleic acid sample is RNA. In a preferred embodiment, the nucleic acid sample is obtained from an airway epithelial cell. In one embodiment, the airway epithelial cell is obtained from a bronchoscopy or buccal mucosal scraping. In one embodiment, individual to be diagnosed is an individual who has been exposed to tobacco smoke, an individual who has smoked, or an individual who smokes.

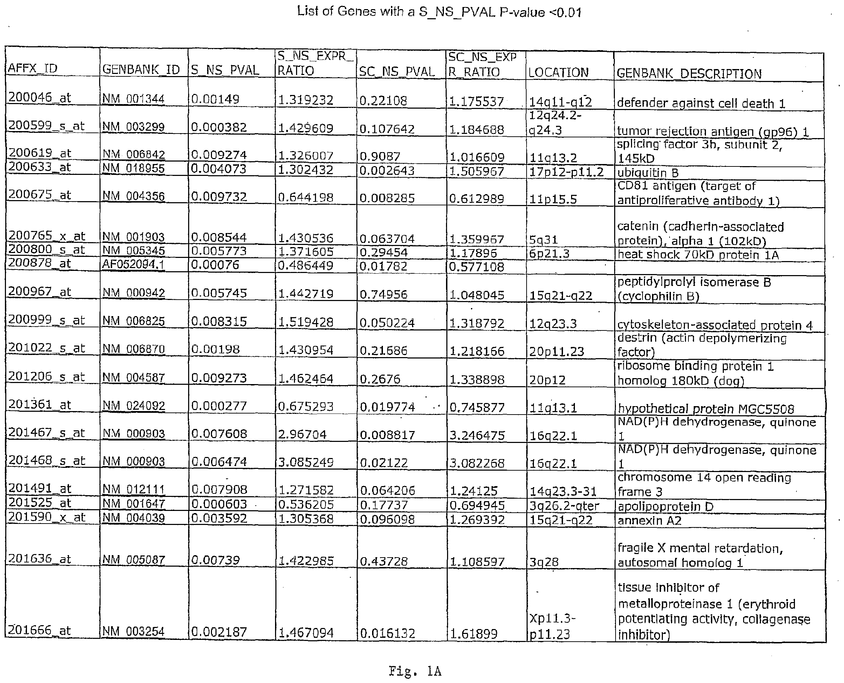

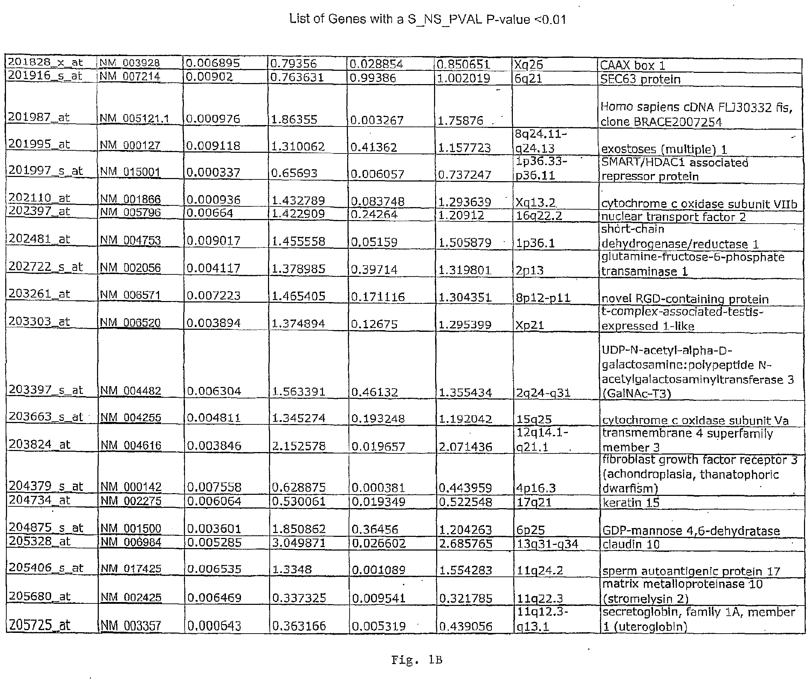

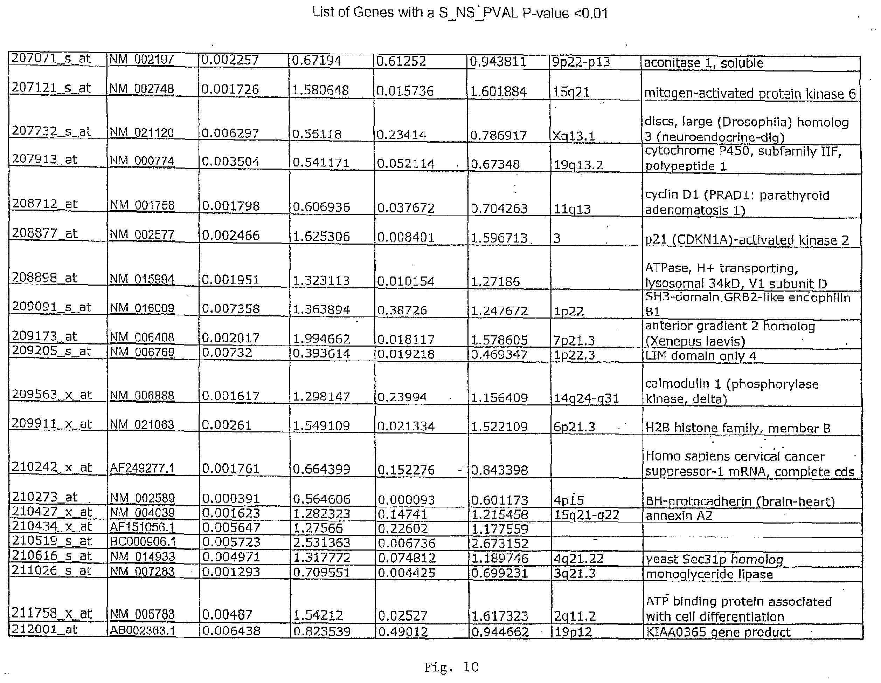

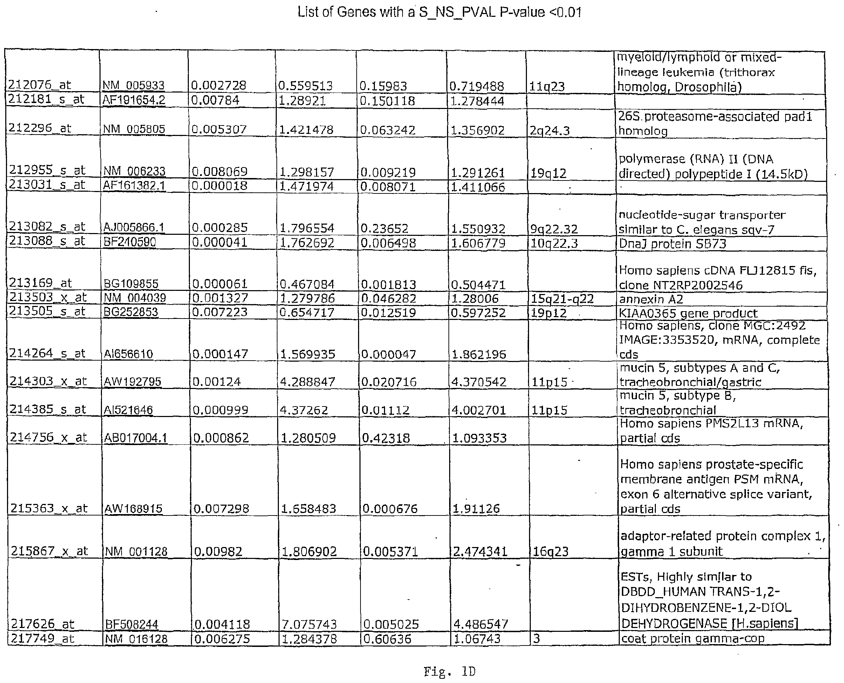

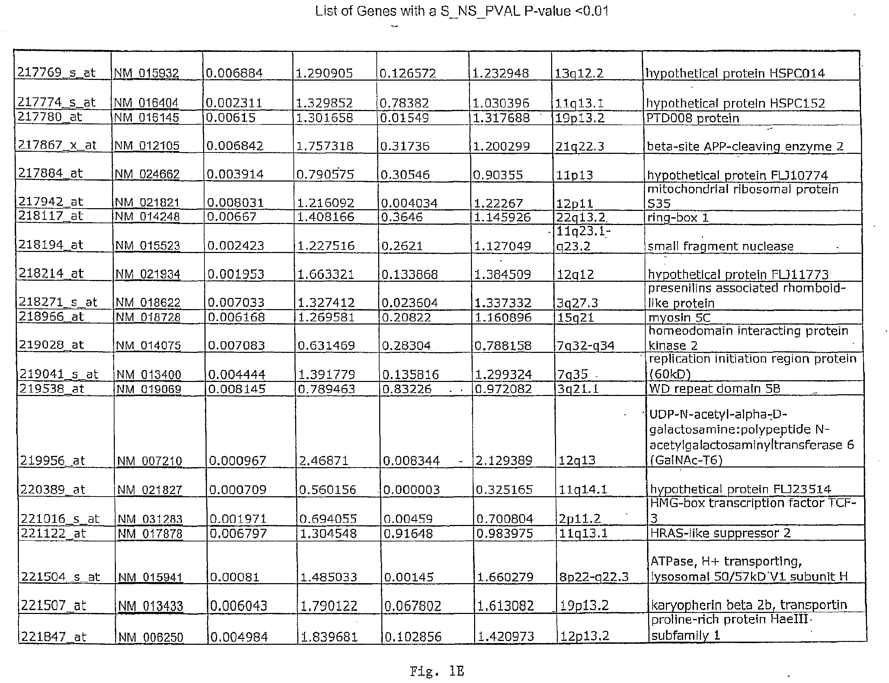

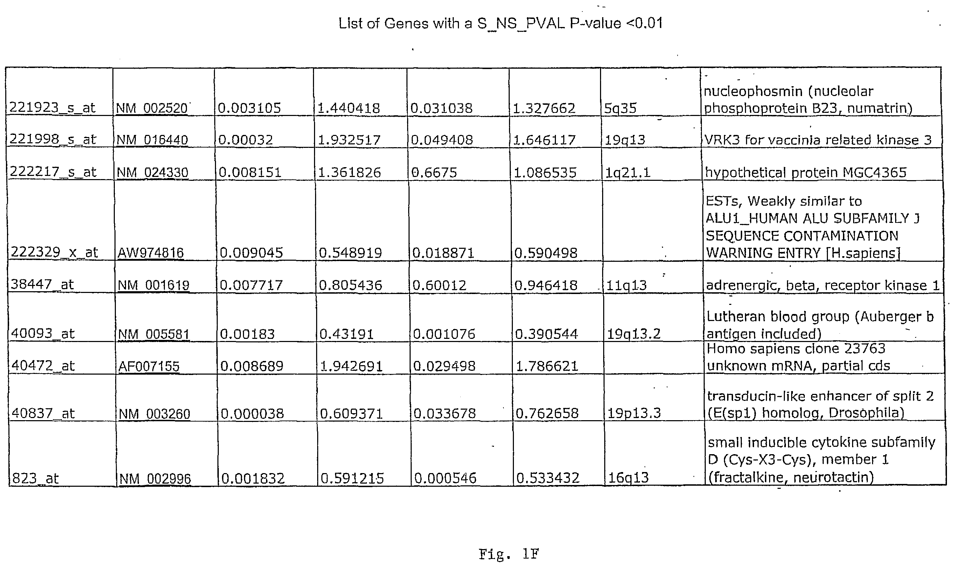

In a preferred embodiment of the method, the genes are selected from the group consisting of the genes shown in Figures 1A-1F; 2A-2B; and Figure 5. Preferably the expression of two or more, five or more, ten or more, fifteen or more, twenty or more, fifty or more or one hundred or more informative genes is determined. In a preferred embodiment, the expression is determined using a microarry having one or more oligonucleotides (probes) for said one or more genes immobilized thereon.

The invention further relates to a method of obtaining a nucleic acid sample for use in expression analysis for a disease of the lung comprising obtaining an airway epithelial cell sample from an individual; and rendering the nucleic acid molecules in said cell sample available for hybridization.
The invention also relates to a method of treating a disease of the lung comprising administering to an individual in need thereof an effective amount of an agent which increases the expression of a gene whose expression is decreased in said individual as compared with a normal individual.

The invention further relates to a method of treating a disease of the lung comprising administering to an individual in need thereof an effective amount of an agent, which changes the expression of a gene to that expression level seen in a healthy individual having the similar life style and environment, and a pharmaceutically acceptable carrier.

The invention also relates to a method of treating a disease of the lung comprising administering to an individual in need thereof an effective amount of an agent which increases the activity of an expression product of such gene whose activity is decreased in said individual as compared with a normal individual.

The invention also relates to a method of treating a disease of the lung comprising administering to an individual in need thereof an effective amount of an agent which decreases the activity of an expression product of such gene whose activity is increased in said individual as compared with a normal individual.

The invention also provides an array, for example, a microarray for diagnosis of a disease of the lung having immobilized thereon a plurality of oligonucleotides which hybridize specifically to one or more genes which are differentially expressed in airways exposed to air pollutants, such as cigarette smoke, and airways which are not exposed to such pollutants. In one embodiment, the oligonucleotides hybridize specifically to one allelic form of one or more genes which are differentially expressed for a disease of the lung. In a particular embodiment, the differentially expressed genes are selected from the group consisting of the genes shown in Figures 1A-1F, 2A-2B and Figure 5.

The prognostic and diagnostic methods of the present invention are based on the finding that deviation from the normal expression pattern in the airway transcriptome is indicative of abnormal response of the airway cells and thus predisposes the subject to diseases of the lung. Therefore, all the comparisons as provided in the methods are performed against a normal airway transcriptome of a "normal" or "healthy" individual exposed to the pollutant, as provided by this invention. Examples of these normal expression patterns of the genes belonging to the airway transcriptome of the present invention are provided in Figure 5.

In one embodiment, the invention provides a prognostic method for lung diseases comprising detecting gene expression changes in the cell adhesion regulating genes of the airway transcriptome, wherein decrease in the expression compared with a "normal" smoker expression pattern is indicative of an increased risk of developing a lung disease. Examples of cell adhesion regulation related genes include carcinoembryonic antigen-related adhesion molecule 6 and claudin 10 encoding genes. For example, an about at least 2-20 fold, preferably about at least 3 fold, still more preferably at least about 4 fold, still more preferably about at least 5 fold decrease in expression of carcinoembryonic antigen-related adhesion molecule 6 encoding gene is indicative of an increased risk of developing a lung disease. Also, for example, an about 2-20, preferably at least about, 3 fold, still more preferably at least about 4 fold, still more preferably at least about 5 fold decrease in the transcript level of claudin 10 encoding gene is indicative of an increased risk of developing a lung disease.

In one embodiment, the invention provides a prognostic method for lung diseases comprising detecting gene expression changes in the detoxification related genes of the airway transcriptome, wherein decrease in the expression compared with a "normal" smoker expression pattern is indicative of an increased risk of developing a lung disease. Examples of these genes include cytochrome P450 subfamily I (dioxin-inducible) encoding genes, NADPH dehydrogenase encoding genes. For example, upregulation of transcripts of cytochrome P450 subfamily I (dioxin-inducible) encoding genes of about 2-50 fold, preferably at least about, 5 fold, still more preferably about 10 fold, still more preferably at least about 15 fold, still more preferably at least about 20 fold, still more preferably at least about 30 fold, and downregulation of transcription of NADPH dehydrogenase encoding genes of about 2-20, preferably about at least 3 fold, still more preferably at least about 4 fold, still more preferably about at least 5 fold decrease compared to expression in a "normal" smoker is indicative of an increased risk of developing a lung disease.

In one embodiment, the invention provides a prognostic method for lung diseases comprising detecting gene expression changes in the immune system regulation associated genes of the airway transcriptome, wherein increase in the expression compared with a "normal" smoker expression pattern is indicative of an increased risk of developing a lung disease. Examples of immunoregulatory genes include small inducible cytokine subfamily D encoding genes. For example, about 1-10 fold difference in the expression of cytokine subfamily D encoding genes is indicative of increased risk of developing lung disease. Preferably, the difference in expression is least about 2 fold preferably about at least 3 fold, still more preferably at least about 4 fold, still more preferably about at least 5 fold decrease decrease in the expression of small inducible cytokine subfamily D encoding genes is indicative of an increased risk of developing a lung disease.

In one embodiment, the invention provides a prognostic method for lung diseases comprising detecting gene expression changes in the metalothionein regulation associated genes of the airway transcriptome, wherein decrease in the expression compared with a "normal" smoker is indicative of an increased risk of developing a lung disease. Examples of metalothionein regulation associated genes include MTX G, X, and L encoding genes. At least about 1.5-10 fold difference in the expression of these genes in indicative of increased risk of developing lung disease. For example, at least about 1.5 fold, still more preferably at least about 2 fold, still more preferably at least about 2.5 fold, still more preferably at least about 3 fold, still more preferably at least about 4 fold, still more preferably about at least 5 fold increase in the expression of metalothionein regulation associated genes include MTX G, X, and L encoding genes indicative of an increased risk of developing a lung disease.

### BRIEF DESCRIPTION OF THE DRAWINGS

Figures 1A-1F show a list of genes which are differentially expressed in smokers and non-smokers. T-test statistical results are shown.

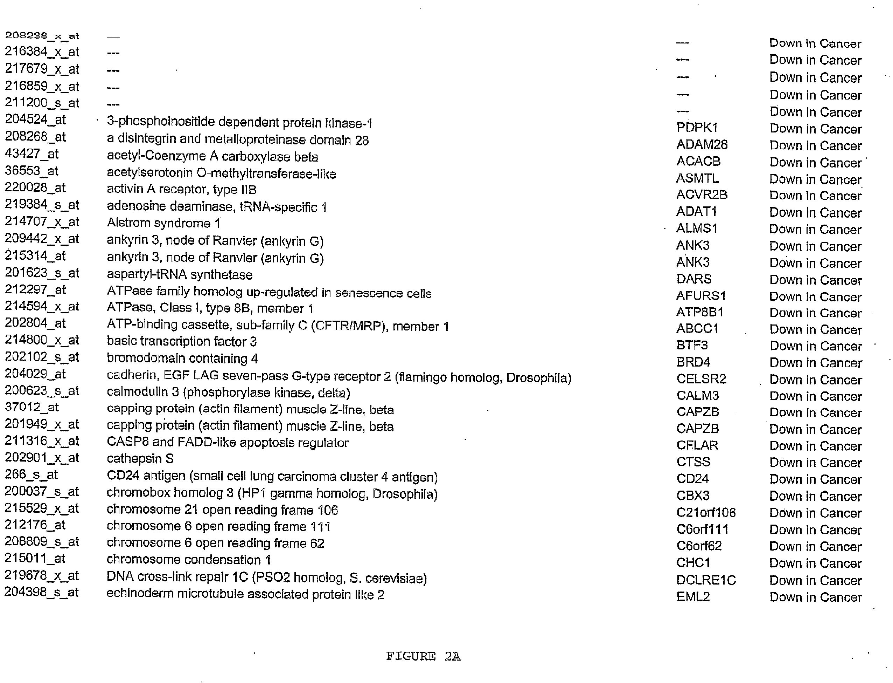

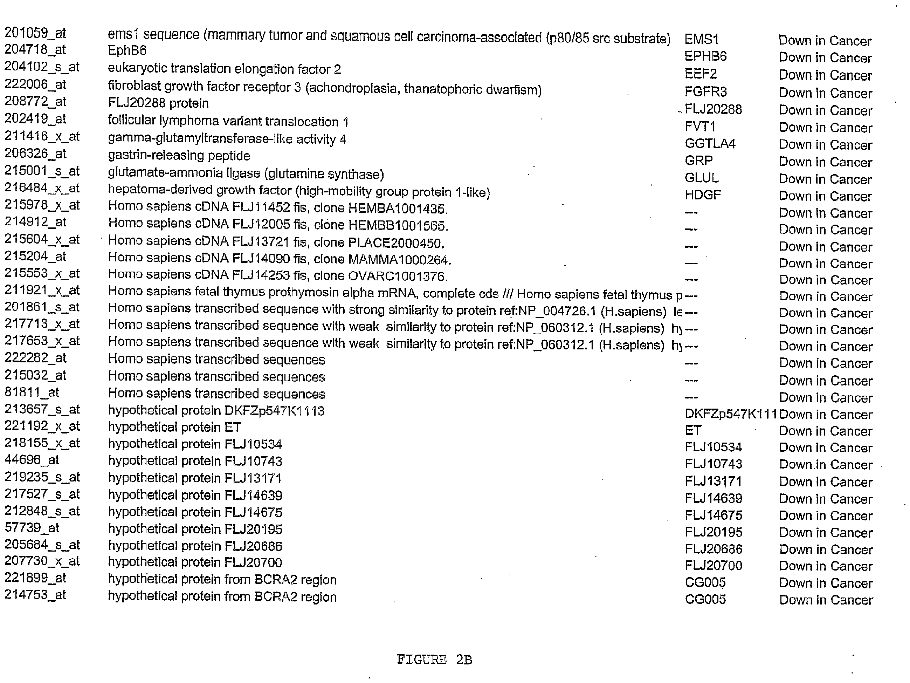

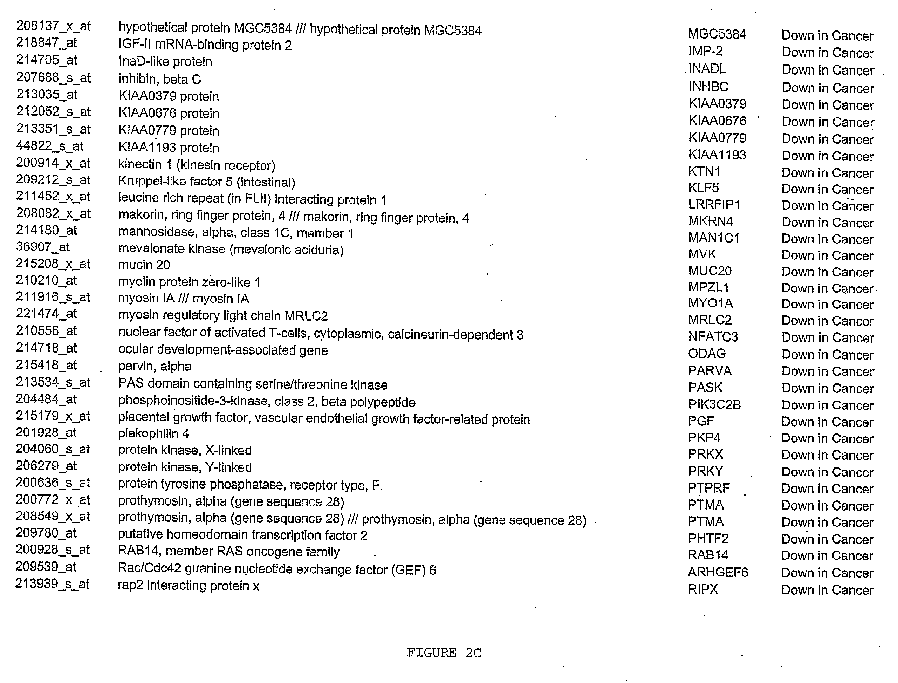

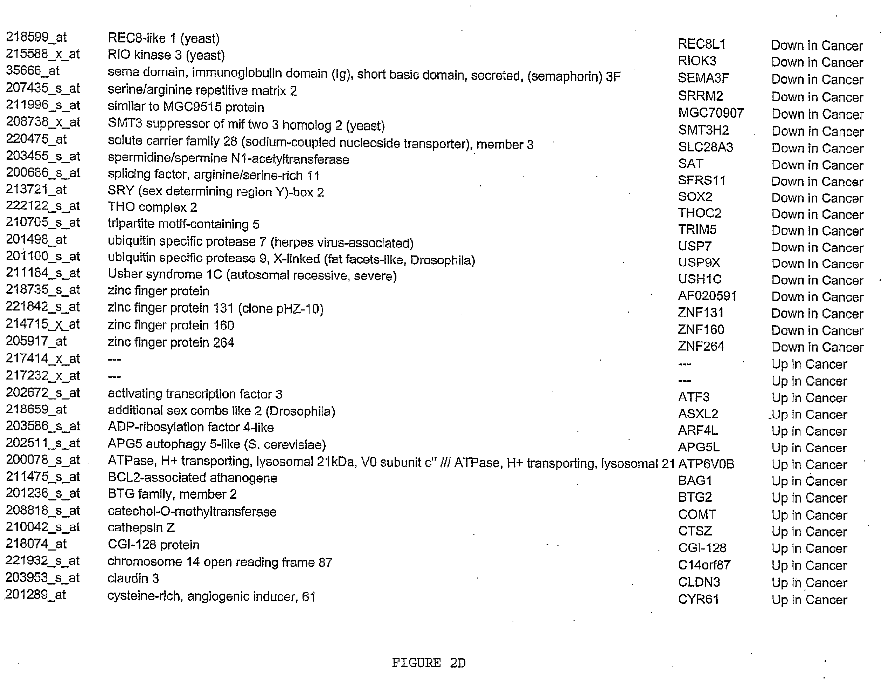

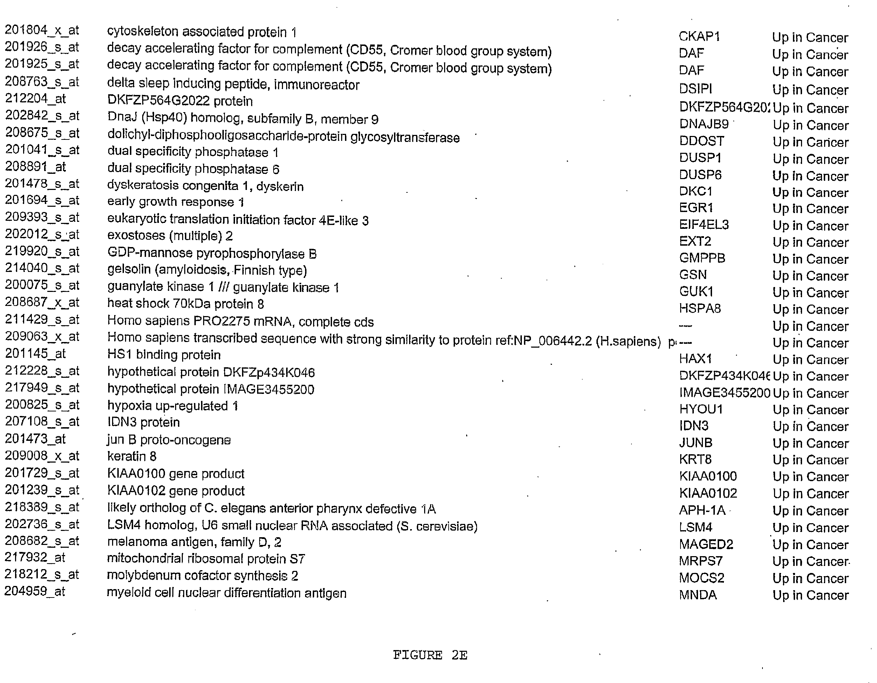

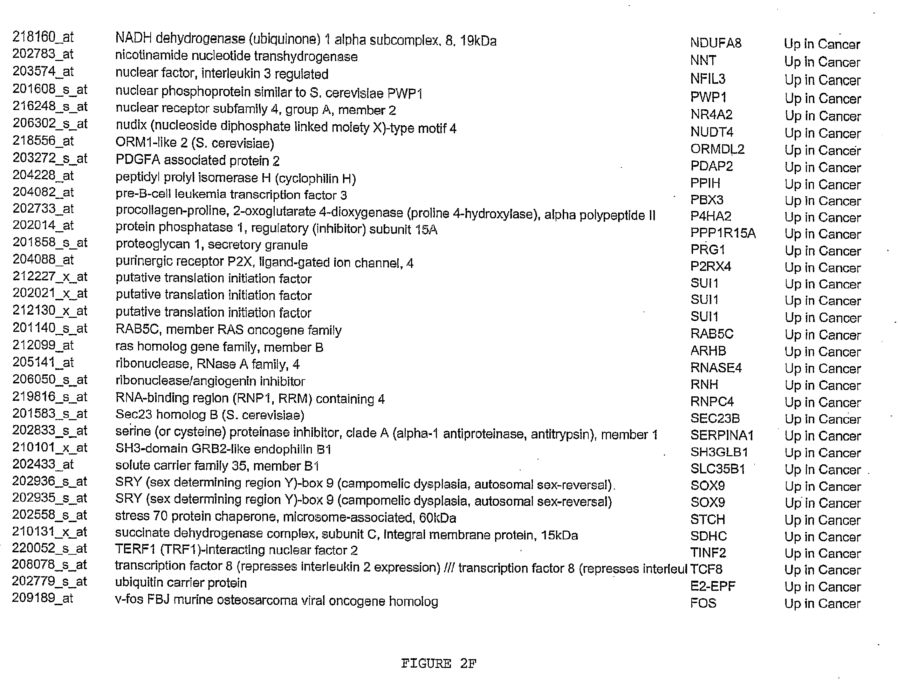

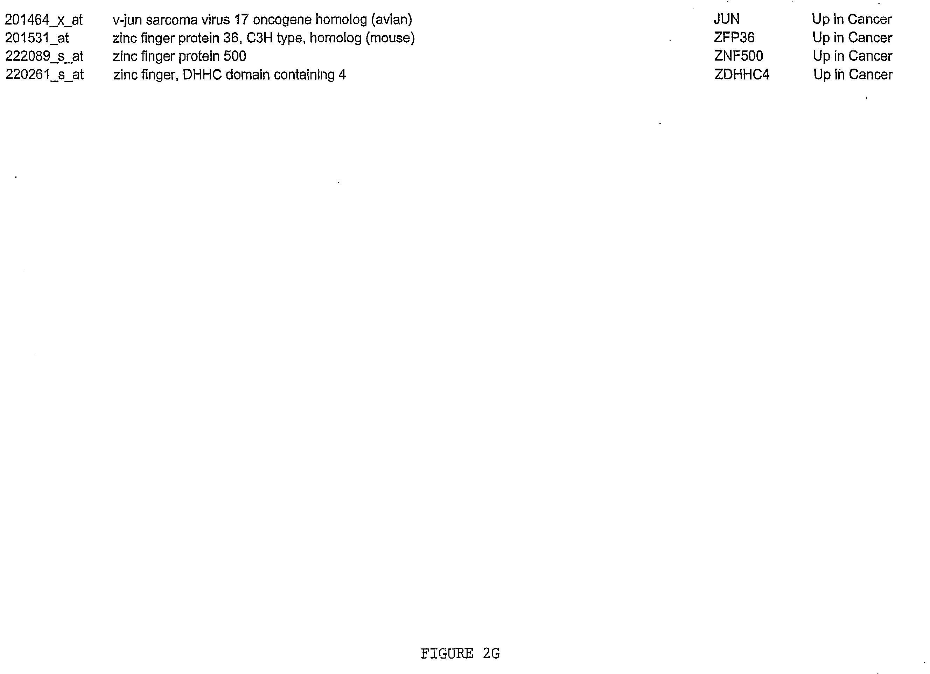

Figures 2A-2G show a list of genes which are differentially expressed in smokers and smokers with lung cancer. T-test statistical results are shown.

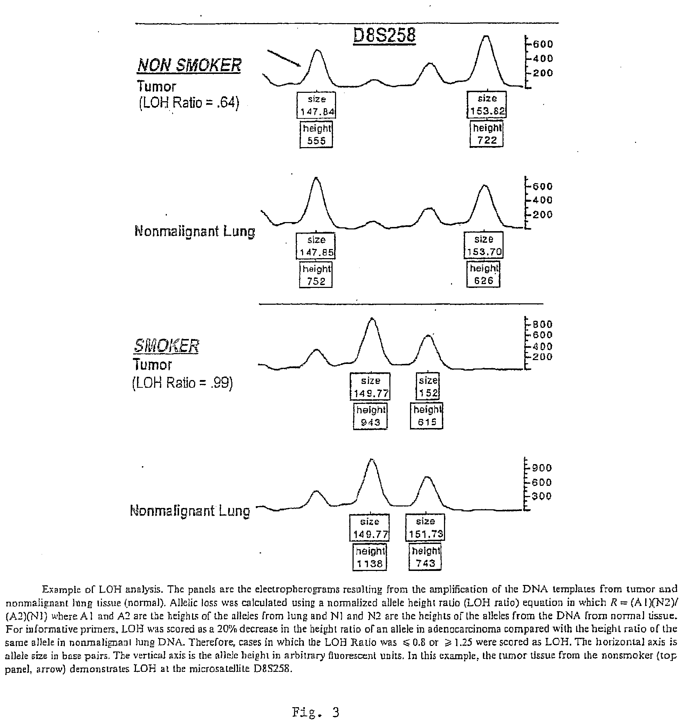

Figures 3 is a schematic diagram showing an example of loss of heterozygosity analysis.

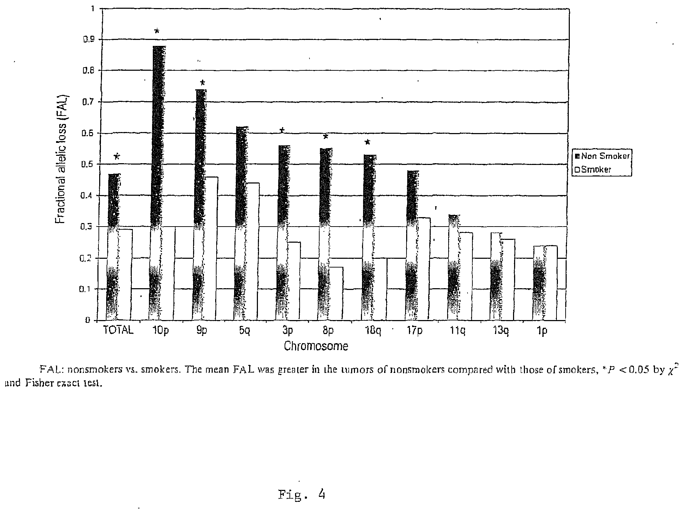

Figures 4 is a graph showing fractional allelic loss in smokers and non-smokers.
Figure 5 shows clustering of current and never smoker samples. Hierarchical clustering of current (n=34) and never (n=23) smokers according to the expression of the 97 probesets representing the 85 genes differentially expressed between current and never smokers. While current and never smokers separate into 2 groups, three current smokers appear to cluster with never smokers (rectangle). Expression of a number of redox-related and xenobiotic genes in these subjects was not increased (brackets) and therefore resembled that of never smokers despite substantial smoke exposure. There was also a subset of current smokers (circled individuals on x-axis) who did not upregulate expression of a number of predominantly redox/xenobiotic genes (circled expression analysis in the middle of the graph) to the same degree as other smokers. In addition, there is a never smoker, 167N (box), who is an outlier among never smokers and expresses a subset of genes at the level of current smokers. HUGO gene ID listed for all 85 genes. Functional classification of select genes is shown. Darker gray=high level of expression, lighter grey=low level of expression, black=mean level of expression.

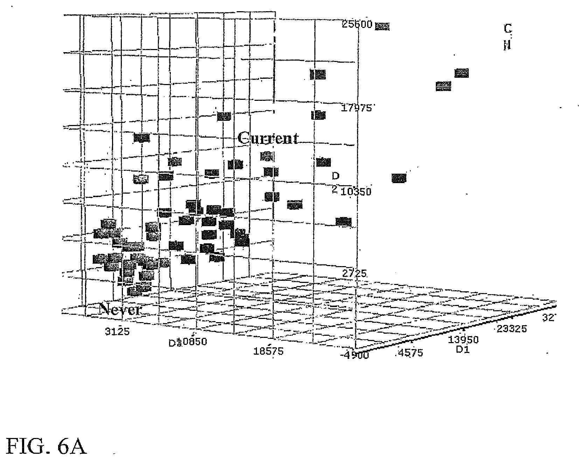

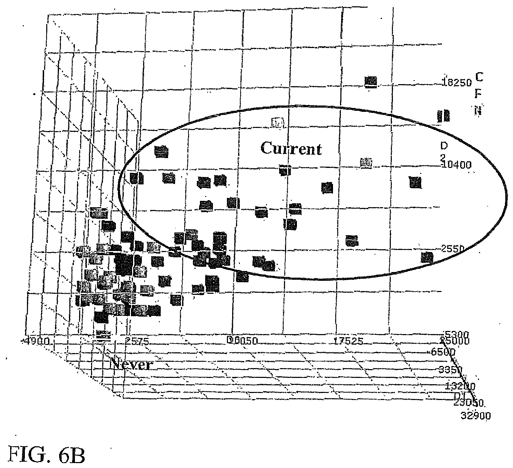

Figures 6A-6B show a multidimensional scaling plot of current, never, and former smoker samples. Multidimensional scaling plot of current (lighter grey boxes), never (medium grey boxes, mainly clustered on the left hand side of the graph) and former smokers (darkest grey boxes) in 97 dimensional space according to the expression of the 97 probesets reflecting the 85 differentially expressed genes between current and never smokers. Figure 6A illustrates that current and never smokers separate into their 2 classes according to the expression of these genes. Figure 6B shows that when former smokers are plotted according to the expression of these genes, a majority of former smokers appear to group more closely to never smokers. There are, however, a number of former smokers who group more closely to current smokers (black circle). The only clinical variable that differed between the 2 groups of former smokers was length of smoking cessation (p<.05), with formers smokers who quit within 2 years clustering with current smokers. The MDS plots are reduced dimension representations of the data and the axes on the figure have no units.

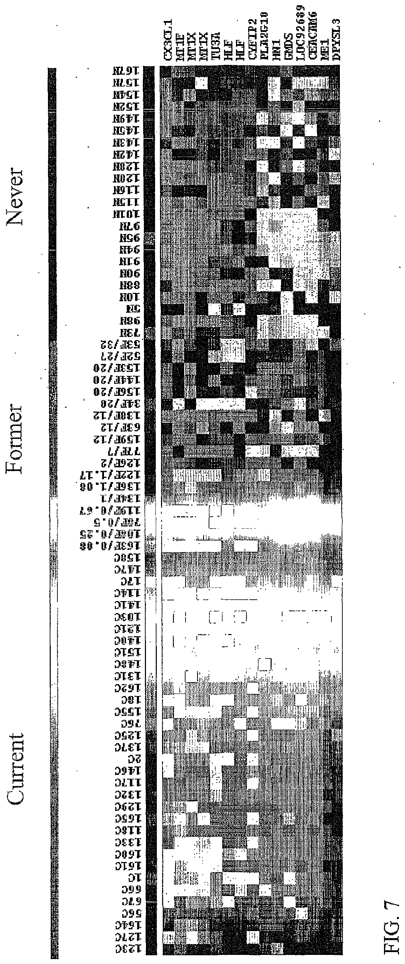

Figure 7 shows genes expression of which is irreversibly altered by cigarette smoke. Hierarchical clustering plot of 15 of the 97 probesets representing the 85 genes from Figure 5 that remain differentially expressed between former vs. never smokers (p<0.0001) as long as 30 years after cessation of smoking. Samples are grouped according to smoking status and length of smoking cessation (samples are not being clustered and thus there is no dendogram on the sample axis). Patient ID, status (C, F or N) and length of time since smoking cessation are shown for each sample. Current = current smokers, former = former smokers and never = never smokers. HUGO gene ID listed for all 15 genes. Two genes (HLF and MT1X) appear twice in the analysis (i.e. two different probe sets corresponding to the same gene). Darker grey shades indicate higher level of expression, lighter colors indicate low level of expression, black= mean level of expression.

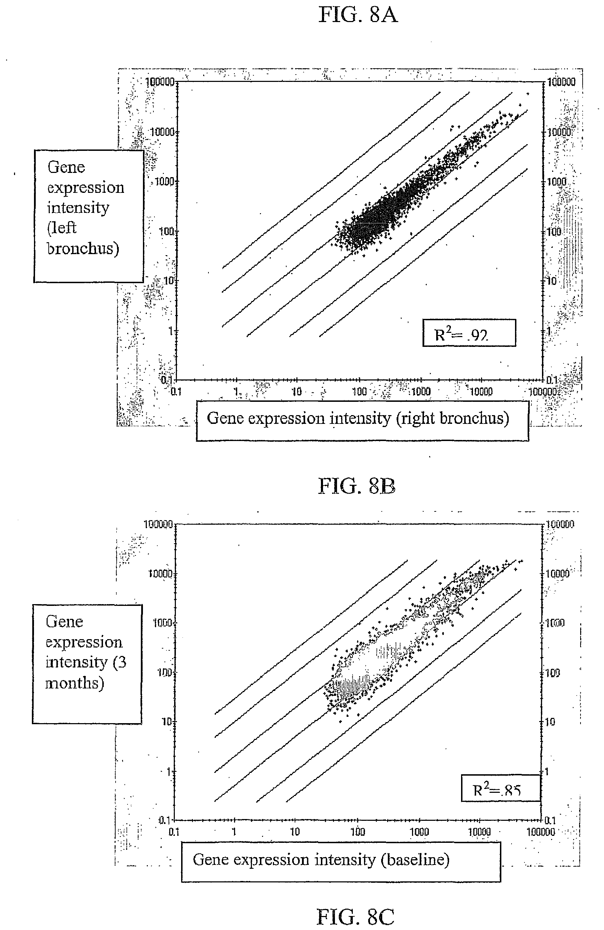

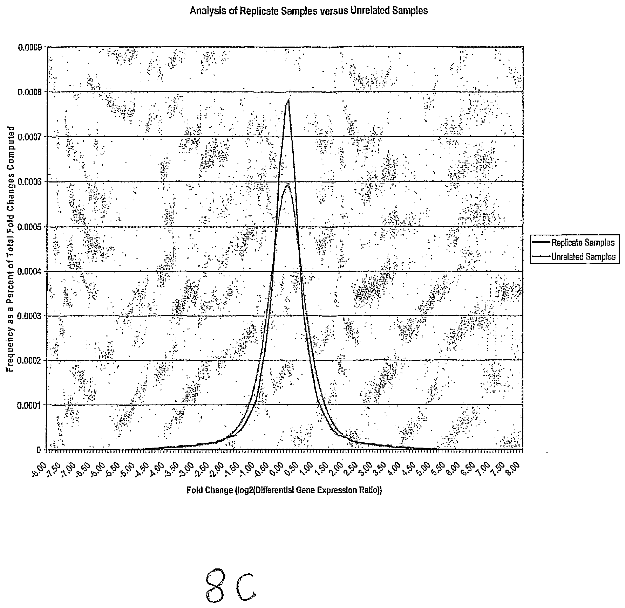

Figures 8A-8C show Scatterplots of spatial (Figure 8A) and temporal (Figure 8B) replicate samples (2 fold, 10 fold and 30 fold lines of change shown; axes are log scaled). Histogram of fold changes computed between all replicates and between unrelated samples (Figure 8C)

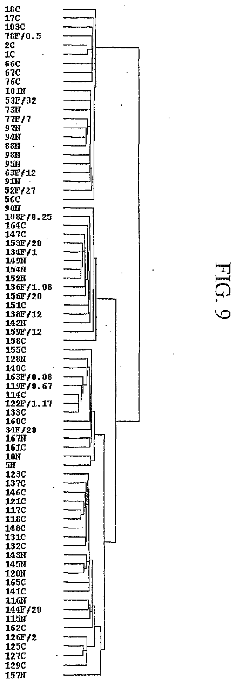

Figure 9 shows a dendogram of samples obtained from hierarchal clustering of the top 1000 most variable genes across all samples. Hierarchical clustering of all samples (n=75 subjects) across the 1000 most variable genes. Current (C), former (F) and never (N) smokers do not cluster into their 3 classes.

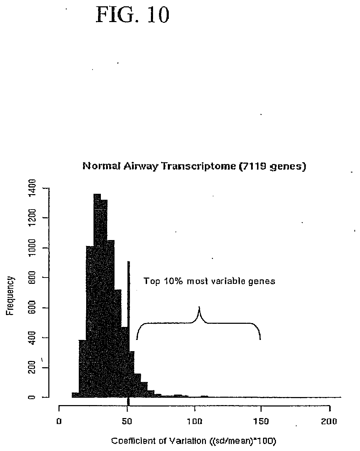

Figure 10 shows variability in gene expression in the normal airway transcriptome. This histogram shows the number of genes in the normal airway transcriptomes (∼7100 genes whose median detection p value < .05) according to their coefficient of variation (standard deviation/mean *100) across the 23 healthy never smokers. Approximately 90% of the genes have a coefficient of variation below 50%

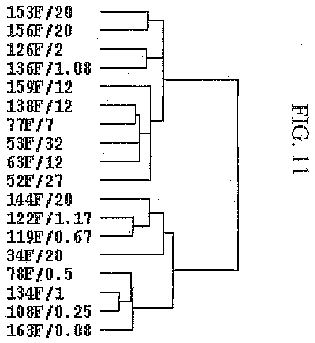

Figure 11 shows hierarchical clustering of all 18 former smokers according to the expression of the top 97 probesets that were differentially expressed between current and never smokers. The only clinical variable that statistically differed (p<.05) between the 2 molecular subclasses of former smokers was length of smoking cessation. Patient ID (denoted with "F") and time since patient quit smoking (in years) are shown

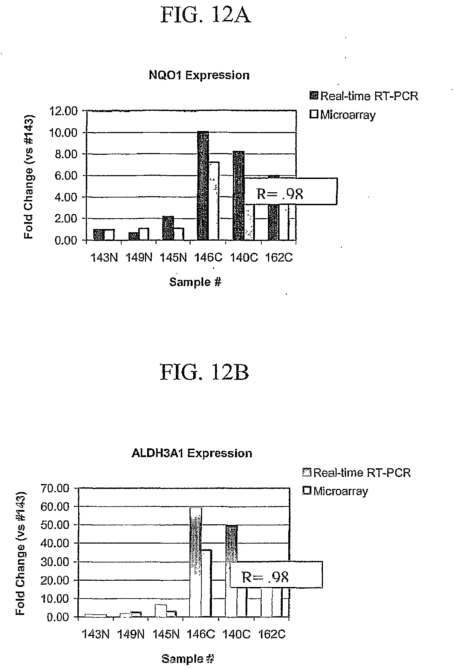

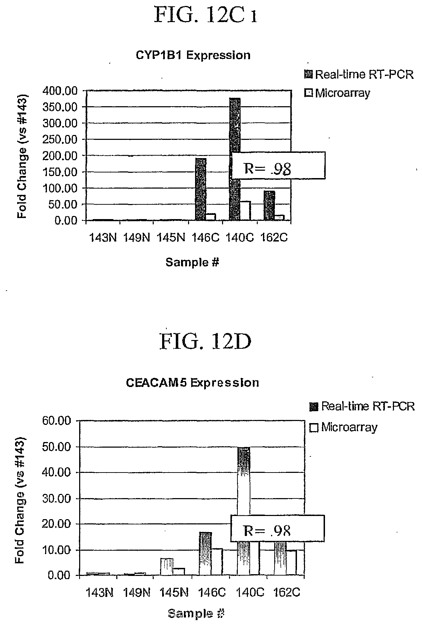

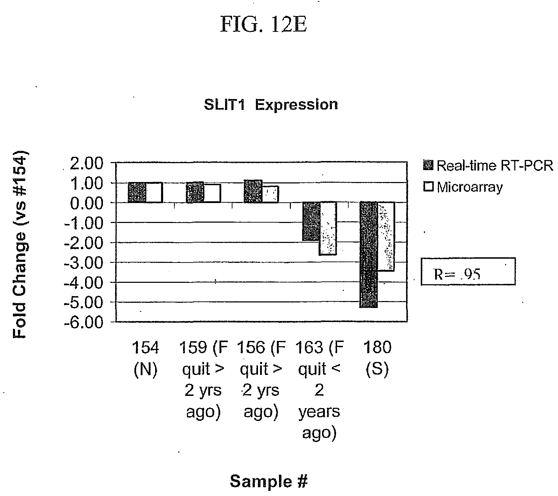

Figures 12A-12E show real time QRT-PCR and microarray data for select genes that were found to be differentially expressed between current and never smokers on microarray analysis. Fold change is relative to one of the never smokers. For NQO1 (NAD(P)H dehydrogenase, quinone 1, Figure 12A), ALDH3A1 (aldehyde dehydrogenase 3 family, memberA1, Figure 12B), CYP1B1 (cytochrome P450, subfamily I (dioxin-inducible), polypeptide 1, Figure 12C) and CEACAM5 (carcinoembryonic antigen-related cell adhesion molecule 5, Figure 12D), gene expression was measured on 3 never smokers (N) and 3 current smokers(S). For SLIT1 (slit homolog 1, Figure 12E), a gene reversibly downregulated by cigarette smoke, gene expression was measured on a never smoker, 2 former smokers who quit smoking more than two years ago, 1 former smoker who quit smoking within the last two years and a current smoker. Pearson correlations between real-time PCR and microarray data for each gene are shown.

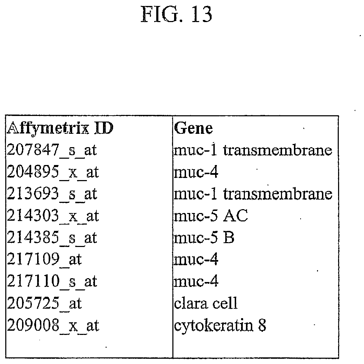

Figure 13 shows a table of genes present in bronchial epithelial cells that should be expressed in bronchial epithelial cells.

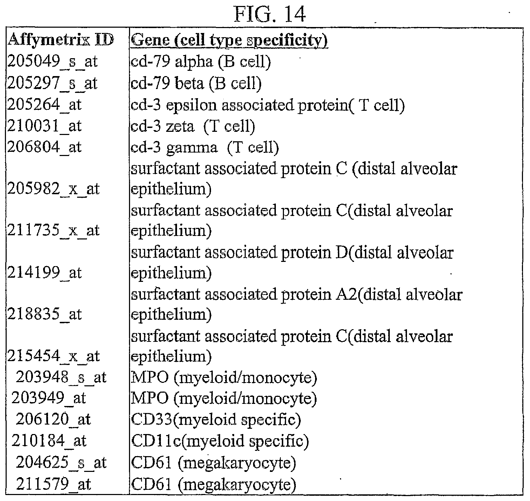

Figure 14 shows genes absent in bronchial epithelial cells that should not be expressed in bronchial airway epithelial cells.

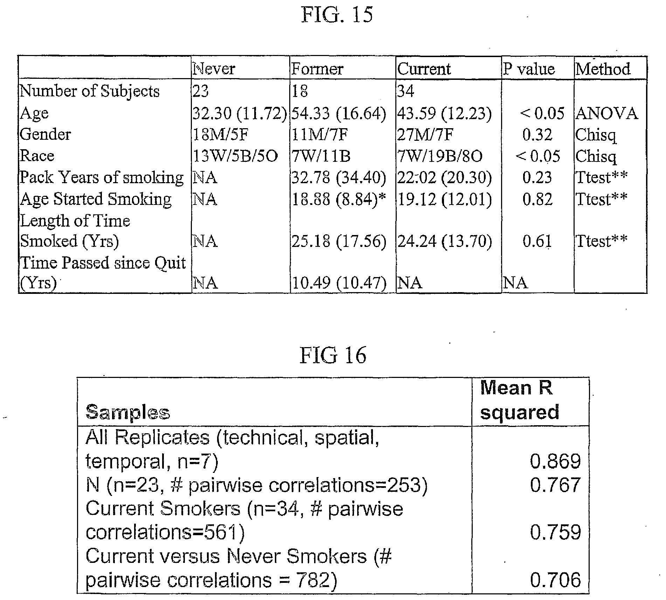

Figure 15 shows demographic features of all 75 patients whose microarrays were included in our study. Three clinical groups were evaluated: never smokers, former smokers and current smokers. For continuous variables, the mean (and the standard deviation) is shown. For gender, M=number of males, F= number of females. For race, W = Caucasian, B = African American, O = other. Pack years of smoking calculated as number of packs of cigarettes per day multiplied by number of years of smoking. ANOVA, t-tests, and Chi-squared tests were used to evaluate differences between groups for continuous variables; chi-square tests were used to evaluate categorical variables. ∗= one value missing, ∗∗ indicates that the data was not normally distributed and therefore, the t-test p-value was computed using logged values.
Figure 16 shows analysis of replicates. Pearson correlation coefficients were computed between replicate samples, between samples from the same group (never or current smoker), and between samples from two different groups (never versus current smoker). The mean R squared values from the analyses are reported.

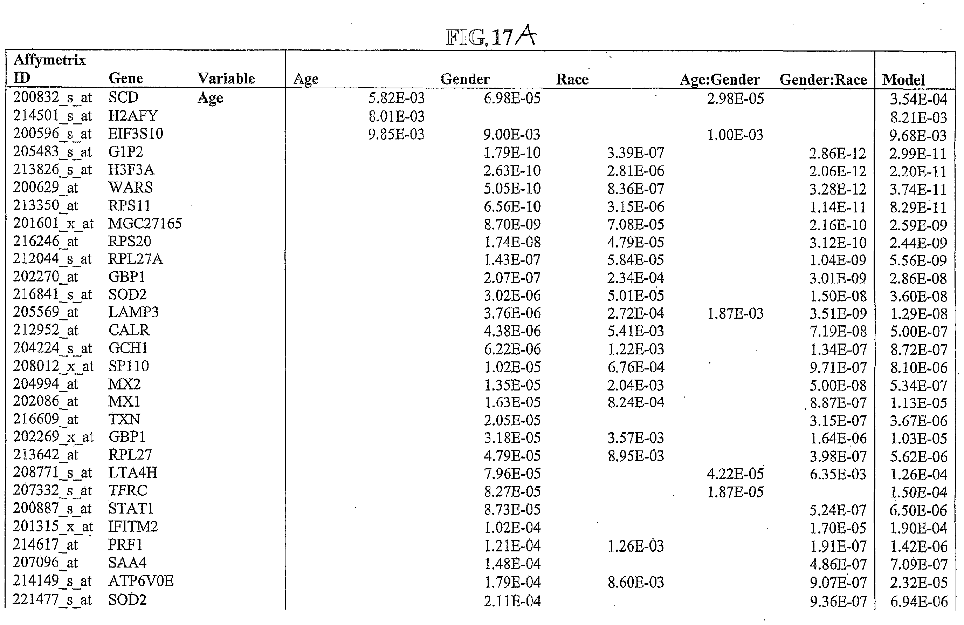

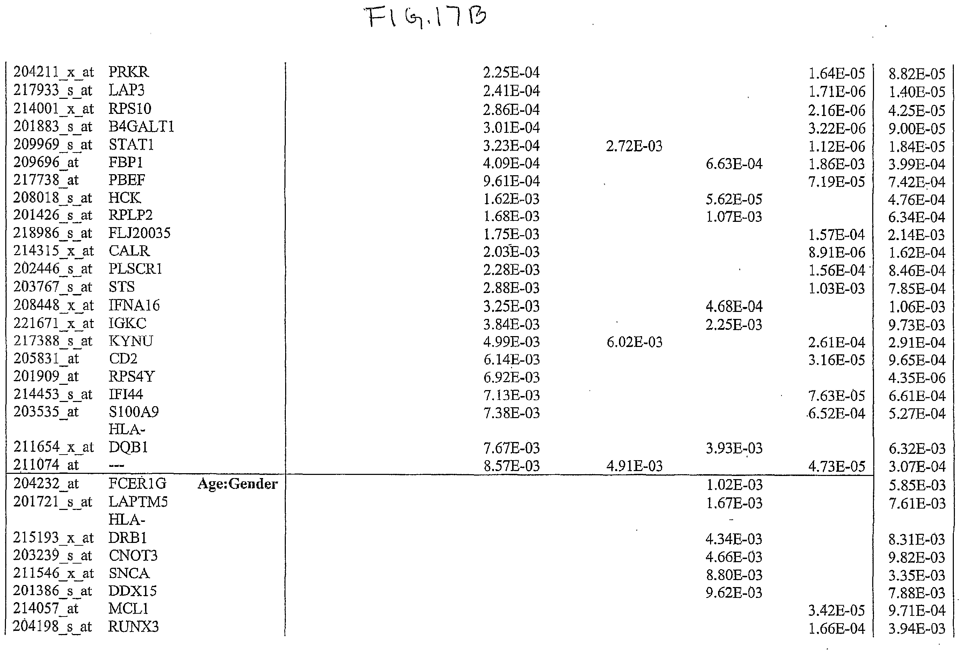

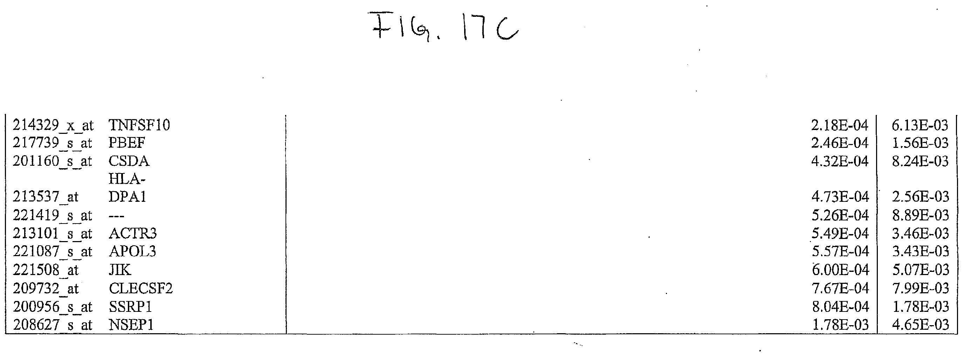

Figure 17A-17C show multiple linear regression results performed on the top 10 percent most variable genes (calculated using the coefficient of variation) in the normal airway transcriptome. A general linear model was used to explore the relationship between gene expression and age, race, gender, and the three possible two-way interaction terms. Seventy models having a p value of 0.01 are shown along with the p values for the significant regressors (p<=0.01).

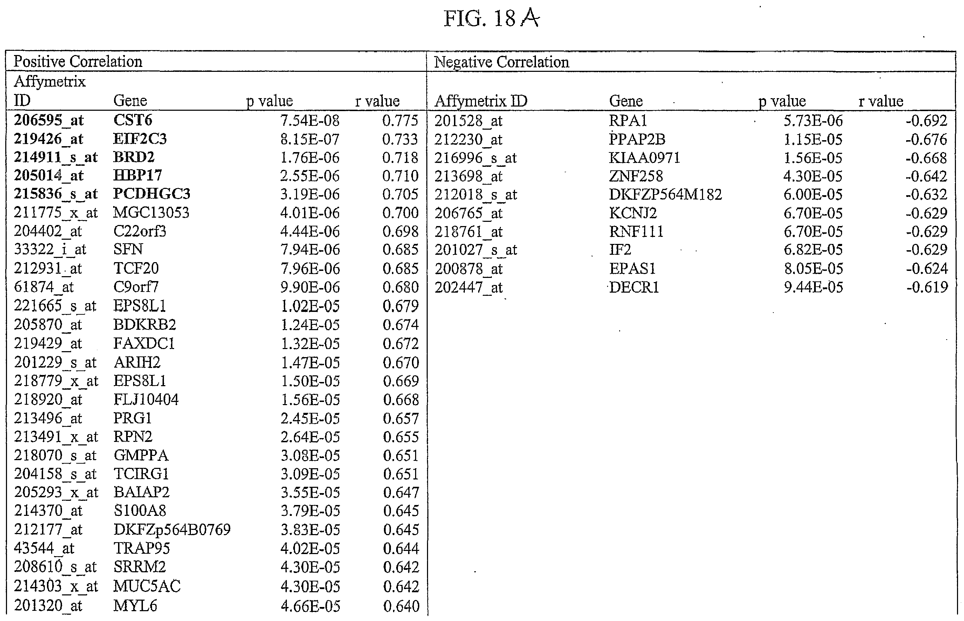

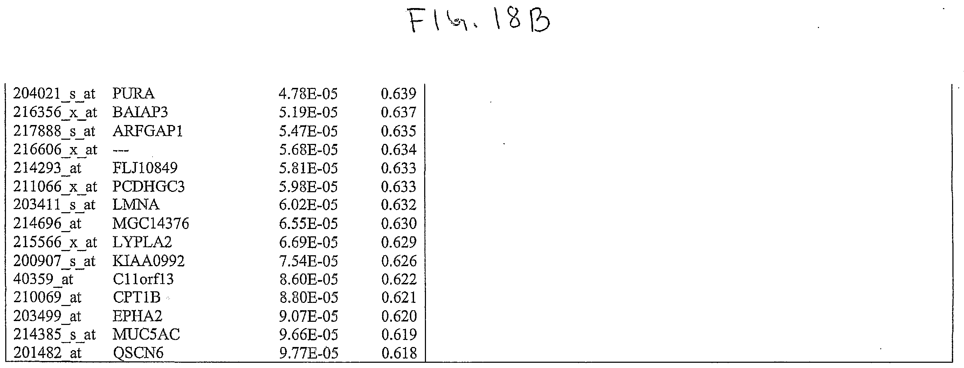

Figures 18A-18B show genes correlated with pack-years among current smokers (p<0.0001). Pearson correlation for gene expression and pack-years smoking. R-values and p-values for 51 genes that were tightly correlated with pack-years among current smokers are reported. The 5 genes shown in bold are the genes whose expression is most significantly correlated to pack-years as assessed by a permutation analysis.

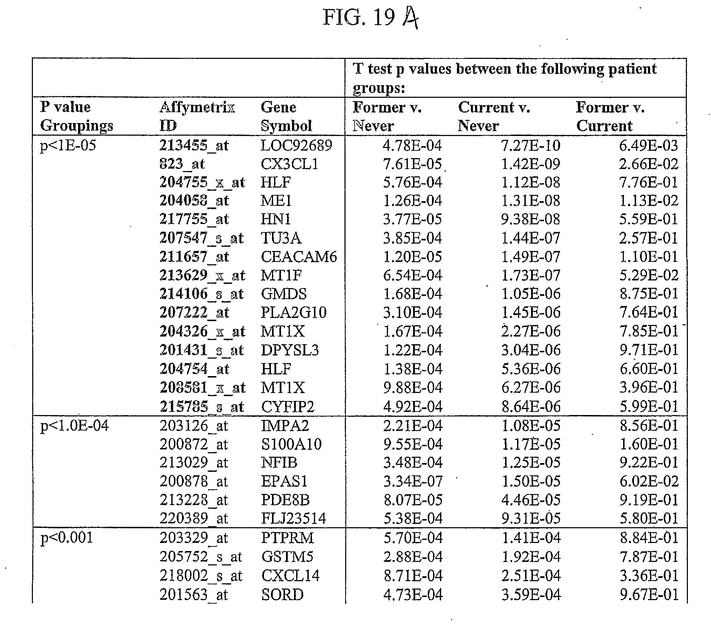

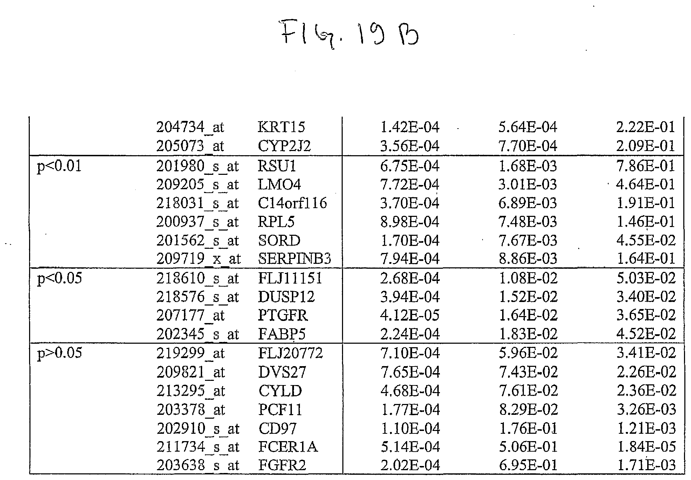

Figure 19A-19B show summary of analysis of genes irreversibly altered by cigarette smoke. A t-test was performed between former and never smoker across all 9968 genes, and 44 genes were found to have a p value threshold below 0.00098. These 44 genes are listed in the table according to their p value on t-test between current and never smokers, as the intersection of these 2 t-tests (former vs. never and current vs. never) correspond to irreversibly altered genes. Fifteen genes (shown in bold) were found to be irreversible altered by cigarette smoking given that they are in common with the list of 97 probesets significantly differentially expressed between current and never smokers. In addition to the 15 genes, 12 more genes had a t-test p value between current and never smokers of less than 0.001, and only 7 of the 44 genes had p values between current and never smokers of greater than 0.05.

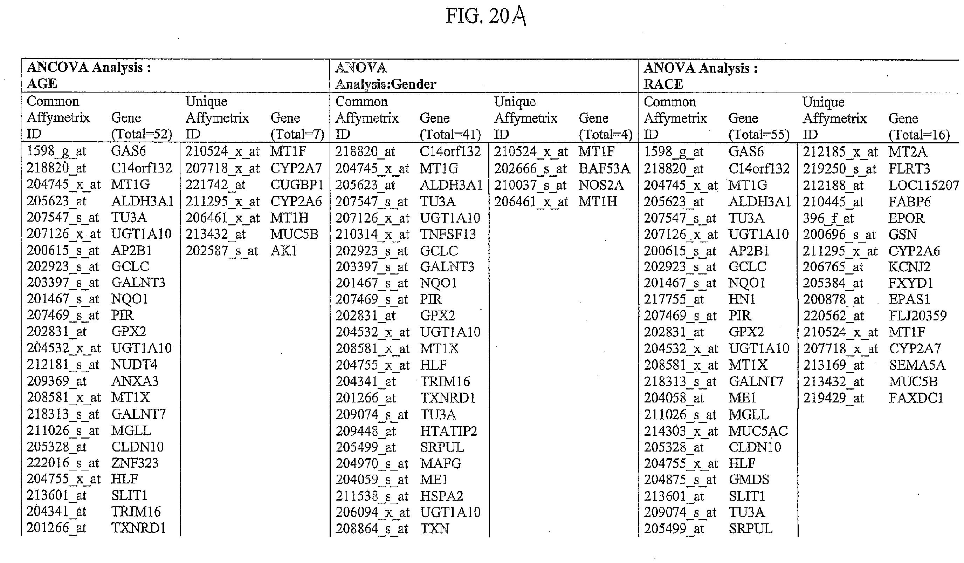

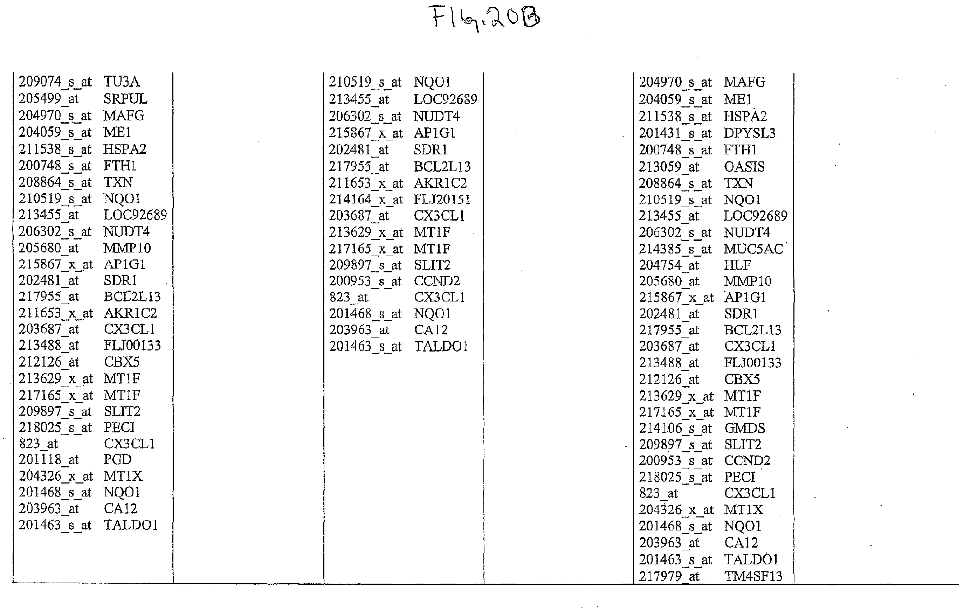

Figures 20A-20B show ANCOVA and 2 way ANOVA. An ANCOVA was performed to test the effect of smoking status (never or current) on gene expression while controlling for the effect of age (the covariate). A two-way ANOVA was performed to test the effect of smoking status (never or current) on gene expression while controlling for the fixed effects of race (encoded as three racial groups: Caucasian, African American, and other) or gender and the interaction terms of status:race or status:gender. The never versus current smoker t-test p value threshold (p value = 1.06 * 10⁻⁵) was used to determine significant genes in the above analyses performed on the filtered set of 9968 genes. The table lists the genes found to be significantly different between never and current smokers controlling for the effects of age, race, and gender. Many of the genes listed are labeled "common" because they are also found in the set of 97 sprobesets found to be significantly different between never and current smokers based on a t-test analysis.

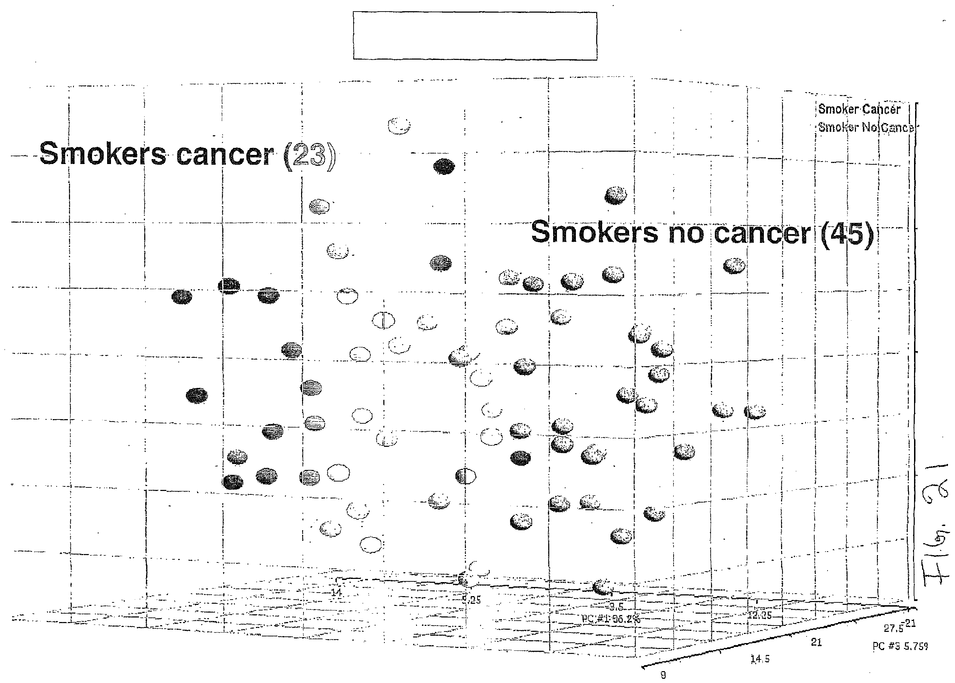

Figure 21 shows a multidimensional scaling plot of all smokers with and without cancer plotted in 208 dimensional space according to the expression of the 208 genes that distinguish the 2 classes on t-test.

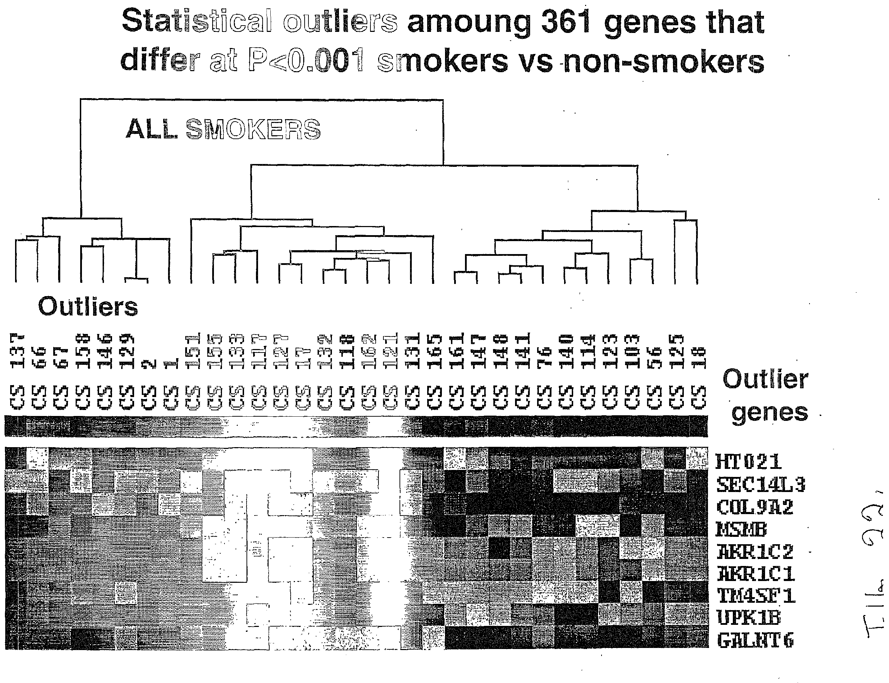

Figure 22 shows a hierarchical clustering plot of all current smokers according to the expression of 9 genes considered to be statistical outliers among at least 3 patients by Grubb's test. These 9 genes were selected from the 361 genes found to be differentially expressed between current and never smokers at p< 0.001. Darker gray=high level of expression, lighter grey=low level of expression, black= mean level of expression.

### DETAILED DESCRIPTION OF THE INVENTION

The present invention provides prognostic, diagnostic, and therapeutic tools for the disorders of lung, particularly, lung cancer. The invention is based on the identification of a "field defect" phenomenon and specific expression patterns related to airway epithelial cell exposure to pollutants, such as cigarette smoke. The airway expression patterns of the present invention can be analyzed using nucleic acids and/or proteins from a biological sample of the airways.

The term "field defect" as used throughout the specification means that the transcription pattern of epithelial cells lining the entire airway including the mouth buccal mucosa, airways, and lung tissue changes in response to airway pollutants. Therefore, the present invention provides methods to identify epithelial cell gene expression patterns that are associated with diseases and disorders of lung.

For example, lung cancer involves histopathological and molecular progression from normal to premalignant to cancer. Gene expression arrays of lung tumors have been used to characterize expression profiles of lung cancers, and to show the progression of molecular changes from non-malignant lung tissue to lung cancer. However, for the screening and early diagnostic purpose, it is not practicable to obtain samples from the lungs. Therefore, the present invention provides for the first time, a method of obtaining cells from other parts of the airways to identify the epithelial gene expression pattern in an individual.

The ability to determine which individuals have molecular changes in their airway epithelial cells and how these changes relate to a lung disorder, such as premalignant and malignant changes is a significant improvement for determining risk and for diagnosing a lung disorder such as cancer at a stage when treatment can be more effective, thus reducing the mortality and morbidity rates of lung cancer. The ease with which airway epithelial cells can be obtained, such as bronchoscopy and buccal mucosal scrapings, shows that this approach has wide clinical applicability and is a useful tool in a standard clinical screening for the large number of subjects at risk for developing disorders of the lung.

The term "control" or phrases "group of control individuals" or "control individuals" as used herein and throughout the specification refer to at least one individual, preferably at least 2, 3, 4, 5, 6, 7, 8, 9, or 10 individuals, still more preferably at least 10-100 individuals or even 100-1000 individuals, whose airways can be considered having being exposed to similar pollutants than the test individual or the individual whose diagnosis/prognosis/therapy is in question. As a control these are individuals who are selected to be similar to the individuals being tested. For example, if the individual is a smoker, the control groups consists of smokers with similar age, race and smoking pattern or pack years of smoking. Whereas if the individual is a non-smoker the control is from a group of non-smokers.

Lung disorders which may be diagnosed or treated by methods described herein include, but are not limited to, asthma, chronic bronchitis, emphysema, bronchietasis, primary pulmonary hypertension and acute respiratory distress syndrome. The methods described herein may also be used to diagnose or treat lung disorders that involve the immune system including, hypersensitivity pneumonitis, eosinophilic pneumonias, and persistent fungal infections, pulmonary fibrosis, systemic sclerosis, ideopathic pulmonary hemosiderosis, pulmonary alveolar proteinosis, cancers of the lung such as adenocarcinoma, squamous cell carcinoma, small cell and large cell carcinomas, and benign neoplasms of the lung including bronchial adenomas and hamartomas.

The biological samples useful according to the present invention include, but are not limited to tissue samples, cell samples, and excretion samples, such as sputum or saliva, of the airways. The samples useful for the analysis methods according to the present invention can be taken from the mouth, the bronchial airways, and the lungs.

In one embodiment, the invention provides an "airway transcriptome" the expression pattern of which is useful in prognostic, diagnostic and therapeutic applications as described herein. The airway transcriptome of the present invention comprises 85 genes the expression of which differs significantly between healthy smokers and healthy non-smokers. The airway transcriptome according to the present invention comprises 85 genes, corresponding to 97 probesets, as a number of genes are represented by more than one probeset on the affymetrix array, identified from the about 7100 probesets the expression of which was statistically analyzed using epithelial cell RNA samples from smokers and non-smokers. Therefore, the invention also provides proteins that are encoded by the 85 genes. The 85 identified airway transcriptome genes are listed on the following Table 3:

**Table 3.**

| | | |
|---|---|---|
| 1. | HLF | hepatic leukemia factor (OMIM#142385) |
| 2. | CYFIP2 | CYTOPLASMIC FMRP-INTERACTING PROTEIN 2 (OMIM#606323) |
| 3. | MGLL | monoglyceride lipase (GenBank gi:47117287) |
| 4. | HSPA2 | HEAT-SHOCK 70-KD PROTEIN 2 (OMIM#140560) |
| 5. | DKFZP586B2420 | GeneCards™ database (Weitzman Institute of Science, Rehovot, Israel) at http://www6.unito.it/cgi-bin/cards/carddisp?DKFZP586B2420 |
| 6. | SLIT1 | SLIT, DROSOPHILA, HOMOLOG OF, 1 (OMIM#603742) |
| 7. | SLIT2 | SLIT, DROSOPHILA, HOMOLOG OF, 2 (OMIM#603746) |
| 8. | C14orf132 | hypothetical protein (GeneCards™ database Id No. GC14P094495 at http://bioinfo.cnio.es/cgi-bin/db/genecards/carddisp?C14orf132) |
| 9. | TU3A | DOWNREGULATED IN RENAL CELL CARCINOMA 1 (OMIM#608295) |
| 10. | MMP10 | MATRIX METALLOPROTEIN 10 (OMIM#185260) |
| 11. | CCND2 | CYCLIN D2; CCND2 (OMIM#123833) |
| 12. | CX3CL1 | CHEMOKINE, CX3C MOTIF, LIGAND 1 (OMIM#601880) |
| 13. | MGC5560 | MutDB database at http://mutdb.org/AnnoSNP/data/48/S1/DE/AC.nt.html |
| 14. | MT1F | METALLOTHIONEIN 1F (OMIM#156352) |
| 15. | RNAHP | Homo sapiens RNA helicase-related protein (Unigene/Hs. 8765) |
| 16. | MT1X | METALLOTHIONEIN 1X (OMIM#156359) |
| 17. | MT1L | METALLOTHIONEIN 1L (OMIM#156358) |
| 18. | MT1G | METALLOTHIONEIN 1G (OMIM#156353) |
| 19. | PEC1 | GenBank ID No. AI541256 |
| 20. | TNFSF13 | TUMOR NECROSIS FACTOR LIGAND SUPERFAMILY, MEMBER 13 (OMIM#604472) |
| 21. | GMDS | GDP-MANNOSE 4,6-DEHYDRATASE (OMIM#602884) |
| 22. | ZNF232 | ZINC FINGER PROTEIN 2 (OMIM#194500) |
| 23. | GALNT12 | UDP-N-ACETYL-ALPHA-D-GALACTOSAMINE:POLYPEPTIDE N-ACETYLGALACTOSAMINYLTRANSFERASE 13 (OMIM#608369) |
| 24. | AP2B1 | ADAPTOR-RELATED PROTEIN COMPLEX 2, BETA-1 SUBUNIT (OMIM#601925) |
| 25. | HN1 | HUMANIN (OMIM#606120) |
| 26. | ABCC1 | ATP-BINDING CASSETTE, SUBFAMILY C, MEMBER 1 (OMIM#158343) |
| 27. | RAB11A | RAS FAMILY, MEMBER RAB11A (OMIM#605570) |
| 28. | MSMB | MICROSEMINOPROTEIN, BETA (OMIM#157145) |
| 29. | MAFGV-MAF | AVIAN MUSCULOAPONEUROTIC FIBROSARCOMA ONCOGENE FAMILY, PROTEIN G (OMIM#602020) |
| 30. | ABHD2 | GeneCards™ ID No. GC15P087361 |
| 31. | ANXA3 | ANNEXIN A3 (OMIM#106490) |
| 32. | VMD2 | VITELLIFORM MACULAR DYSTROPHY GENE 2 (OMIM#607854) |
| 33. | FTH1 | FERRITIN HEAVY CHAIN 1 (OMIM#134770) |
| 34. | UGT1A3 | UDP-GLYCOSYLTRANSFERASE 1 FAMILY, POLYPEPTIDE A3 (OMIM#606428) |
| 35. | TSPAN-1 | tetraspan 1 (GeneID: 10103 at Entrez Gene, NCBI Database) |
| 36. | CTGF | CONNECTIVE TISSUE GROWTH FACTOR (OMIM#121009) |
| 37. | PDG | phosphoglycerate dehydrogenase (GeneID: 26227 at Entrez Gene, NCBI Database) |
| 38. | HTATIP2 | HIV-1 TAT-INTERACTING PROTEIN 2, 30-KD (OMIM#605628) |
| 39. | CYP4F11 | CYTOCHROME P450, SUBFAMILY IVF, POLYPEPTIDE 11 |
| 40. | GCLM | GLUTAMATE-CYSTEINE LIGASE, MODIFIER SUBUNIT (OMIM#601176) |
| 41. | ADH7 | ALCOHOL DEHYDROGENASE 7 (OMIM#600086) |
| 42. | GCLC | GLUTAMATE-CYSTEINE LIGASE, CATALYTIC SUBUNIT (OMIM#606857) |
| 43. | UPK1B | UROPLAKIN 1B (OMIM#602380) |
| 44. | PLEKHB2 | pleckstrin homology domain containing, family B (evectins) member 2, GENEATLAS GENE DATABASE AT http://www.dsi.univ-paris5.fr/genatlas/fiche1.php?symbol=PLEKHB2 |
| 45. | TCN1 | TRANSCOBALAMIN I (OMIM#189905) |
| 46. | TRIM16 | TRIPARTITE MOTIF-CONTAINING PROTEIN 16 |
| 47. | UGT1A9 | UDP-GLYCOSYLTRANSFERASE 1 FAMILY, POLYPEPTIDE A9 (OMIM#606434) |
| 48. | UGT1A1 | UDP-GLYCOSYLTRANSFERASE 1 FAMILY, POLYPEPTIDE A1 (OMIM#191740) |
| 49. | UGT1A6 | UDP-GLYCOSYLTRANSFERASE 1 FAMILY, POLYPEPTIDE A6 (OMIM#606431) |
| 50. | NQ01 | NAD(P)H dehydrogenase, quinone 1 (OMIM# 125860) |
| 51. | TXNRD1 | THIOREDOXIN REDUCTASE 1 (OMIM#601112) |
| 52. | PRDX1 | PEROXIREDOXIN 1 (OMIM#176763) |
| 53. | ME1 | MALIC ENZYME 1 (OMIM#154250) |
| 54. | PIR | PIRIN (OMIM# 603329) |
| 55. | TALDO1 | TRANSALDOLASE 1 (OMIM#602063) |
| 56. | GPX2 | GLUTATHIONE PEROXIDASE 2 (OMIM#138319) |
| 57. | AKR1C3 | ALDO-KETO REDUCTASE FAMILY 1, MEMBER C3 (OMIN#603966) |
| 58. | AKR1C1 | ALDO-KETO REDUCTASE FAMILY 1, MEMBER 1 (OMIM#600449) |
| 59. | AKR1C-pseudo | ALDO-KETO REDUCTASE FAMILY 1, pseudo gene, GeneCards™ No. GC10U990141 |
| 60. | AKR1C2 | ALDO-KETO REDUCTASE FAMILY 1, MEMBER C2 (OMIM#600450) |
| 61. | ALDH3A1 | ALDEHYDE DEHYDROGENASE, FAMILY 3, SUBFAMILY A, MEMBER 1 (OMIM#100660) |
| 62. | CLDN10 | CLAUDIN 10 (GeneCards™ ID: GC13P093783) |
| 63. | TXN | thioredoxin (OMIM#187700) |
| 64. | TKT | TRANSKETOLASE (OMIM#606781) |
| 65. | CYP1B1 | CYTOCHROME P450, SUBFAMILY I, POLYPEPTIDE 1 (OMIM#601771) |
| 66. | CBR1 | CARBONYL REDUCTASE 1 (OMIM#114830) |
| 67. | AKR1B1 | ALDO-KETO REDUCTASE FAMILY 1, MEMBER B1 (OMIM#103880) |
| 68. | NET6 | Transmembrane 4 superfamily member 13 (GenBank ID gi:11135162) |
| 69. | NUDT4 | nudix (nucleoside diphosphate linked moiety X)-type motif 4 (Entrez GeneID: 378990) |
| 70. | GALNT3 | UDP-N-ACETYL-ALPHA-D-GALACTOSAMINE:POLYPEPTIDE N-ACETYLGALACTOSAMINYLTRANSFERASE 3 (OMIM#601756) |
| 71. | GALNT7 | UDP-N-ACETYL-ALPHA-D-GALACTOSAMINE:POLYPEPTIDE N-ACETYLGALACTOSAMINYLTRANSFERASE 7 (OMIM#605005) |
| 72. | CEACAM6 | CARCINOEMBRYONIC ANTIGEN-RELATED CELL ADHESION MOLECULE 6 (OMIM#163980) |
| 73. | AP1G1 | ADAPTOR-RELATED PROTEIN COMPLEX 1, GAMMA-1 SUBUNIT (OMIM#603533) |
| 74. | CA12 | CARBONIC ANHYDRASE XII (OMIM#603263) |
| 75. | FLJ20151 | hypothetical protein (GeneCards™ ID:GC15MO61330) |
| 76. | BCL2L13 | apoptosis facilitator (GeneID: 23786, Entrez) |
| 77. | SRPUL | Homo sapiens sushi-repeat protein (MutDB at http://mutdb.org/AnnoSNP/data/DD/S0/9U/AC.nt.html) |
| 78. | FLJ13052 | Homo sapiens NAD kinase (GenBank ID gi:20070325) |
| 79. | GALNT6 | UDP-N-ACETYL-ALPHA-D-GALACTOSAMINE:POLYPEPTIDE N-ACETYLGALACTOSAMINYLTRANSFERASE 6 (OMIM#605148) |
| 80. | OASIS cAMP | responsive element binding protein 3-like 1 (GenBank ID gi:21668501) |
| 81. | MUC5B | MUCIN 5, SUBTYPE B, TRACHEOBRONCHIAL (OMIM#600770) |
| 82. | S100P | S100 CALCIUM-BINDING PROTEIN P (OMIM#600614) |
| 83. | SDR1 | dehydrogenase/reductase (SDR family) member 3 (GeneID: 9249, Entrez) |
| 84. | PLA2G10 | PHOSPHOLIPASE A2, GROUP X (OMIM#603603) |
| 85. | DPYSL3 | DIHYDROPYRIMIDINASE-LIKE 3 (OMIM#601168) |

The invention further provides a lung cancer diagnostic airway transcriptome comprising at least 208 genes that are differentially expressed between smokers with lung cancer and smokers witout lung cancer. The genes identified as being part of the diagnostic airway transcriptome are 208238_x_at-probeset; 216384_x_at-probeset; 217679_x_at-probeset; 216859_x_at-probeset; 211200_s_at-probeset; PDPK1; ADAM28; ACACB; ASMTL; ACVR2B; ADAT1; ALMS1; ANK3; ANK3; DARS; AFURS1; ATP8B1; ABCC1; BTF3; BRD4; CELSR2; CALM31 CAPZB; CAPZB1 CFLAR; CTSS; CD24; CBX3; C21orf106; C6orf111; C6orf62; CHC1; DCLRE1C; EML2; EMS1; EPHB6; EEF2; FGFR3; FLJ20288; FVT1; GGTLA4; GRP; GLUL; HDGF; Homo sapiens cDNA FLJ11452 fis, clone HEMBA1001435; Homo sapiens cDNA FLJ12005 fis, clone HEMBB1001565; Homo sapiens cDNA FLJ13721 fis, clone PLACE2000450; Homo sapiens cDNA FLJ14090 fis, clone MAMMA1000264; Homo sapiens cDNA FLJ14253 fis, clone OVARC1001376; Homo sapiens fetal thymus prothymosin alpha mRNA, complete cds Homo sapiens fetal thymus prothymosin alpha mRNA; Homo sapiens transcribed sequence with strong similarity to protein ref:NP_004726.1 (H.sapiens) leucine rich repeat (in FLII) interacting protein 1; Homo sapiens transcribed sequence with weak similarity to protein ref:NP_060312.1 (H.sapiens) hypothetical protein FLJ20489; Homo sapiens transcribed sequence with weak similarity to protein ref:NP_060312.1 (H.sapiens) hypothetical protein FLJ20489; 222282_at-probeset corresponding to Homo sapiens transcribed sequences; 215032_at-probeset corresponding to Homo sapiens transcribed sequences; 81811_at-probeset corresponding to Homo sapiens transcribed sequences; DKFZp547K1113; ET; FLJ10534; FLJ10743; FLJ13171; FLJ14639; FLJ14675; FLJ20195; FLJ20686; FLJ20700; CG005; CG005; MGC5384; IMP-2; INADL; INHBC; KIAA0379; KIAA0676; KIAA0779; KIAA1193; KTN1; KLF5; LRRFIP1; MKRN4; MAN1C1; MVK; MUC20; MPZL1; MYO1A; MRLC2; NFATC3; ODAG; PARVA; PASK; PIK3C2B; PGF; PKP4; PRKX; PRKY; PTPRF; PTMA; PTMA; PHTF2; RAB14; ARHGEF6; RIPX; REC8L1; RIOK3; SEMA3F; SRRM21 MGC709071 SMT3H2; SLC28A3; SAT; SFRS111 SOX2; THOC2; TRIM51 USP7; USP9X; USH1C; AF020591; ZNF131; ZNF160; ZNF264; 217414_x_at-probeset;; 217232_x_at-probeset;; ATF3; ASXL2; ARF4L; APG5L; ATP6V0B; BAG1; BTG2; COMT; CTSZ; CGI-128; C14orf87; CLDN3; CYR61; CKAP1; DAF; DAF; DSIPI; DKFZP564G2022; DNAJB9; DDOST; DUSP1; DUSP6; DKC1; EGR1; EIF4EL3; EXT2; GMPPB; GSN; GUK1; HSPA8; Homo sapiens PRO2275 mRNA, complete cds; Homo sapiens transcribed sequence with strong similarity to protein ref:NP_006442.2, polyadenylate binding protein-interacting protein 1; HAX1; DKFZP434K046; IMAGE3455200; HYOU1; IDN3; JUNB; KRT8; KIAA0100; KIAA0102; APH-1A; LSM4; MAGED2; MRPS7; MOCS2; MNDA; NDUFA8; NNT; NFIL3; PWP1; NR4A2; NUDT4; ORMDL2; PDAP2; PPIH; PBX3; P4HA2; PPP1R15A; PRG11 P2RX4; SUI1; SUI1; SUI1; RAB5C; ARHB; RNASE4; RNH; RNPC4; SEC23B; SERPINA1; SH3GLB1; SLC35B1; SOX9; SOX9; STCH; SDHC; TINF2; TCF8; E2-EPF; FOS; JUN; ZFP36; ZNF500; and ZDHHC4.

Deviation in the expression compared to control group can be increased expression or decreased expression of one or more of the 208 genes. Preferably, downregulation of expression of at least one, preferably at least 10, 15, 25, 30, 50, 60, 75, 80, 90, 100, 110, or all of the 121 genes consisting of 208238_x_at-probeset; 216384_x_at-probeset; 217679_x_at-probeset; 216859_x_at-probeset; 211200_s_at- probeset; PDPK1; ADAM28; ACACB; ASMTL; ACVR2B; ADAT1; ALMS1; ANK3; ANK3; DARS; AFURS1; ATP8B1; ABCC1; BTF3; BRD4; CELSR2; CALM31 CAPZB; CAPZB1 CFLAR; CTSS; CD24; CBX3; C21orf106; C6orf111; C6orf62; CHC1; DCLRE1C; EML2; EMS1; EPHB6; EEF2; FGFR3; FLJ20288; FVT1; GGTLA4; GRP; GLUL; HDGF; Homo sapiens cDNA FLJ11452 fis, clone HEMBA1001435; Homo sapiens cDNA FLJ12005 fis, clone HEMBB1001565; Homo sapiens cDNA FLJ13721 fis, clone PLACE2000450; Homo sapiens cDNA FLJ14090 fis, clone MAMMA1000264; Homo sapiens cDNA FLJ14253 fis, clone OVARC1001376; Homo sapiens fetal thymus protliymosin alpha mRNA, complete cds; Homo sapiens transcribed sequence with strong similarity to protein ref:NP_004726.1 (H.sapiens) leucine rich repeat (in FLII) interacting protein 1; Homo sapiens transcribed sequence with weak similarity to protein ref:NP_060312.1 (H.sapiens) hypothetical protein FLJ20489; Homo sapiens transcribed sequence with weak similarity to protein ref:NP_060312.1 (H.sapiens) hypothetical protein FLJ20489; 222282_at-probeset corresponding to Homo sapiens transcribed sequences; 215032_at-probeset corresponding to Homo sapiens transcribed sequences; 81811_at-probeset corresponding to Homo sapiens transcribed sequences; DKFZp547K1113; ET; FLJ10534; FLJ10743; FLJ13171; FLJ14639; FLJ14675; FLJ20195; FLJ20686; FLJ20700; CG005; CG005; MGC5384; IMP-2; INADL; INHBC; KIAA0379; KIAA0676; KIAA0779; KIAA1193; KTN1; KLF5; LRRFIP1; MKRN4; MAN1C1; MVK; MUC20; MPZL1; MYO1A; MRLC2; NFATC3; ODAG; PARVA; PASK; PIK3C2B; PGF; PKP4; PRKX; PRKY; PTPRF; PTMA; PTMA; PHTF2; RAB14; ARHGEF6; RIPX; REC8L1; RIOK3; SEMA3F; SRRM21 MGC709071 SMT3H2; SLC28A3; SAT; SFRS111 SOX2; THOC2; TRIM51 USP7; USP9X; USH1C; AF020591; ZNF131; ZNF160; andZNF264, when compared to a control group is indicative of lung cancer.

Preferably increase, or up-regulation of expression of at least one, preferably at least 10, 15, 25, 30, 50, 60, 75, 80, or all of the 87 genes consisting of of 217414_x_at-probeset;; 217232_x_at-probeset;; ATF3; ASXL2; ARF4L; APG5L; ATP6V0B; BAG1; BTG2; COMT; CTSZ; CGI-128; C14orf87; CLDN3; CYR61; CKAP1; DAF; DAF; DSIPI; DKFZP564G2022; DNAJB9; DDOST; DUSP1; DUSP6; DKC1; EGR1; EIF4EL3; EXT2; GMPPB; GSN; GUK1; HSPA8; Homo sapiens PRO2275 mRNA, complete cds; Homo sapiens transcribed sequence with strong similarity to protein ref:NP_006442.2, polyadenylate binding protein-interacting protein 1; HAX1; DKFZP434K046; IMAGE3455200; HYOU1; IDN3; JUNB; KRT8; KIAA0100; KIAA0102; APH-1A; LSM4; MAGED2; MRPS7; MOCS2; MNDA; NDUFA8; NNT; NFIL3; PWP1; NR4A2; NUDT4; ORMDL2; PDAP2; PPIH; PBX3; P4HA2; PPP1R15A; PRG11 P2RX4; SUI1; SUI1; SUI1; RAB5C; ARHB; RNASE4; RNH; RNPC4; SEC23B; SERPINA1; SH3GLB1; SLC35B1; SOX9; SOX9; STCH; SDHC; TINF2; TCF8; E2-EPF; FOS; JUN; ZFP36; ZNF500; and ZDHHC4 as compared to a control group indicated that the individual is affected with lung cancer.

The probeset numbers as referred to herein and throughout the specification, refer to the Affymetrix probesets.

The methods to identify the airway transcriptomes can be used to identify airway transcriptomes in other animals than humans by performing the statistical comparisons as provided in the Examples below in any two animal groups, wherein one group is exposed to an airway pollutant and the other group is not exposed to such pollutant and performing the gene expression analysis of any large probeset, such as the probeset of 7119 genes used in the Examples. Therefore, the subject or individual as described herein and throughout the specification is not limited to human, but encompasses other mammals and animals, such as murine, bovine, swine, and other primates. This methodology can also be carried out with lung disorders to create new clusters of genes wherein change in their expression is related to specific disorders.

We identified a subset of three current smokers who did not upregulate expression of a number of predominantly redox/xenobiotic genes to the same degree as other smokers. One of these smokers developed lung cancer within 6 months of the analysis. In addition, there is a never smoker, who is an outlier among never smokers and expresses a subset of genes at the level of current smokers (see Figure 5 and associated Figure legend). These outlier genes are as shown on Table 4 below.

**Table 4:**

| **GENBANK_ID HUGO_ID GENBANK_DESCRIPTION** | | |
|---|---|---|
| | | aldo-keto reductase family 1, |
| | | member C1 (dihydrodiol dehydrogenase 1; |
| NM_001353.2 | AKR1C1 | 20-alpha (3-alpha)-hydroxysteroid dehydrogenase) |
| NM_002443.1 | MSMB | microseminoprotein, beta- |
| AI346835 | TM4SF1 | transmembrane 4 superfamily member 1 |
| NM_006952.1 | UPK1B | uroplakin 1B |
| AI740515 | FLJ20152 | hypothetical protein FLJ20152 |
| AC004832 | SEC14L3 | SEC14-like 3 (S. cerevisiae) |
| NM_020685.1 | HT021 | HT021 |
| | | UDP-N-acetyl-alpha-D-galactosamine:polypeptide |
| NM_007210.2 | GALNT6 | N-acetylgalactosaminyltransferase 6 (GalNAc-T6) |
| NM_001354 | AKR1C2 | aldo-keto reductase family 1, member C2 |

These divergent patterns of gene expression in a small subset of smokers represent a failure of these smokers to mount an appropriate response to cigarette exposure and indicate a linkage to increased risk for developing lung cancer. As a result, these "outlier" genes can thus serve as biomarkers for susceptibility to the carcinogenic effects of cigarette smoke and other air pollutants.

Therefore, in one embodiment, the invention provides a method of determining an increased risk of lung disease, such as lung cancer, in a smoker comprising taking an airway sample from the individual, analyzing the expression of at least one, preferably at least two, still more preferably at least 4, still more preferably at least 5, still more preferably at least 6, still more preferably at least 7, still more preferably at least 8, still more preferably at least 8, and still more preferably at least all 9 of the outlier genes including AKR1C1; MSMB; TM4SF1; UPK1B; FLJ20152; SEC14L3; HT021; GALNT6; and AKR1C2, wherein deviation of the expression of at least one, preferably at least two, still more preferably at least 4, still more preferably at least 5, still more preferably at least 6, still more preferably at least 7, still more preferably at least 8, still more preferably at least 8, and still more preferably at least all 9 as compared to a control group is indicative of the smoker being at increased risk of developing a lung disease, for example, lung cancer.

Figure 22 shows a hierarchical clustering plot of all current smokers according to the expression of 9 genes considered to be statistical outliers among at least 3 patients by Grubb's test. These 9 genes were selected from the 361 genes found to be differentially expressed between current and never smokers at p< 0.001. Darker gray=high level of expression, lighter grey=low level of expression, black= mean level of expression. It can be clearly seen that the "outlier" individuals have significantly different expression pattern of these 9 nine genes.

We have shown that if the cells in the airways of an individual exposed to pollutant, such as cigarette smoke, do not turn on, or increase the expression of one or more of the certain genes encoding proteins associated with detoxification, and genes encoding mucins and cell adhesion molecules, this individual is at increased risk of developing lung diseases.

We have also shown that if the cells in the airways of an individual exposed to pollutant, such as cigarette smoke, do not turn off, or decrease the transcription of genes encoding one or more of certain proteins associated with immune regulation and metallothioneins, the individual has an increased risk of developing lung disease.

We have also shown that if the cells in the airways of an individual exposed to pollutant, such as cigarette smoke, do not turn off one or more tumor suppressor genes or turn on one or more protooncogenes, the individual is at increased risk of developing lung disease.

The methods disclosed herein can also be used to show exposure of a non-smoker to environmental pollutants by showing increased expression in a biological sample taken from the airways of the non-smoker of genes encoding proteins associated with detoxification, and genes encoding mucins and cell adhesion molecules or decreased expression of genes encoding certain proteins associated with immune regulation and metallothioneins. If such changes are observed, an entire group of individuals at work or home environment of the exposed individual may be analyzed and if any of them does not show the indicative increases and decreases in the expression of the airway transcriptome, they may be at greater risk of developing a lung disease and susceptible for intervention. These methods can be used, for example, in a work place screening analyses, wherein the results are useful in assessing working environments, wherein the individuals may be exposed to cigarette smoke, mining fumes, drilling fumes, asbestos and/or other chemical and/or physical airway pollutants. Screening can be used to single out high risk workers from the risky environment to transfer to a less risky environment.

Accordingly, in one embodiment, the invention provides prognostic and diagnostic methods to screen for individuals at risk of developing diseases of the lung, such as lung cancer, comprising screening for changes in the gene expression pattern of the airway transcriptome. The method comprises obtaining a cell sample from the airways of an individual and measuring the level of expression of 1-85 gene transcripts of the airway transcriptome as provided herein. Preferably, the level of at least two, still more preferably at least 3, 4, 5, 6, 7, 8, 9, 10 transcripts, and still more preferably, the level of at least 10-15, 15-20, 20-50, or more transcripts, and still more preferably all of the 97 trasncripts in the airway transcriptome are measured, wherein difference in the expression of at least one, preferably at least two, still more preferably at least three, and still more preferably at least 4, 5, 6, 7, 8, 9, 10, 10-15, 15-20, 20-30, 30-40, 40-50, 50-60, 60-70, 70-80, 80-85 genes present in the airway transcriptome compared to a normal airway transcriptome is indicative of increased risk of a lung disease. The control being at least one, preferably a group of more than one individuals exposed to the same pollutant and having a normal or healthy response to the exposure.

In one embodiment, difference in at least one of the detoxification related genes, mucin genes, and/or cell adhesion related genes compared to the level of these genes expressed in a control, is indicative of the individual being at an increased risk of developing diseases of the lung. The differences in expression of at least one immune system regulation and/or metallothionein regulation related genes compared to the level of these genes expressed in a control group indicates that the individual is at risk of developing diseases of the lung.

In one embodiment, the invention provides a prognostic method for lung diseases comprising detecting gene expression changes in at least on of the mucin genes of the airway transcriptome, wherein increase in the expression compared with control group is indicative of an increased risk of developing a lung disease. Examples of mucin genes include muc 5 subtypes A, B, and C.

In one preferred embodiment, the invention provides a tool for screening for changes in the airway transcriptome during long time intervals, such as weeks, months, or even years. The airway trasncriptome expression analysis is therefore performed at time intervals, preferably two or more time intervals, such as in connection with an annual physical examination, so that the changes in the airway transcriptome expression pattern can be tracked in individual basis. The screening methods of the invention are useful in following up the response of the airways to a variety of pollutants that the subject is exposed to during extended periods. Such pollutants include direct or indirect exposure to cigarette smoke or other air pollutants.

The control as used herein is a healthy individual, whose responses to airway pollutants are in the normal range of a smoker as provided by, for example, the transcription patterns shown in Figure 5.

Analysis of transcript levels according to the present invention can be made using total or messenger RNA or proteins encoded by the genes identified in the airway trascriptome of the present invention as a starting material. In the preferred embodiment the analysis is an immunohistochemical analysis with an antibody directed against at least one, preferably at least two, still more preferably at least 4-10 proteins encoded by the genes of the airway transcriptome.

The methods of analyzing transcript levels of one or more of the 85 transcripts in an individual include Northern-blot hybridization, ribonuclease protection assay, and reverse transcriptase polymerase chain reaction (RT-PCR) based methods. The different RT-PCR based techniques are the most suitable quantification method for diagnostic purposes of the present invention, because they are very sensitive and thus require only a small sample size which is desirable for a diagnostic test. A number of quantitative RT-PCR based methods have been described and are useful in measuring the amount of transcripts according to the present invention. These methods include RNA quantification using PCR and complementary DNA (cDNA) arrays (Shalon et al., Genome Research 6(7):639-45, 1996; Bernard et al., Nucleic Acids Research 24(8):1435-42, 1996), solid-phase mini-sequencing technique, which is based upon a primer extension reaction (U.S. Patent No. 6,013,431, Suomalainen et al. Mol. Biotechnol. Jun;15(2):123-31, 2000), ion-pair high-performance liquid chromatography (Doris et al. J. Chromatogr. A May 8;806(1):47-60, 1998), and 5'nuclease assay or real-time RT-PCR (Holland et al. Proc Natl Acad Sci USA 88: 7276-7280, 1991).

Methods using RT-PCR and internal standards differing by length or restriction endonuclease site from the desired target sequence allowing comparison of the standard with the target using gel electrophoretic separation methods followed by densitometric quantification of the target have also been developed and can be used to detect the amount of the transcripts according to the present invention(see, e.g., U.S. Patent Nos. 5,876,978; 5,643,765; and 5,639,606.

Antibodies can be prepared by means well known in the art. The term "antibodies" is meant to include monoclonal antibodies, polyclonal antibodies and antibodies prepared by recombinant nucleic acid techniques that are selectively reactive with a desired antigen. Antibodies against the proteins encoded by any of the genes in the diagnostic transcriptome of the present invention are either known or can be easily produced using the methods well known in the art. Sites such as Biocompare at http://www.biocompare.com/abmatrix.asp?antibody=y provide a useful tool to anyone skilled in the art to locate existing antibodies against any of the proteins provided according to the present invention.

Antibodies against the diagnostic proteins according to the present invention can be used in standard techniques such as Western blotting or immunohistochemistry to quantify the level of expression of the proteins of the diagnostic airway proteome.

Immunohistochemical applications include assays, wherein increased presence of the protein can be assessed, for example, from a saliva sample.

The immunohistochemical assays according to the present invention can be performed using methods utilizing solid supports. The solid support can be a any phase used in performing immunoassays, including dipsticks, membranes, absorptive pads, beads, microtiter wells, test tubes, and the like. Preferred are test devices which may be conveniently used by the testing personnel or the patient for self-testing, having minimal or no previous training. Such preferred test devices include dipsticks, membrane assay systems as described in U.S. Pat. No. 4,632,901. The preparation and use of such conventional test systems is well described in the patent, medical, and scientific literature. If a stick is used, the anti-protein antibody is bound to one end of the stick such that the end with the antibody can be dipped into the solutions as described below for the detection of the protein. Alternatively, the samples can be applied onto the antibody-coated dipstick or membrane by pipette or dropper or the like.

The antibody against proteins encoded by the diagnostic airway transcriptome (the "protein") can be of any isotype, such as IgA, IgG or IgM, Fab fragments, or the like. The antibody may be a monoclonal or polyclonal and produced by methods as generally described, for example, in Harlow and Lane, Antibodies, A Laboratory Manual, Cold Spring Harbor Laboratory, 1988, incorporated herein by reference. The antibody can be applied to the solid support by direct or indirect means. Indirect bonding allows maximum exposure of the protein binding sites to the assay solutions since the sites are not themselves used for binding to the support. Preferably, polyclonal antibodies are used since polyclonal antibodies can recognize different epitopes of the protein thereby enhancing the sensitivity of the assay.

The solid support is preferably non-specifically blocked after binding the protein antibodies to the solid support. Non-specific blocking of surrounding areas can be with whole or derivatized bovine serum albumin, or albumin from other animals, whole animal serum, casein, non-fat milk, and the like.

The sample is applied onto the solid support with bound protein-specific antibody such that the protein will be bound to the solid support through said antibodies. Excess and unbound components of the sample are removed and the solid support is preferably washed so the antibody-antigen complexes are retained on the solid support. The solid support may be washed with a washing solution which may contain a detergent such as Tween-20, Tween-80 or sodium dodecyl sulfate.

After the protein has been allowed to bind to the solid support, a second antibody which reacts with protein is applied. The second antibody may be labeled, preferably with a visible label. The labels may be soluble or particulate and may include dyed immunoglobulin binding substances, simple dyes or dye polymers, dyed latex beads, dye-containing liposomes, dyed cells or organisms, or metallic, organic, inorganic, or dye solids. The labels may be bound to the protein antibodies by a variety of means that are well known in the art. In some embodiments of the present invention, the labels may be enzymes that can be coupled to a signal producing system. Examples of visible labels include alkaline phosphatase, beta-galactosidase, horseradish peroxidase, and biotin. Many enzyme-chromogen or enzyme-substrate-chromogen combinations are known and used for enzyme-linked assays. Dye labels also encompass radioactive labels and fluorescent dyes.

Simultaneously with the sample, corresponding steps may be carried out with a known amount or amounts of the protein and such a step can be the standard for the assay. A sample from a healthy non-smoker can be used to create a standard for any and all of the diagnostic airway transcriptome encoded proteins.

The solid support is washed again to remove unbound labeled antibody and the labeled antibody is visualized and quantified. The accumulation of label will generally be assessed visually. This visual detection may allow for detection of different colors, for example, red color, yellow color, brown color, or green color, depending on label used. Accumulated label may also be detected by optical detection devices such as reflectance analyzers, video image analyzers and the like. The visible intensity of accumulated label could correlate with the concentration of C-reactive protein in the sample. The correlation between the visible intensity of accumulated label and the amount of the protein may be made by comparison of the visible intensity to a set of reference standards. Preferably, the standards have been assayed in the same way as the unknown sample, and more preferably alongside the sample, either on the same or on a different solid support.

The concentration of standards to be used can range from about 1 mg of protein per liter of solution, up to about 50 mg of protein per liter of solution. Preferably, several different concentrations of an airway transcriptome encoded protein are used so that quantification of the unknown by comparison of intensity of color is more accurate.

For example, the present invention provides a method for detecting risk of developing lung cancer in a subject exposed to cigarette smoke comprising measuring the level of 1-97 proteins encoded by the airway transcriptome in a biological sample of the subject. Preferably at least one, still more preferably at least two, still more preferably at least three, and still more preferably at least 4-10, or more of the proteins encoded by the airway transcriptome in a biological sample of the subject are analyzed. The method comprises binding an antibody against one or more of the proteins encoded by the airway transcriptome (the "protein") to a solid support chosen from the group consisting of dip-stick and membrane; incubating the solid support in the presence of the sample to be analyzed under conditions where antibody-antigen complexes form; incubating the support with an anti-protein antibody conjugated to a detectable moeity which produces a signal; visually detecting said signal, wherein said signal is proportional to the amount of protein in said sample; and comparing the signal in said sample to a standard, wherein a difference in the amount of the protein in the sample compared to said standard of at least one, preferably at least two, still more preferably at least 3-5, still more preferably at least 5-10, proteins is indicative of an increased risk of developing lung cancer. The standard levels are measured to indicate expression levels in a normal airway exposed to cigarette smoke, as exemplified in the smoker transcript pattern shown, for example on Figure 5.

The assay reagents, pipettes/dropper, and test tubes may be provided in the form of a kit. Accordingly, the invention further provides a test kit for visual detection of one or more proteins encoded by the airway transcriptome, wherein detection of a level that differs from a pattern in a control individual is considered indicative of an increased risk of developing lung disease in the subject. The test kit comprises one or more solutions containing a known concentration of one or more proteins encoded by the airway transcriptome (the "protein") to serve as a standard; a solution of a anti-protein antibody bound to an enzyme; a chromogen which changes color or shade by the action of the enzyme; a solid support chosen from the group consisting of dip-stick and membrane carrying on the surface thereof an antibody to the protein.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, New York, Gait, "Oligonucleotide Synthesis: A Practical Approach" 1984, IRL Press, Lond*on,* Nelson and Cox (2000), Lehninger, Principles of Biochemistry 3rd Ed., W.H. Freeman Pub., New York, NY and Berg et al. (2002) Biochemistry, 5th Ed., W.H. Freeman Pub., New York, NY, all of which are herein incorporated in their entirety by reference for all purposes.

The methods of the present invention can employ solid substrates, including arrays in some preferred embodiments. Methods and techniques applicable to polymer (including protein) array synthesis have been described in U.S.S.N 09/536,841, WO 00/58516, U.S. Patents Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication Number WO 99/36760) and PCT/US01/04285, which are all incorporated herein by reference in their entirety for all purposes.

Patents that describe synthesis techniques in specific embodiments include U.S. Patents Nos. 5,412,087, 6,147,205, 6,262,216, 6,310,189, 5,889,165, and 5,959,098. Nucleic acid arrays are described in many of the above patents, but the same techniques are applied to polypeptide and protein arrays.

Nucleic acid arrays that are useful in the present invention include, but are not limited to those that are commercially available from Affymetrix (Santa Clara, CA) under the brand name GeneChip7. Example arrays are shown on the website at affymetrix.com.

The present invention also contemplates many uses for polymers attached to solid substrates. These uses include gene expression monitoring, profiling, library screening, genotyping and diagnostics. Examples of gene expression monitoring, and profiling methods are shown in U.S. Patents Nos. 5,800,992, 6,013,449, 6,020,135, 6,033,860, 6,040,138, 6,177,248 and 6,309,822. Examples of genotyping and uses therefore are shown in USSN 60/319,253, 10/013,598, and U.S. Patents Nos. 5,856,092, 6,300,063, 5,858,659, 6,284,460, 6,361,947, 6,368,799 and 6,333,179. Other examples of uses are embodied in U.S. Patents Nos. 5,871,928, 5,902,723, 6,045,996, 5,541,061, and 6,197,506.

The present invention also contemplates sample preparation methods in certain preferred embodiments. Prior to or concurrent with expression analysis, the nucleic acid sample may be amplified by a variety of mechanisms, some of which may employ PCR. *See,* e.g., PCR Technology Principles and Applications for DNA Amplification (Ed. H.A. Erlich, Freeman Press, NY, NY, 1992); PCR Protocols: A Guide to Methods and Applications (Eds. Innis, et al., Academic Press, San Diego, CA, 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Patent Nos. 4,683,202, 4,683,195, 4,800,159 4,965,188,and 5,333,675, and each of which is incorporated herein by reference in their entireties for all purposes. The sample may be amplified on the array. See, for example, U.S Patent No 6,300,070 and U.S. patent application 09/513,300, which are incorporated herein by reference.

Other suitable amplification methods include the ligase chain reaction (LCR) *(e.g.,* Wu and Wallace, Genomics 4, 560 (1989), Landegren et al., Science 241, 1077 (1988) and Barringer et al. Gene 89:117 (1990)), transcription amplification (Kwoh et al., Proc. Natl. Acad. Sci. USA 86, 1173 (1989) and WO88/10315), self-sustained sequence replication (Guatelli et al., Proc. Nat. Acad. Sci. USA, 87, 1874 (1990) and WO90/06995), selective amplification of target polynucleotide sequences (U.S. Patent No 6,410,276), consensus sequence primed polymerase chain reaction (CP-PCR) (U.S. Patent No 4,437,975), arbitrarily primed polymerase chain reaction (AP-PCR) (U.S. Patent No 5, 413,909, 5,861,245) and nucleic acid based sequence amplification (NABSA). (*See,* US patents nos. 5,409,818, 5,554,517, and 6,063,603, each of which is incorporated herein by reference). Other amplification methods that may be used are described in, U.S. Patent Nos. 5,242,794, 5,494,810, 4,988,617 and in USSN 09/854,317, each of which is incorporated herein by reference.

Additional methods of sample preparation and techniques for reducing the complexity of a nucleic sample are described, for example, in Dong et al., Genome Research 11, 1418 (2001), in U.S. Patent No 6,361,947, 6,391,592 and U.S. Patent application Nos. 09/916,135, 09/920,491, 09/910,292, and 10/013,598.

Methods for conducting polynucleotide hybridization assays have been well developed in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y, 1989); Berger and Kimmel Methods in Enzymology, Vol. 152, Guide to Molecular Cloning Techniques (Academic Press, Inc., San Diego, CA, 1987); Young and Davism, P.N.A.S, 80: 1194 (1983). Methods and apparatus for carrying out repeated and controlled hybridization reactions have been described, for example, in US patent 5,871,928, 5,874,219, 6,045,996 and 6,386,749, 6,391,623 each of which are incorporated herein by reference

The present invention also contemplates signal detection of hybridization between ligands in certain preferred embodiments. See, for example, U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in provisional U.S. Patent application 60/364,731 and in PCT Application PCT/US99/06097 (published as WO99/47964), each of which also is hereby incorporated by reference in its entirety for all purposes.

Examples of methods and apparatus for signal detection and processing of intensity data are disclosed in, for example, U.S. Patents Numbers 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Patent application 60/364,731 and in PCT Application PCT/US99/06097 (published as WO99/47964), each of which also is hereby incorporated by reference in its entirety for all purposes.

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The present invention also makes use of various computer program products and software for a variety of purposes, such as probe design, management of data, analysis, and instrument operation. See, for example, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention may have preferred embodiments that include methods for providing genetic information over networks such as the Internet as shown in, for example, U.S. Patent applications 10/063,559, 60/349,546, 60/376,003, 60/394,574, 60/403,381.

Throughout this specification, various aspects of this invention are presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. In addition, the fractional ranges are also included in the exemplified amounts that are described. Therefore, for example, a range between 1-3 includes fractions such as 1.1, 1.2, 1.3, 1.4, 1.5, 1.6, etc.

The present invention has many preferred embodiments and relies on many patents, applications and other references for details known to those of the art. Therefore, when a patent, application, or other reference is cited or repeated throughout the specification, it should be understood that it is incorporated by reference in its entirety for all purposes as well as for the proposition that is recited.

### EXAMPLE 1

Primary lung tumors and histologically normal lung tissue were collected from the tumor bank of Brigham and Women's Hospital. Research specimens were snap frozen on dry ice and stored at - 140°C. Each sample was accompanied by an adjacent section embedded in Optimum Cutting Temperature Compound for histological confirmation. The thoracic surgery clinical data-base was abstracted for details of smoking history, clinical staging and other demographic details. From the tumor bank, six cases of adenocarcinoma in life-time never smokers were selected and six cases of adenocarcinoma from cigarette smokers were then chosen for comparison by matching for the following criteria in a descending hierarchy of priority: (1) cell type; (2) histological stage of differentiation; (3) pathologic TNM stage; and (4) patient age (Table 1). All of the subjects except for one smoker were female. The collection of anonymous discarded tumor specimens was approved by the Brigham and Women's Institutional Review Board Hospital and the study was approved by the Human Studies Committee of Boston University Medical Center. Once the cases were selected, specimens and clinical data were de-identified in accordance with the discarded tissue protocol governing the study; thus, linkage of each paired tumor and normal tissue sample with specific additional clinical characteristics other than smoking status, cell type, differentiation and gender was not possible.

Histological sections were reviewed by a pathologist, blinded to original pathological diagnosis. Tumor histology agreed in all cases and the mean percentage of tumor in each sample was 60%. DNA was extracted from tumor and non-involved samples using QIAamp Tissue Kit (Qiagen, Valencia, CA). LOH studies were performed using fluorescent microsatellite LOH analysis as described previously (Powell CA, et al., Clin. Cancer Res., 5:2025-34 (1999)). Tumor and normal lung DNA templates from samples were amplified with a panel of 52 fluorescent PCR primers from ten chromosomal regions that have been reported to harbor lung cancer tumor suppressor genes or have demonstrated LOH in lung tumors or bronchial epithelium of cigarette smokers. Based on our prior studies and results of other investigators using fluorescent methods to detect LOH, we defined LOH as a >20% change in normalized allele height ratio (Fig. 3) (Liloglou T, et al., Cancer Res., 61:1624-1628 (2001); Liloglou T, et al., Int. J. Oncol., 16:5-14 (2000)). All instances of LOH were verified by repetition and the mean allele height ratio was used for data analysis. LOH was measured by comparing tumor DNA to nonmalignant lung DNA rather than to lymphocyte DNA, which was unavailable for this study. Thus, LOH represented allelic loss between two somatic sites in the same lung, rather than between tumor tissue and constitutional genomic DNA.

The extent of LOH was expressed as fractional allelic loss (FAL) which equals the number of primers with LOH per template/number of informative primers. Fisher exact test and x 2 were used to determine the difference in FAL in smokers compared with nonsmokers.

**Results.** All tumors demonstrated LOH in at least one microsatellite on each of the ten chromosomal arms evaluated in this study (Table 2). With respect to nonmalignant lung epithelium, LOH was more frequent in the tumors of nonsmokers than in those of smokers (Fig. 4). FAL ranged from 6 to 93% with a mean of 46%, in nonsmokers, and from 2 to 60% with a mean of 28%, in smokers (P < 0.05). In the pairwise comparison of nonsmokers and clinically matched smokers, LOH was more frequent in five of six nonsmokers.

Chromosomes 10p, 9p, and 5q were the most frequent sites of LOH in nonsmokers'tumors while 9p and 5q were the most frequent sites in smokers. Increased FAL in nonsmokers was most pronounced at five chromosomal arms: 3p, 8p, 9p, 10p, and 18q with FAL ranging from 55 to 87%. These microsatellites harbor several known or candidate tumor suppressor genes such as FHIT, DLCL (Daigo Y, et al., Cancer Res., 59:1966-1972 (1999)), RASSF1 (Dammann R, et al., Nat. Genet., 25:315-319 (2000)) (chromosome 3p), PRK (Li B, et al., J. Biol. Chem., 271:19402-19408 (1996) (chromosome 8p), p16 (chromosome 9p), SMAD2 and SMAD4 (Takei K, et al., Cancer Res., 58:3700-3705 (1998)) (chromosome 18q).

In most tumors, there were instances of microsatellites demonstrating LOH interspersed with microsatellites that retained heterozygosity (see chromosome 1p in subject S3, Table 2). This pattern of discontinuous allelic loss was evident on all chromosomes that were evaluated, and is considered a potential mutational signature of lung carcinogenesis attributable to mitotic recombination (Wistuba, II, Behrens C, et al., Cancer Res., 60:19491960 (2000)). However, in other instances there was LOH at a number of contiguous loci suggesting larger chromosomal deletions (see chromosome 3p in subject NS3, Table 2). This was particularly true on 3p, a fragile site previously found to be involved in smokers with and without tumors.

### EXAMPLE 2

**Methods.** Samples of epithelial cells, obtained by brushing airway surfaces, were obtained from intra- and extra-pulmonary airways in 11 normal non-smokers (NS), 15 smokers without lung cancer (S), and 9 smokers with lung cancer (SC). 5-10 ug of RNA was extracted using standard trizol-based methods, quality of RNA was assayed in gels, and the RNA was processed using standard protocols developed by Affymetrix for the U133 human array. Expression profiles, predictive algorithms, and identification of critical genes are made using bioinformatic methods.

Results. There are 5169 genes in the NS Transcriptome, 4960 genes in the S Transcriptome, and 5518 genes in the SC Transcriptome. There are 4344 genes in common between the 3 Transcriptomes. There are 327 unique genes in the NS Transcriptome, 149 unique genes in the S Transcriptome, and 551 unique genes in the SC Transcriptome. Figs. 1A-1F show a list of genes which are differentially expressed in smokers and non-smokers. Figs. 2A-2B show a list of genes which are differentially expressed in smokers and smokers with lung cancer. T-test statistical results are shown.

### EXAMPLE 3

There are approximately 1.25 billion daily cigarette smokers in the world(1). Cigarette smoking is responsible for 90% of all lung cancers, the leading cause of cancer deaths in the US and the world(2, 3). Smoking is also the major cause of chronic obstructive pulmonary disease (COPD), the fourth leading cause of death in the US(4). Despite the well-established causal role of cigarette smoking in lung cancer and COPD, only 10-20% of smokers actually develop these diseases(5). There are few indicators of which smokers are at highest risk for developing either lung cancer or COPD, and it is unclear why individuals remain at high risk decades after they have stopped smoking(6).

Given the burden of lung disease created by cigarette smoking, surprisingly few studies(7, 8) have been done in humans to determine how smoking affects the epithelial cells of the pulmonary airways that are exposed to the highest concentrations of cigarette smoke or what smoking-induced changes in these cells are reversible when subjects stop smoking. With the two exceptions noted above, which examine a specific subset of genes in humans, studies investigating the effects of tobacco on airway epithelial cells have been in cultured cells, in human alveolar lavage samples in which alveolar macrophages predominate, or in rodent smoking models (summarized in Gebel et al(9)).

A number of recent studies have used DNA microarray technology to study normal and cancerous whole lung tissue and have identified molecular profiles that distinguish the various subtypes of lung cancer as well as predict clinical outcome in a subset of these patients(10-13).

Based on the concept that genetic alterations in airway epithelial cells of smokers represent a "field defect"(14, 15), we obtained human epithelial cells at bronchoscopy from brushings of the right main bronchus proximal to the right upper lobe of the lung, and defined profiles of gene expression in these cells using the U133A GeneChip® array (Affymetrix Inc., Santa Clara, CA). We here describe the subset of genes expressed in large airway epithelial cells (the airway transcriptome) of healthy never smokers, thereby gaining insights into the biological functions of these cells.

Surprisingly, we identified a large number of genes whose expression is altered by cigarette smoking, defined genes whose expression correlates with cumulative pack years of smoking, and identified genes whose expression does and does not return to normal when subjects discontinue smoking.

In addition, we identified a subset of smokers who were "outliers" expressing some genes in a fashion that significantly differed from most smokers. One of these "outliers" developed lung cancer within 6 months of expression profiling, suggesting that gene expression profiles of smokers with cancer differ from that of smokers without lung cancer.

### Materials and Methods:

**Study Population and Sample Collection:** We recruited non-smoking and smoking subjects (n=93) to undergo fiberoptic bronchoscopy at Boston Medical Center between November 2001 and June 2003. Non-smoking volunteers with significant environmental cigarette exposure and subjects with respiratory symptoms or regular use of inhaled medications were excluded. For each subject, a detailed smoking history was obtained including number of pack-years, number of packs per day, age started, age quit, and environmental tobacco exposure.

All subjects in our study underwent fiberoptic bronchoscopy between November 2001 and June 2003. Risks from the procedure were minimized by carefully screening volunteers (medical history, physical exam, chest X-ray, spirometry and EKG), by minimizing topical lidocaine anesthesia, and by monitoring the EKG and SaO₂ throughout the procedure. After passage of the bronchoscope through the vocal cords, brushings were obtained via 3 cytobrushes (CELEBRITY Endoscopy Cytology Brush, Boston Scientific, Boston, MA) from the right upper lobe bronchus.

Bronchial airway epithelial cells were obtained from brushings of the right mainstem bronchus taken during fiberoptic bronchoscopy using an endoscopic cytobrush (CELEBRITY Endoscopy Cytology Brush, Boston Scientific, Boston, MA). The brushes were immediately placed in TRIzol reagent (Invitrogen, Carlsbad, CA) after removal from the bronchoscope and kept at -80° C until RNA isolation was performed. Any other RNA protection protocol known to one skilled in the art can also be used. RNA was extracted from the brushes using TRIzol Reagent (Invitrogen) as per the manufacturer protocol, with a yield of 8-15 µg of RNA per patient. Other methods of RNA isolation or purification can be used to isolate RNA from the samples. Integrity of the RNA was confirmed by running it on a RNA denaturing gel. Epithelial cell content of representative bronchial brushing samples was quantified by cytocentrifugation (ThermoShandon Cytospin, Pittsburgh, PA) of the cell pellet and staining with a cytokeratin antibody (Signet, Dedham MA). The study was approved by the Institutional Review Board of Boston University Medical Center and all participants provided written informed consent.

**Microarray Data Acquisition and Preprocessing:** We obtained sufficient quantity of good quality RNA for microarray studies from 85 of the 93 subjects recruited into our study. Total RNA was processed, labeled, and hybridized to Affymetrix HG-U133A GeneChips containing approximately 22,500 human genes, any other type of nucleic acid or protein array may also be used. Six to eight µg of total RNA from bronchial epithelial cells was converted into double-stranded cDNA with the SuperScript II reverse transcriptase (Invitrogen) using an oligo-dT primer containing a T7 RNA polymerase promoter (Genset, Boulder, CO). The ENZO Bioarray RNA transcript labeling kit (Affymetrix) was used for in vitro transcription of the purified double stranded cDNA. The biotin-labeled cRNA was purified using the RNeasy kit (Qiagen) and fragmented into approximately 200 base pairs by alkaline treatment (200mM Tris-acetate, pH 8.2, 500mM potassium acetate, 150mM magnesium acetate). Each verified cRNA sample was then hybridized overnight onto the Affymetrix HG-U133A array and confocal laser scanning (Agilent) was then performed to detect the streptavidin-labeled fluor. A single weighted mean expression level for each gene along with a p_{(derection)} -value (which indicates whether the transcript was reliably detected) was derived using Microarray Suite 5.0 software (Affymetrix, SantaClara, California).

Using a one-sided Wilcoxon Signed rank test, the MAS 5.0 software also generated a detection p-value (p_{(detection)} -value) for each gene which indicates whether the transcript was reliably detected. We scaled the data from each array in order to normalize the results for inter-array comparisons. Microarray data normalization was accomplished in MAS 5.0 , where the mean intensity for each array (top and bottom 2% of genes excluded) was corrected (by a scaling factor) to a set target intensity of 100. The list of genes on this array is available at http://www.affymetrix.com/analysis/download center.affx.

Arrays of poor quality were excluded based on several quality control measures. Each array's scanned image was required to be free of any significant artifacts and the bacterial genes spiked into the hybridization mix had to have a p_{(detection)} -value below 0.05 (called present). If an array passed this criteria, it was evaluated based on three other quality measures: the 3' to 5' ratio of the intensity for Glyceraldehyde-3-phosphate dehydrogenase (GAPDH), the percent of genes detected as present, and the percent of "outlier" genes as determined by a computational algorithm we developed (see http://pulm.bumc.hu.edu/aged/supplemental.html for further details, which are herein incorporated by reference).

In addition to the above set of rules, one further quality control measure was applied to each array. While cytokeratin stains of selected specimens reveal that approximately 90% of nucleated cells are epithelial, we developed a gene filter to exclude specimens potentially contaminated with inflammatory cells. A group of genes on the U133A array was identified that should be expressed in bronchial epithelial cells as well as a list of genes that are specific for various lineages of white blood cells and distal alveolar epithelial cells (see Figures 13 and 14). Arrays whose 90^{th} percentile for the p_{(detection)}-value was more than 0.05 for genes that should be detected in epithelial cells or whose 80^{th} percentile p_{(detection)}-value was less than 0.05 for genes that should not be expressed in bronchial epithelial cells were excluded from the study. 10 of the 85 samples were excluded based on the quality control filter and the epithelial content filter described above (see http://pulm.bumc.bu.edu/aged/supplemental.html for details regarding excluded samples).

In addition to filtering out poor quality arrays, a gene filter was applied to remove genes that were not reliably detected. From the complete set of ∼22500 probesets on the U133 array, we filtered out probesets whose p_{(detection)} -value was not less than 0.05 in at least 20% of all samples. 9968 probesets passed our filter and were used in all further statistical analyses for the dataset.

**Microarray Data Analysis:** Clinical information and array data as well as gene annotations are stored in an interactive MYSQL database coded in Perl available at http://pulm.bumc.bu.edu/aged/index.html. All statistical analyses below and within the database were performed using R software version 1.6.2 (available at http://r-project.org)*.* The gene annotations used for each probe set were from the October 2003 NetAffx HG-U133A Annotation Files.

Technical, spatial (right and left bronchus from same subject) and temporal (baseline and at 3 months from same subject) replicates were obtained from selected subjects for quality control. Pearson correlations were calculated for technical, spatial and temporal replicate samples from the same individual. RNA isolated from the epithelial cells of one patient was divided in half and processed separately as detailed in the methods for the technical replicates (data not shown). Different brushings were obtained from the right and left airways of the same patient and processed separately for the spatial replicates (Figure 8A). Brushings of the right airway were obtained approximately 3 months apart and processed separately for the temporal replicates (Figure 8B).

In addition to the correlation graphs in Figures 8A and 8B, two systematic approaches were implemented to assess the variability between replicates versus the variability between unrelated samples. Pearson correlation coefficients were computed between replicates as well as between unrelated samples within a group (never or current smoker) and between groups (never versus current smoker) using the filtered gene list (9968 genes). Figure 16 reports the mean R squared values for each of the four comparisons. The results demonstrate that the mean correlation among replicates is higher than between two unrelated samples, and that the within group correlations between unrelated samples are higher than the between group correlations between unrelated samples.

The second approach uses a different methodology, but yields similar results to those described in Figure 16. For each of the 9968 genes, a differential gene expression ratio was computed between replicate samples and between all possible combinations of two unrelated samples (Lenburg M, Liou L, Gerry N, Frampton G, Cohen H & Christman M. (2003) BMC Cancer 3, 31). A histogram of the log base 2 ratio values or fold changes is displayed in Figure 8C. The number of fold changes computed for the replicate samples is less than the number of fold changes computed for unrelated samples, therefore, the frequencies in the histogram are calculated as a percent of the total fold changes calculated. As expected, the histogram clearly shows that there is less variability among the replicate samples. In the replicate samples there is a higher frequency of genes having a fold change close to or equal to one compared to unrelated samples.

An unsupervised analysis of the Microarray data was performed by hierarchal clustering the top 1000 most variable probe sets (determined by coefficient of variation) across all samples using log transformed z-score normalized data. The analysis was performed using a Pearson correlation (uncentered) similarity metric and average linkage clustering with CLUSTER and TREEVIEW software programs obtained at http://rana.lbl.gov/EisenSoftware.htm (see Figure 9).

The normal large airway transcriptome was defined by the genes whose median p_{(detection)}-value was less than 0.05 across all 23 healthy never smokers (7119 genes expressed across majority of subjects), as well as a subset of these 7119 genes whose p_{(detection)}-value was less than 0.05 in all 23 subjects (2382 genes expressed across all subjects). The coefficient of variation for each gene in the transcriptome was calculated as the standard deviation divided by the mean expression level multiplied by 100 for that gene across all nonsmoking individuals. In order to identify functional categories that were over- or underrepresented within the airway transcriptome, the GOMINER software (16) was used to functionally classify the genes expressed across all nonsmokers (2382 probesets) by the molecular functions categories within Gene Ontology (GO). Multiple linear regressions were performed on the top ten percent most variable probesets (712 probesets, as measured by the coefficient of variation) in the normal airway transcriptome (7119 probesets) in order to study the effects of age, gender, and race on gene expression.

It should be noted, that genes expressed at low levels are not necessarily accurately detected by microarray technology. The probe sets which define the normal airway transcriptome, therefore, will represent genes which are expressed at a measurable level in either the majority or all of the nonsmoking healthy subjects. One of the limitations to this approach, however, is that we will be excluding genes expressed at low levels in the normal airway transcriptome.

Multiple linear regressions were performed on the top ten percent most variable genes (712 genes, as measured by the coefficient of variation, defined here as sd/mean *100) in the normal airway transcriptome (7119 genes) in order to study the effects of age, gender, and race on gene expression (see Figures 17A-17C) using R statistical software version 1.6.2. Figure 10 shows that the majority of genes in the normal airway transcriptome have coefficients of variation below 50. As a result, we choose to focus on a smaller subset of the 7119 genes, specifically the top ten percent most variable genes, in order to explore whether or not various demographic variables could explain the patterns of gene expression. The coefficients of variation for the top ten percent most variable genes ranged from 50.78 to 273.04. A general linear model was used to explore the relationship between gene expression and age (numerical variable), race (categorical variable with two groups Caucasian or Other), and gender (categorical variable). The model included the three main effects plus the three possible two-way interactions. Models having a p-value less than 0.01 (83 genes) were chosen for further analysis. For each of these models, the following diagnostic plots were assessed: residuals versus the fitted values plot, normal Q-Q plot, and Cook's distance plot. Based on the graphs, 13 models were removed because the residuals were not normally distributed or had unequal variance. The regression results for the remaining 70 genes are included in Figures 17A-17C as well as the p-values for the significant regressors (p<=0.01). The age:race interaction term is absent from the table because none of the models had p-values less than 0.01 for this term.

To examine the effect of smoking on the airway, a two-sample t-test was used to test for genes differentially expressed between current smokers (n=34) and never smokers (n=23). In order to quantify how well a given gene's expression level correlates with number of pack-years of smoking among current smokers, Pearson correlation coefficients were calculated (see supplementary information). For multiple comparison correction, a permutation test was used to assess the significance of our p-value threshold for any given gene's comparison between two groups (p₍ₜ₋ₜₑₛₜ₎-value) or between a clinical variable (p_{(correlation)}-value) (see supporting information for details). In order to further characterize the behavior of current smokers, two-dimensional hierarchical clustering of all never smokers and current smokers using the genes that were differentially expressed between current vs. never smokers was performed. Hierarchical clustering of the genes and samples was performed using log transformed z-score normalized data using a Pearson correlation (uncentered) similarity metric and average linkage clustering using CLUSTER and TREEVIEW software programs.

Multidimensional scaling and principal component analysis were used to characterize the behavior of former smokers (n=18) based on the set genes differentially expressed between current and never smokers using Partek 5.0 software (http://www.partek.com). In addition, we executed an unsupervised hierarchical clustering analysis of all 18 former smokers according to the expression of the genes differentially expressed between current and never smoker. In order to identify genes irreversibly altered by cigarette smoking, we performed a t-test between former smokers (n=18) and never smokers (n=23) across the genes that were considered differentially expressed between current and never smokers. Coefficients of variation (sd/mean * 100) were computed across never, former, and current smoker subjects for each of the 9968 probesets. The top 1000 most variable probesets (%CV > 56.52) were selected and hierarchical clustering of these probesets and samples was performed using log transformed z-score normalized data using a Pearson correlation (uncentered) similarity metric and average linkage clustering using CLUSTER and TREEVIEW software programs obtained at http://rana.lbl.gov/EisenSoftware.htm. The clustering dendogram of the samples is displayed in Figure 9. The samples do not cluster according to their classification of never, former, or current smokers, and therefore, a supervised approach was needed (see below). In addition, the dendogram does not reveal a clustering pattern that is related to technical variation in the processing of the samples. Table 2 below List of genes whose expression did not return to normal even after about 20 years of smoking:

**Table 2**

| **Affymetrix ID** | **Gene Symbol** |
|---|---|
| **213455_at** | LOC92689 |
| **823_at** | CX3CL1 |
| **204755_x_at** | HLF |
| **204058_at** | ME1 |
| **217755_at** | HN1 |
| **207547_s_at** | TU3A |
| **211657_at** | CEACAM6 |
| **213629_x_at** | MT1F |
| **214106_s_at** | GMDS |
| **207222_at** | PLA2G10 |
| **204326_x_at** | MT1X |
| **201431_s_at** | DPYSL3 |
| **204754_at** | HLF |
| **208581_x_at** | MT1X |
| **215785_s_at** | CYFIP2 |

Given the invasive nature of the bronchoscopy procedure, we were unable to recruit age-, race- and gender-matched patients for the smoker vs. nonsmoker comparison. Due to baseline differences in age, gender, and race between never and current smoker groups (see Figure 15), we performed an ANCOVA to test the effect of smoking status (never or current) on gene expression while controlling for the effects of age (the covariate). In addition, a two way ANOVA was performed to test the effect of smoking status (never or current) on gene expression while controlling for the fixed effects of race (encoded as three racial groups: Caucasian, African American, and other) or gender and the interaction terms of status:race or status:gender. Both the ANCOVA and two-way ANOVA were performed with Partek 5.0 software.

**Genes that distinguish smokers with and without cancer.** In order to identify airway gene expression profiles diagnostic of lung cancer, a two-sample t-test was performed to test for genes differentially expressed between smokers with lung cancer (n=23) and smokers without lung cancer (n=45). 202 genes were differentially expressed between the groups at p < 0.001 (see table 1). In order to correct for multiple comparisons, we calculated a q-value (Storey JD & Tibshirani R (2003). Proc. Natl. Acad. Sci. U. S. A 100, 9449-9445) for each gene, which represents the proportion of false positives present in the group of genes with smaller p-values than the gene.

**Outlier genes among current smokers:** Among airway epithelial genes altered by cigarette smoke, there are a number of genes expressed at extremely high or low levels among a subset of current smokers. In order to identify these "outlier genes, we performed a Grubbs test on the 320 genes differentially expressed between current (n=34) and never (n=23) smokers at p<0.001. Nine genes were found to be outliers in 3 or more of the current smokers (see table 2). These divergent patterns of gene expression in a small subset of smokers represent a failure to mount an appropriate response to cigarette exposure and may be linked to increased risk for developing lung cancer. As a result, these "outlier" genes can thus serve as biomarkers for susceptibility to the carcinogenic effects of cigarette smoke.

**Quantitative PCR Validation:** Real time PCR (QRT-PCR) was used to confirm the differential expression of a select number of genes. Primer sequences were designed with Primer Express software (Applied Biosystems, Foster City, CA). Forty cycles of amplification, data acquisition, and data analysis were carried out in an ABI Prism 7700 Sequence Detector (Applied Biosystems, Foster City, CA). All real time PCR experiments were carried out in triplicate on each sample.

In further detail, real time PCR (QRT-PCR) primer sequences were designed with Primer Express software (Applied Biosystems, Foster City, Calif.) based on alignments of candidate gene sequences. RNA samples (500ng of residual sample from array experiment) were treated with DNAfree (Ambion), as per the manufacturer protocol, to remove contaminating genomic DNA. Total RNA was reverse transcribed using Superscript II (Gibco). Five microliters of the reverse transcription reaction was added to 45 µl of SYBR Green PCR master mix (Applied Biosystems). Forty cycles of amplification, data acquisition, and data analysis were carried out in an ABI Prism 7700 Sequence Detector (PE Applied Biosystems). Threshold determinations were automatically performed by the instrument for each reaction. The cycle at which a sample crosses the threshold (a PCR cycle where the fluorescence emission exceeds that of nontemplate controls) is called the threshold cycle, or CT. A high CT value corresponds to a small amount of template DNA, and a low CT corresponds to a large amount of template present initially. All real time PCR experiments were carried out in triplicate on each sample (mean of the triplicate shown). Data from the QRT-PCR for 5 genes that changed in response to cigarette exposure along with the microarray results for these genes is shown in Figures 12A-12E.

**Additional Information:** Additional information from this study including the raw image data from all microarray samples (.DAT files), expression levels for all genes in all samples (stored in a relational database), user-defined statistical and graphical analysis of data and clinical data on all subjects is available at http://pulm.bumc.bu.edu/aged/. Data from our microarray experiments has also been deposited in NCBI's Gene Expression Omnibus under accession GSE994.

**Results and Discussion: Study Population and replicate samples:** Microarrays from 75 subjects passed the quality control filters described above and are included in this study. Demographic data on these subjects, including 23 never smokers, 34 current smokers, and 18 former smokers, is presented in Figure 15. Bronchial brushings yielded 90% epithelial cells, as determined by cytokeratin staining, with the majority being ciliated cells. Samples taken from the right and left main bronchi in the same individual were highly reproducible with an R² value of 0.92, as were samples from the same individual taken 3 months apart with an R² value of 0.85 (see Figures 8A-8C).

**The Normal Airway Transcriptome:** 7119 genes were expressed at measurable levels in the majority of never smokers and 2382 genes were expressed in all of the 23 healthy never smokers. There was relatively little variation in expression levels of the 7119 genes; 90% had a coefficient of variation (SD/mean) of <50% (see Figure 10). Only a small part of the variation between subjects could be explained by age, gender or race on multiple linear regression analysis (see Figures 17A-17C).

Table 1 depicts the GOMINER molecular functions(16) of the 2382 genes expressed in large airway epithelial cells of all healthy never smokers. Genes associated with oxidant stress, ion and electron transport, chaperone activity, vesicular transport, ribosomal structure and binding functions are over-represented. Genes associated with transcriptional regulation, signal transduction, pores and channels are under-represented as well as immune, cytokine and chemokine genes. Upper airway epithelial cells, at least in normal subjects, appear to serve as an oxidant and detoxifying defense system for the lung, but serve few other complex functions in the basal state.

**Table 1: GOMINER molecular functions of genes in airway epithelial cells. Major molecular functional categories and subcategories of 2382 genes expressed in all never smoker subjects. Over- or under-representation of categories is determined using Fisher's Exact Test. The null hypothesis is that the number of genes in our flagged set belonging to a category divided by the total number of genes in the category is equal to the number of flagged genes NOT in the category divided by the total number of genes NOT in the category. Equivalency in these two proportions is consistent with a random distribution of genes into functional categories and indicates no enrichment or depletion of genes in the category being tested. Categories considered to be statistically (p_{(GO)} <.05) over- or under-represented by GOMINER are shown. Cells/arrays refers to the ratio of the number of genes expressed in epithelial cells divided by the number of genes on U133A array in each functional category. Actual numbers are in parentheses.**

| **Molecular Functions** | **Over represented (cells/array)** | **Under represented (cells/array)** |
|---|---|---|
| ***Binding Activity*** | | |
| RNA binding | 0.76 (273/366) | |
| Translation | 0.72 (72/101) | |
| Transcription | | 0.30 (214/704) |
| GTP binding | 0.55 (106/194) | |
| GTPase | 0.55 (83/152) | |
| G nucleotide | 0.52 (128/246) | |
| Receptor | | 0.20 (79/396) |
| ***Chaperone*** | 0.62 (80/119) | |
| ***Chemokine*** | | 0.24 (10/42) |
| ***Cytokine*** | | 0.20 (39/194) |
| ***Enzyme activity*** | 0.46 (1346/2925) | |
| Oxidoreductase | 0.54 (225/417) | |
| Isomerase | 0.56 (48/82) | |
| ***Signal transduction*** | | 0.29 (490/1716) |
| ***Structural*** | 0.46 (253/548) | |
| ***Transcription regulator*** | | 0.35 (321/917) |
| ***Transporter*** | | |
| Carrier | 0.48 (175/363) | |
| Ion | 0.56 (130/231) | |
| *Anion* | | 0.26 (15/61) |
| *Cation* | 0.64 (116/180 | |
| *Metal* | 0.68 (42/62) | |
| Electron | 0.58 (131/226) | |
| Channel/pore | | 0.16 (43/269) |

**Effects of Cigarette Smoking on the Airway Transcriptome:** Smoking altered the airway epithelial cell expression of a large number of genes. Ninety-seven genes were found to be differentially expressed by t-test between current and never smokers at p < 1.06*10⁻⁵. This p₍ₜ₋ₜₑₛₜ₎-value threshold was selected based on a permutation analysis performed to address the multiple comparison problem inherent in any microarray analysis (see supporting information for further details). We chose a very stringent multiple comparison correction and p₍ₜ₋ₜₑₛₜ₎-value threshold in order to identify a subset of genes altered by cigarette smoking with only a small probability of having a false positive. Of the 97 genes that passed the permutation analysis, 68 (73%) represented increased gene expression among current smokers. The greatest increases were in genes that coded for xenobiotic functions such as CYP1B1 (30 fold) and DBDD (5 fold), antioxidants such as GPX2 (3 fold), and ALDH3A1 (6 fold) and genes involved in electron transport such as NADPH (4 fold). In addition, several cell adhesion molecules, CEACAM6 (2 fold) and claudin 10 (3 fold), were increased in smokers, perhaps in response to the increased permeability that has been found on exposure to cigarette smoke(17). Genes that decreased included TU3A (-4 fold), MMP10 (-2 fold), HLF (-2 fold), and CX3CL1 (-2 fold). In general, genes that were increased in smokers tended to be involved in regulation of oxidant stress and glutathione metabolism, xenobiotic metabolism, and secretion. Expression of several putative oncogenes (pirin, CA12, and CEACAM6) were also increased. Genes that decreased in smokers tended to be involved in regulation of inflammation, although expression of several putative tumor suppressor genes (TU3A, SLIT1 and 2, GAS6) were decreased. Changes in the expression of select genes were confirmed by real time RT-PCR (see Figures 12A-12E).

Figure 5 shows two-dimensional hierarchical clustering of all the current and never smokers based on the 97 genes that are differentially expressed between the two groups (tree for genes not shown). There were three current smokers (patients #56, #147 and #164) whose expression of a subset of genes was similar to that of never smokers. These three smokers, who were similar clinically to other smokers, also segregated in the same fashion when clusters were based on the top 361 genes differentially expressed between never and current smokers (p<0.001). Expression of a number of redox-related and xenobiotic genes was not increased in these 3 smokers (147C, 164C, 56C), and therefore, their profile resembled that of never smokers despite their substantial and continuing exposure to cigarette smoke. Thus, these individuals failed to increase expression of a number of genes that serve as protective detoxification and anti-oxidant genes, potentially putting them a risk of more severe smoking-related damage. Whether or not these differences represent genetic polymorphisms, and whether these individuals represent the 10-15% of smokers who ultimately develop lung cancer is uncertain. However, one of these subjects (147C) subsequently developed lung cancer during one year follow up, suggesting some link between the divergent patterns of gene expression and presence of or risk for developing lung cancer. There was also a subset of four additional current smokers who clustered with current smokers, but did not up-regulate expression of a cluster of predominantly redox/xenobiotic genes to the same degree as other smokers, although none of these smokers had developed lung cancer in six months of follow up. In addition, there is a never smoker (167N) who is an outlier among never smokers and expresses a subset of genes at the level of current smokers. We reviewed this subject's clinical history and were unable to identify any obvious environmental exposures (i.e. second hand smoke exposure) that might explain the divergent pattern of gene expression.

As might be expected, changes in gene expression were also correlated with cumulative cigarette exposure (pack-years). While 159 and 661 genes correlated with cumulative smoking history at p<0.041 and p<0.01 levels respectively (see Figures 18A-18B), only 5 genes correlated with pack-years at the p< 3.1 x10⁻⁶ threshold (based on permutation analysis; see supporting information for details). They include cystatin, which has been shown to correlate with tumor growth and inflammation(18), HBP17 has been shown to enhance FGF growth factor activity(19), and BRD2, which is a transcription factor that acts with E2F proteins to induce a number of cell cycle-related genes(20). Among the genes that were correlated at the p < .0001 level, there were a number of genes that decreased with increasing cumulative smoking history including genes that are involved in DNA repair (RPA1).

Due to baseline differences in age, sex, and race between never and current smoker groups, ANCOVA and 2-way ANOVA were performed to test the effect of smoking status on gene expression while controlling for the effects of age, gender, race and two-way interactions. Many of the genes found to be modulated by smoking in this analysis were also found using the simpler t-test. Age and gender had little effect on gene expression changes induced by smoking, while race appeared to influence the effect of smoking on the expression of a number of genes. The ANOVA analysis controlling for race yielded 16 genes, not included in the set of 97 genes differentially expressed between current and never smokers (see Figures 20A-20B). Given the relatively small sample size for this subgroup analysis, these observations must be confirmed in a larger study but may account in part for the reported increased incidence of lung cancer in African American cigarette smokers(21).

Thus, the general effect of smoking on large airway epithelial cells was to induce expression of xenobiotic metabolism and redox stress-related genes and to decrease expression of some genes associated with regulation of inflammation. Several putative oncogenes were upregulated and tumor suppressor genes were downregulated although their roles, in smoking-induced lung cancer remain to be determined. Risk for developing lung cancer in smokers has been shown to increase with cumulative pack-years of exposure(22), and a number of putative oncogenes correlate positively with pack-years, while putative tumor suppressor genes correlate negatively.

It is unlikely that the alterations we observed in smokers were due to a change in cell types obtained at bronchoscopy. Several dynein genes were expressed at high levels in never smokers in our study, consistent with the predominance of ciliated cells in our samples. The level of expression of various dynein genes, and therefore the balance of cell types being sampled, did not change in smokers. This is consistent with a previous study of antioxidant gene expression in airway epithelial cells from never and current smokers that showed no change in histologic types of cells obtained from smokers(8). Our findings that drug metabolism and antioxidant genes are induced by smoking in airway epithelial cells is consistent with *in vitro* and *in vivo* animal studies (summarized in (9)). The high density arrays used in our studies allowed us to define the effect of cigarette smoking on a large number of genes not previously described as being affected by smoking.

Two sample unequal variance t-tests were performed to find differentially expressed genes between never and current smokers. Due to the presence of multiple comparisons in array data, there is the potential problem of finding genes differentially expressed between the 2 groups when no difference actually exists(Benjamini, Y. & Hochberg, Y. (1995) Journal of the Royal Statistical Society Series B 57, 289-300). Current methods available to adjust for multiple comparisons, such as the Bonferroni correction (where the p ₍ₜ₋ₜₑₛₜ₎-value threshold is divided by the number of hypotheses tested), are often too conservative when applied to microarray data (MacDonald, T. J., Brown, K. M., LaFleur, B., Peterson, K., Lawlor, C., Chen, Y., Packer, R. J., Cogen, P. & Stephan, D. A. (2001) Nat. Genet. 29, 143-152). However, we chose to employ a very stringent multiple comparison correction and p₍ₜ₋ₜₑₛₜ₎-value threshold in order to identify a subset of genes altered by cigarette smoking with only a small probability of having a false positive. The Bonferroni correction controls the probability of committing even one error in all the hypotheses tested; however, the correction assumes independence of the different tests which is unlikely to hold true in the microarray setting where multiple genes are co-regulated (Tusher, V. G., Tibshirani, R. & Chu, G. (2001) Proc. Natl. Acad. Sci. U. S. A 98, 5116-5121). Therefore, we have elected to employ a permutation-based correction (coded in PERL in our database) to assess the significance of the p₍ₜ₋ₜₑₛₜ₎-value for any given gene. The permutation test is similar to the Bonferroni correction in that it controls the probability of finding even one gene by chance in the hypotheses tested, however, a permutation-based correction is data dependent. After calculating a t-test statistic and p₍ₜ₋ₜₑₛₜ)-value for each gene, we permute the group assignments of all samples 1000 times and calculate for each permutation the t-statistic and corresponding p₍ₜ₋ₜₑₛₜ₎-value for each gene. After all permutations are completed, the result is a 9968 (# of genes) by 1000 (# of permutations) matrix of p₍ₜ₋ₜₑₛₜ₎-values. For each permutation, a gene's actual p₍ₜ₋ₜₑₛₜ₎-value is compared to all other permuted p₍ₜ₋ₜₑₛₜ₎-values to determine if the any of the permuted p₍ₜ₋ₜₑₛₜ₎-values is equal to or lower than the actual gene's p₍ₜ₋ₜₑₛₜ₎-value. An adjusted p₍ₜ₋ₜₑₛₜ₎-value is computed for each gene based on the permutation test. The adjusted p₍ₜ₋ₜₑₛₜ₎-value is the probability of observing at least as small a p₍ₜ₋ₜₑₛₜ) -value (in any gene) as the gene's actual p₍ₜ₋ₜₑₛₜ₎ -value in any random permutation. A gene is considered significant if less than 50 out of 1000 permutations (.05) yield a gene with a permuted p₍ₜ₋ₜₑₛₜ₎ -value equal to or lower than the actual gene's p₍ₜ₋ₜₑₛₜ₎ -value.

For our t-test comparing current vs. never smokers, the permuted p₍ₜ₋ₜₑₛₜ₎-value threshold was found to be 1.06*10⁻⁵. Ninety-seven genes were considered differentially expressed between current and never smokers at this threshold. One shortcoming of this methodology is that is impossible to compute all possible permutations of the group assignments for large sample sizes. As a result, we repeated the permutation analysis 15 times yielding an average p₍ₜ₋ₜₑₛₜ₎-value of 1.062*10⁻⁵ (sd=1.52*10⁻⁶). The mean p₍ₜ₋ₜₑₛₜ₎-value was used as a cutoff and yielded a gene list of ninety-seven genes. In this case, the distribution of the data is such that the permuted p₍ₜ₋ₜₑₛₜ₎ -value threshold is slightly less strict than the equivalent Bonferroni cutoff.

By only focusing on the list of 97 genes that pass the p₍ₜ₋ₜₑₛₜ₎ -value threshold of 1.06*10⁻⁵, we recognize that we are ignoring a number of genes differentially expressed between never and current smokers (false negatives), but we wanted to be very confident regarding biological conclusions derived from genes that were considered differentially expressed. A broader list of genes was defined by calculating the q-value for each gene in the analysis as proposed by Storey JD & Tibshirani R (2003). Proc. Natl. Acad. Sci. U. S. A 100, 9449-9445. A given gene's q-value is the proportion of false positives present in the group of genes with smaller p-values than the gene. The q-value of the 97^{th} gene was 0.005, which means that among all 97 t-tests that we designate as significant only 0.5% of them will be false positives. A less strict p₍ₜ₋ₜₑₛₜ₎ -value cutoff of 4.06*10⁻⁴ (q-value = 0.01) yields 261 genes with approximately 3 false positive genes. The q-values were calculated using the program Q-Value which can be downloaded from http://faculty.washington.edu/∼jstorey/qvalue/. Larger lists of genes can be accessed through our database by selecting a less restrictive p₍ₜ₋ₜₑₛₜ₎-value threshold (http://pulm.bumc.bu.edu/aged).

In order to further characterize the effect of tobacco smoke on bronchial epithelial cells, we wanted to explore how genes' expression changes with amount of smoking. Pearson correlation calculations exploring the relationship between gene expression among current smokers and pack-years of smoking were computed. A less strict permutation analysis was performed to correct for multiple Pearson correlation calculations. The analysis is analogous to the procedure described above, except only the genes having a correlation with a p_{(correlation)}-value of less than 0.05 are permuted (2099 probesets instead of 9968 probesets). In addition, instead of permuting the class labels as described above, the pack-years were permuted (in a given permutation, gene expression values for a gene are assigned randomly to pack-year values). Using the less strict permutation analysis, the threshold was found to be 3.19*10⁻⁶, with 5 genes falling below this threshold. Supplementary Table 6 displays the top 51 genes with unadjusted p(_{(correlation)}-values below 0.0001. The p_{(correlation)}-value threshold found using the permutation based multiple comparison correction is more strict than the Bonferroni threshold of 2.4*10⁻⁵ because the correction is data dependent and pack-year values in our study are quite variable. The current smokers in our study have an average number of pack-years of 22, but there are 3 "outlier" current smokers with extremely high pack-year histories (>70 pack-years). These smokers with extremely high pack years underpin the linear fit and result in better correlations even for random permutations, and thus lead to a stricter multiple comparison correction threshold.

**Effects of Smoking Cessation:** There is relatively little information about how smoking cessation alters the effects of smoking on airways. Cough and sputum production decreases rapidly in smokers with bronchitis who cease to smoke(23). The accelerated decline in forced expiratory volume (FEV1), that characterizes smokers with COPD, reverts to an age appropriate decline of FEV1 when smoking is discontinued(24). However, the allelic loss in airway epithelial cells obtained at biopsy, changes relatively little in former smokers and the risk for developing lung cancer remains high for at least 20 years after smoking cessation(6).

Figure 6A shows a multidimensional scaling plot of never and current smokers according to the expression of the 97 genes that distinguish current smokers from never smokers. Figure 6B shows that former smokers who discontinued smoking less than 2 years prior to this study tend to cluster with current smokers, whereas former smokers who discontinued smoking for more than 2 years group more closely with never smokers. Hierarchical clustering of all 18 former smokers according to the expression of these same 97 genes also reveals 2 subgroups of former smokers, with the length of smoking cessation being the only clinical variable that was statistically different between the 2 subgroups (see Figure 11). Reversible genes were predominantly drug metabolizing and antioxidant genes.

There were 13 genes that did not return to normal levels in former smokers, even those who had discontinued smoking 20-30 year prior to testing (p <9*10⁻⁴; threshold determined by permutation analysis). These genes include a number of potential tumor suppressor genes, e.g. TU3A and CX3CL1, that are permanently decreased, and several putative oncogenes, e.g. CEACAM6 and HN1, which are permanently increased (see Figure 7). Three metallothionein genes remain decreased in former smokers. Metallothioneins have metal binding, detoxification and antioxidant properties and have been reported to affect cell proliferation and apoptosis(25). The metallothionein genes that remained abnormal in former smokers are located at 16q13, suggesting that this may represent a fragile site for DNA injury in smokers. The persistence of abnormal expression of select genes after smoking cessation may provide growth advantages to a subset of epithelial cells allowing for clonal expansion and perpetuation of these cells years after smoking had been discontinued. These permanent changes might explain the persistent risk of lung cancer in former smokers.

We performed an unsupervised hierarchical clustering analysis of all 18 former smokers according to the expression of the 97 genes differentially expressed between current and never smoker (Figure 11). In addition, a multidimensional scaling (MDS) plot was constructed of all samples according to the expression of these 97 genes (Figures 6A-6B). The MDS plot in Figure 6 was constructed from the raw expression data for the 97 genes across all the samples using orthogonal initialization and euclidean distance as the similarity metric. Principal component analysis using the same data yielded similar results. Hierarchical clustering of the genes and samples was performed using log transformed z-score normalized data using a Pearson correlation (uncentered) similarity metric and average linkage clustering using CLUSTER and TREEVIEW software programs obtained at http://rana.lbl.gov/EisenSoftware.htm. MDS and PCA were performed using Partek 5.0 software obtained at www.partek.com.

In order to identify genes irreversibly altered by cigarette smoking, we performed a t-test between former smokers (n=18) and never smokers (n=23) across the 97 genes that were considered differentially expressed between current and never smokers. A permutation analysis (as described above) was used to determine the p₍ₜ₋ₜₑₛₜ₎-value threshold of 9.8*10⁻⁴. Using this threshold, 15 of the 97 probesets were found to be significantly irreversible altered by cigarette smoking. In order to strengthen the argument that the 15 irreversibly altered probesets are related to smoking, the analysis was expanded to all 9968 genes. A t-test was performed between former and never smoker across all 9968 genes, and 44 genes were found to have a p₍ₜ₋ₜₑₛₜ₎-value threshold below 0.00098. While the permuted p₍ₜ₋ₜₑₛₜ₎ -value threshold for this extension of our t-test should have been computed across all 9968 genes, the former smokers are the smallest group in our study and thus we chose a less restrictive p₍ₜ₋ₜₑₛₜ₎-value threshold. Although there was about a 100-fold increase in the amount of genes analyzed there was only about a 3-fold increase in the number of genes found to be significantly different between never and former smokers. Therefore, most genes that are significantly different between never and former smokers are also significantly different between current and never smokers. Also, in addition to the 15 genes, 12 more genes had a p₍ₜ₋ₜₑₛₜ₎-value between current and never smokers of less than 0.001, and only 7 of the 44 genes had p₍ₜ₋ₜₑₛₜ) -values between current and never smokers of greater than 0.05 (Figures 19A-19B).

We have, for the first time, characterized the genes expressed, and by extrapolation, defined the functions of a specific set of epithelial cells from a complex organ across a broad cross section of normal individuals. Large airway epithelial cells appear to serve antioxidant, metabolizing, and host defense functions.

Cigarette smoking, a major cause of lung disease, induces xenobiotic and redox regulating genes as well as several oncogenes, and decreases expression of several tumor suppressor genes and genes that regulate airway inflammation. We also identified a subset of three smokers who respond differently to cigarette smoke, i.e. individuals who do not turn on the genes needed to deal with getting rid of the pollutants, i.e., their airway transcriptome expression pattern resembles that of a non-smoker, and these smokers are thus predisposed to the carcinogenic effects.

Finally, we have explored the reversibility of altered gene expression when smoking was discontinued. The expression level of smoking induced genes among former smokers began to resemble that of never smokers after two years of smoking cessation. Genes that reverted to normal within two years of cessation tended to serve metabolizing and antioxidant functions.

Several genes, including potential oncogenes and tumor suppressor genes, failed to revert to never smoker levels years after cessation of smoking. Without wishing to be bound by a theory, these later findings explain the continued risk for developing lung cancer many years after individuals have ceased to smoke. In addition, results from this study show that the airway gene expression profile in smokers serves as a biomarker for lung cancer.

### REFERENCES

**1.** Proctor, R. N. (2001) Nat. Rev. Cancer 1, 82-86.
2. Greenlee, R. T., Hill-Harmon, M. B., Murray, T. & Thun, M. (2001) CA Cancer J. Clin. 51, 15-36.
3. Hecht, S. S. (2003) Nat. Rev. Cancer 3, 733-744.
**4.** Anderson R & Smith B. (2003) National Vital Statistics Reports 52. 7-11.
**5.** Shields, P. G. (1999) Ann. Oncol. 10 Suppl 5, S7-11
**6.** Ebbert, J. O., Yang, P., Vachon, C. M., Vierkant, R. A., Cerhan, J. R., Folsom, A. R. & Sellers, T. A. (2003) J. Clin. Oncol. 21, 921-926.
**7.** Belinsky, S. A., Palmisano, W. A., Gilliland, F. D., Crooks, L. A., Divine, K. K., Winters, S. A., Grimes, M. J., Harms, H. J., Tellez, C. S., Smith, T. M. et al. (2002) Cancer Res. 62, 2370-2377.
**8.** Hackett, N. R., Heguy, A., Harvey, B. G., O'Connor, T. P., Luettich, K., Flieder, D. B., Kaplan, R. & Crystal, R. G. (2003) Am. J. Respir. Cell Mol. Biol. 29,331-43.
**9.** Gebel, S., Gerstmayer, B., Bosio, A., Haussmann, H. J., Van Miert, E. & Muller, T. (2004) Carcinogenesis. 25, 169-78.
**10**. Bhattacharjee, A., Richards, W. G., Staunton, J., Li, C., Monti, S., Vasa, P., Ladd, C., Beheshti, J., Bueno, R., Gillette, M. et al. (2001) Proc. Natl. Acad. Sci. U. S. A 98, 13790-13795.
11. Garber, M. E., Troyanskaya, O. G., Schluens, K., Petersen, S., Thaesler, Z., Pacyna-Gengelbach, M., van de, R. M., Rosen, G. D., Perou, C. M., Whyte, R. I. et al. (2001) Proc. Natl. Acad. Sci. U. S. A 98, 13784-13789.
12. Beer, D. G., Kardia, S. L., Huang, C. C., Giordano, T. J., Levin, A. M., Misek, D. E., Lin, L., Chen, G., Gharib, T. G., Thomas, D. G. et al. (2002) Nat. Med. 8, 816-824.
13. Miura, K., Bowman, E. D., Simon, R., Peng, A. C., Robles, A. I., Jones, R. T., Katagiri, T., He, P., Mizukami, H., Charboneau, L. et al (2002) Cancer Res. 62, 3244-3250.
14. Wistuba, I. I., Lam, S., Behrens, C., Virmani, A. K., Fong, K. M., LeRiche, J., Samet, J. M., Srivastava, S., Minna, J. D. & Gazdar, A. F. (1997) J. Natl. Cancer Inst. 89, 1366-1373.
15. Powell, C. A., Spira, A., Derti, A., DeLisi, C., Liu, G., Borczuk, A., Busch, S., Sahasrabudhe, S., Chen, Y., Sugarbaker, D. et al. (2003) Am. J. Respir. Cell Mol. Biol. 29, 157-162.
16. Zeeberg, B. R., Feng, W., Wang, G., Wang, M. D., Fojo, A. T., Sunshine, M., Narasimhan, S., Kane, D. W., Reinhold, W. C., Lababidi, S. et al. (2003) Genome Biol. 4, R28.
17. Rusznak, C., Mills, P. R., Devalia, J. L., Sapsford, R. J., Davies, R. J. & Lozewicz, S. (2000) Am. J. Respir. Cell Mol. Biol 23, 530-536.
18. Abrahamson, M., Alvarez-Fernandez, M. & Nathanson, C. M. (2003) Biochem. Soc. Symp. 179-199
19. Mongiat, M., Otto, J., Oldershaw, R., Ferrer, F., Sato, J. D. & Iozzo, R. V. (2001) J. Biol, Chem. 276,10263-10271.
20. Denis, G. V., Vaziri, C., Guo, N. & Faller, D. V. (2000) Cell Growth Differ. 11, 417-424.
21. Stewart, J. H. (2001) Cancer 91, 2476-2482
22. Doll, R., Peto, R., Wheatley, K., Gray, R. & Sutherland, I. (1994) BMJ 309, 901-911.
23. Kanner, R. E., Connett, J. E., Williams, D. E. & Buist, A. S. (1999) Am. J. Med. 106, 410-416.
24. Anthonisen, N. R., Connett, J. E., Kiley, J. P., Altose, M. D., Bailey, W. C., Buist, A. S., Conway, W. A., Jr., Enright, P. L., Kanner, R. E., O'Hara, P. et al. (1994) JAMA 272, 1497-1505.
25. Theocharis, S. E., Margeli, A. P. & Koutselinis, A. (2003) Int. JBiol. Markers 18, 162-169.

While this invention has been particularly shown and described with references to preferred embodiments thereof, it will be understood by those skilled in the art that various changes in form and details may be made therein without departing from the scope of the invention encompassed by the appended claims.

The following numbered paragraphs define particular embodiments of the present invention.
1. An airway transcriptome comprising a group consisting of genes encoding HLF; CYFIP2; MGLL; HSPA2; DKFZP586B2420; SLIT1; SLIT2; TU3A; MMP10; CCND2; CX3CL; MT1F; RNAHP; MT1X; MT1L; MT1G; PEC1; TNFSF13; GMDS; ZNF232; GALNT12; AP2B 1; HN1; ABCC 1; RAB11A; MSMB; MAFG; ABHD2; ANXA3; VMD2; FTH1; UGT1A3; TSPAN-1; CTGF; PDG; HTATIP2; CYP4F11; GCLM; ADH7; GCLC; UPK1B; TCN1 ; TRIM16; UGT1A9; UGTIA1; UGT1A6; NQ01; TXNRD1; PRDX1; ME1; PIR; TALDO1; GPX2; AKR1C3; AKR1C1; AKR1C-pseudo ; AKR1C2; ALDH3A1; CLDN10; TXN; TKT; CYP1B1; CBR1; AKR1B1; NET6; NUDT4; GALNT3; CEACAM6; AP1G1; CA12; FLJ20151; BCL2L13; SRPUL; FLJ13052; GALNT6; OASIS; MUC5B; S100P ; SDR1; PLA2G10; and DPYSL3.
2. An airway sub-transcriptome comprising mucin encoding genes of the airway transcriptome of paragraph 1.
3. The airway sub-transcriptome of paragraph 2, wherein the mucin encoding genes are selected from mucin 5 subtypes A, B, and C.
4. An airway sub-transcriptome comprising cell adhesion molecule encoding genes of the airway transcriptome of paragraph 1.
5. The airway sub-transcriptome of paragraph 4, wherein the cell adhesion molecule encoding genes are selected from carcinoembryonic antigen-related adhesion molecule 6 and claudin 10.
6. An airway sub-transcriptome comprising detoxification related molecule encoding genes of the airway transcriptome of paragraph 1.
7. The airway sub-transcriptome of paragraph 6, wherein the detoxification related molecule encoding genes are selected from cytochrome P450 subfamily I (dioxin-inducible) encoding genes and NADPH dehydrogenase encoding genes.
8. An airway sub-transcriptome comprising immunoregulatory molecule encoding genes of the airway transcriptome of paragraph 1.
9. The airway sub-transcriptome of paragraph 8, wherein the immunoregulatory molecule encoding genes are selected from cytokine subfamily D encoding genes.
10. An airway sub-transcriptome comprising metallothionein genes of the airway transcriptome of paragraph 1.
11. The airway sub-transcriptome of paragraph 10, wherein the metallothionein genes are selected from MTX G, X, and L encoding genes.
12. An airway sub-transcriptome comprising protooncogene encoding genes of the airway transcriptome of paragraph 1.
13. The airway sub-transcriptome of paragraph 12, wherein the protooncogene encoding genes are selected from RAB11A and CEACAM6.
14. An airway transcriptome comprising tumor suppressor encoding genes of the airway transcriptome of paragraph 1.
15. The airway sub-transcriptome of paragraph 14, wherein the tumor suppressor encoding genes are selected from MT1X, MT1L, MT1G, MT1F, SLIT1, and SLIT2.
16. A method of determining an individual at increased risk of developing a lung disease comprising taking a biological sample from the airways of an individual exposed to an airway pollutant or at risk of being exposed to an airway pollutant and analyzing the expression pattern of at least one gene of the airway transcriptome genes of paragraph 1, wherein deviation in the expression of one or more of the airway transcriptome genes as compared to the expression of the same at least one gene in a group of control individuals is indicative that the individual has an increased risk of developing a lung disease.
17. The method of paragraph 16, wherein the air pollutant is smoke from cigarette or a cigar and the lung disease is lung cancer.
18. The method of paragraph 17, wherein the lung cancer is selected from adenocarcinoma, squamous cell carcinoma, small cell carcinoma, large cell carcinoma, and benign neoplasms of the lung.
19. The method of paragraph 17 or 18, wherein one looks for expression of at least one gene from the substranscriptomes of paragraphs 2-15.
20. The method of paragraph 17 or 18, wherein the individual is a smoker and one looks at expression of at least one gene selected from the group consisting of subtranscriptomes encoding mucins, detoxification molecules and cell adhesion molecules, wherein lower expression of that at least one gene in the smoker than in a control group of corresponding smokers is indicative of an increased risk of developing lung cancer.
21. The method of paragraph 20, wherein lower expression of at least one gene in each of the subtranscriptomes encoding mucins, detoxification related molecules and cell adhesion molecules is indicative of an increased risk of developing lung cancer.
22. The method of paragraph 21, wherein lower expression of at least three genes in each of the subtranscriptomes encoding mucins, detoxification related molecules and cell adhesion molecules is indicative of an increased risk of developing lung cancer.
23. The method of paragraph 17 or 18, wherein the individual is a smoker and one looks at expression of at least one gene selected from the substranscriptomes encoding immunoregulatory molecules or metallothioneins, wherein higher expression of that at least one gene in the smoker than in a control group of corresponding smokers is indicative of an increased risk of developing lung cancer.
24. The method of paragraph 23, wherein higher expression of at least one gene in each of the subtranscriptomes encoding immunoregulatory molecules or metallothioneins is indicative of an increased risk of developing lung cancer.
25. The method of paragraph 24, wherein higher expression of at least three genes in each of the subtranscriptomes encoding immunoregulatory molecules or metallothioneins is indicative of an increased risk of developing lung cancer.
26. The method of paragraph 17 or 18, wherein the individual is a smoker and one looks at expression of at least one gene selected from the substranscriptomes encoding protooncogenes, wherein higher expression of that at least one gene in the smoker than in a control group of corresponding smokers is indicative of an increased risk of developing lung cancer.
27. The method of paragraph 26, wherein higher expression of at least one gene in each of the subtranscriptomes encoding protooncogenes is indicative of an increased risk of developing lung cancer.
28. The method of paragraph 17 or 18, wherein the individual is a smoker and one looks at expression of at least one gene selected from a subtranscriptomes encoding tumor suppressor genes, wherein lower expression of that at least one gene in the smoker than in a control group of corresponding smokers is indicative of an increased risk of developing lung cancer.
29. The method of paragraph 28, wherein lower expression of at least one gene in each of the subtranscriptomes encoding tumor suppressor genes is indicative of an increased risk of developing lung cancer.
30. A method of diagnosing predisposition of a smoker to lung disease comprising analyzing an expression pattern of one or more genes selected from the group consisting of AKR1C1, MSMB; TM4SF1; UPK1B; FLJ20152; SEC14L3; HT21; GALNT6; and AKR1C2 in a biological sample taken from the airways of the smoker, wherein a divergent expression pattern of one or more of these genes as compared to the expression pattern of these genes in group of control individuals is indicative of the predisposition of the individual to lung disease.
31. A method of diagnosing predisposition of a non-smoker to lung disease comprising analyzing an expression pattern of one or more genes selected from the group consisting of AKR1C1, MSMB; TM4SF1; UPK1B; FLJ20152; SEC14L3; HT21; GALNT6; and AKR1C2 in a biological sample taken from the airways of the non-smoker wherein a divergent expression pattern of one or more of these genes as compared to the expression pattern of these genes in group of control individuals is indicative of the predisposition of the individual to lung disease.
32. The method of paragraph 31, wherein the lung disease is lung cancer.
33. The method of paragraph 32, wherein the lung cancer is selected from adenocarcinoma, squamous cell carcinoma, small cell carcinoma, large cell carcinoma, and benign neoplasms of the lung.
34. The method of any of paragraphs 16-33, wherein the biological sample is a nucleic acid sample.
35. The method of paragraph 34, wherein the nucleic acid is mRNA.
36. The method of paragraphs 34 or 35, wherein the analysis is performed using a nucleic acid array.
37. The method of the paragraphs 16-33, wherein the biological sample is a protein obtained from epithelial tissue of an airway.
38. The method of paragraph 37, wherein the analysis is performed using antibodies directed against one or more of the proteins or antigenic fragment thereof encoded by the airway transcriptome of paragraph 1.
39. A method of diagnosing lung cancer in an individual which comprises looking for a deviation in expression in said individual of at least 10 genes selected from the group consisting of 208238_x_at-probeset; 216384_x_at-probeset; 217679_x_at-probeset; 216859_x_at-probeset; 211200_s_at-probeset; PDPK1; ADAM28; ACACB; ASMTL; ACVR2B; ADAT1; ALMS1; ANK3; ANK3; DARS; AFURS1; ATP8B1; ABCC1; BTF3; BRD4; CELSR2; CALM31 CAPZB; CAPZB1 CFLAR; CTSS; CD24; CBX3; C21orf106; C6orf111; C6orf62; CHC1; DCLRE1C; EML2; EMS1; EPHB6; EEF2; FGFR3; FLJ20288; FVT1; GGTLA4; GRP; GLUL; HDGF; Homo sapiens cDNA FLJ11452 fis, clone HEMBA1001435; Homo sapiens cDNA FLJ12005 fis, clone HEMBB1001565; Homo sapiens cDNA FLJ13721 fis, clone PLACE2000450; Homo sapiens cDNA FLJ14090 fis, clone MAMMA1000264; Homo sapiens cDNA FLJ14253 fis, clone OVARC1001376; Homo sapiens fetal thymus prothymosin alpha mRNA, complete cds Homo sapiens fetal thymus prothymosin alpha mRNA; Homo sapiens transcribed sequence with strong similarity to protein ref:NP_004726.1 (H. sapiens) leucine rich repeat (in FLII) interacting protein 1; Homo sapiens transcribed sequence with weak similarity to protein ref:NP_060312.1 (H. sapiens) hypothetical protein FLJ20489; Homo sapiens transcribed sequence with weak similarity to protein ref:NP_060312.1 (H. sapiens) hypothetical protein FLJ20489; 222282_at-probeset corresponding to Homo sapiens transcribed sequences; 215032_at-probeset corresponding to Homo sapiens transcribed sequences, 81811_at-probeset corresponding to Homo sapiens transcribed sequences; DKFZp547K1113; ET; FLJ10534; FLJ10743; FLJ13171; FLJ14639; FLJ14675; FLJ20195; FLJ20686; FLJ20700; CG005; CG005; MGC5384; IMP-2; INADL; INHBC; KIAA0379; KIAA0676; KIAA0779; KIAA1193; KTN1; KLF5; LRRFIP 1; MKRN4 ; MAN1C1; MVK; MUC20; MPZL1 ; MYO1A; MRLC2; NFATC3; ODAG; PARVA; PASK; PIK3C2B; PGF; PKP4; PRKX; PRKY; PTPRF; PTMA; PTMA; PHTF2; RAB14; ARHGEF6; RIPX; REC8L1; RIOK3; SEMA3F; SRRM21 MGC709071 SMT3H2; SLC28A3; SAT; SFRS111 SOX2; THOC2; TRIM51 USP7; USP9X; USH1C; AF020591; ZNF131; ZNF160; ZNF264; 217414_x_at- probeset;; 217232_x_at-probeset;; ATF3; ASXL2; ARF4L; APG5L; ATP6V0B; BAG1; BTG2; COMT; CTSZ; CGI-128; C14orf87; CLDN3; CYR61; CKAP1; DAF; DAF; DSIPI; DKFZP564G2022; DNAJB9; DDOST; DUSP1; DUSP6 ; DKC1; EGR1; EIF4EL3; EXT2; GMPPB; GSN; GUK1; HSPA8; Homo sapiens PRO2275 mRNA, complete cds; Homo sapiens transcribed sequence with strong similarity to protein ref:NP_006442.2, polyadenylate binding protein-interacting protein 1; HAX1; DKFZP434K046; IMAGE3455200; HYOU1; IDN3; JUNB; KRT8; KIAA0100; KIAA0102; APH-1A; LSM4; MAGED2; MRPS7 ; MOCS2; MNDA; NDUFA8; NNT; NFIL3; PWP1; NR4A2; NUDT4; ORMDL2; PDAP2; PPIH; PBX3; P4HA2; PPP1R15A; PRG11 P2RX4; SUI1; SUI1; SUI1; RAB5C; ARHB; RNASE4; RNH; RNPC4; SEC23B; SERPINA1; SH3GLB1; SLC35B1; SOX9; SOX9; STCH; SDHC; TINF2; TCF8; E2-EPF; FOS; JUN; ZFP36; ZNF500; and ZDHHC4, wherein said expression of those genes is compared to a control group, where the deviation of expression of those genes is indicative of the individual having lung cancer.
40. The method of paragraph 39, wherein one looks at the deviation in expression of at least 50 of the genes as compared to a control group, wherein the deviation of at least ten of the genes looked at as compared to the control group is indicative of the individual having lung cancer.
41. The method of paragraph 39, wherein one looks at the deviation in expression of at least 100 of the genes as compared to a control group, wherein the deviation of at least twenty-five of the genes looked at as compared to the control group is indicative of the individual having lung cancer.
42. The method of paragraph 39, wherein one looks at the deviation in expression of at least 150 of the genes as compared to a control group, wherein the deviation of at least twenty-five of the genes looked at as compared to the control group is indicative of the individual having lung cancer.
43. The method of paragraph 39, wherein one looks at the deviation in expression of at least 200 of the genes as compared to a control group, wherein the deviation of at least fifty of the genes looked at as compared to the control group is indicative of the individual having lung cancer.
44. A method of diagnosing lung cancer in an individual which comprises looking for an decrease in expression of at least 10 genes selected from the group consisting of 208238_ x_at-probeset; 216384_x_at-probeset; 217679_x_at-probeset; 216859_x_at-probeset; 211200_s_at-probeset; PDPK1; ADAM28; ACACB; ASMTL; ACVR2B; ADAT1; ALMS1; ANK3; ANK3; DARS; AFURS1; ATP8B1; ABCC1; BTF3; BRD4; CELSR2; CALM31 CAPZB; CAPZB1 CFLAR; CTSS; CD24; CBX3; C21orfl06 ; C6orfl11; C6orf62; CHCl; DCLRE1C; EML2; EMS1; EPHB6; EEF2; FGFR3; FLJ20288; FVT1; GGTLA4; GRP; GLUL; HDGF; Homo sapiens cDNA FLJ11452 fis, clone HEMBA1001435; Homo sapiens cDNA FLJ12005 fis, clone HEMBB1001565; Homo sapiens cDNA FLJ13721 fis, clone PLACE2000450; Homo sapiens cDNA FLJ14090 fis, clone MAMMA1000264; Homo sapiens cDNA FLJ14253 fis, clone OVARC1001376; Homo sapiens fetal thymus prothymosin alpha mRNA, complete cds; Homo sapiens transcribed sequence with strong similarity to protein ref:NP_004726.1 (H. sapiens) leucine rich repeat (in FLII) interacting protein 1; Homo sapiens transcribed sequence with weak similarity to protein ref:NP_060312.1 (H. sapiens) hypothetical protein FLJ20489; Homo sapiens transcribed sequence with weak similarity to protein ref:NP_060312.1 (H. sapiens) hypothetical protein FLJ20489; 222282_at-probeset corresponding to Homo sapiens transcribed sequences; 215032_at-probeset corresponding to Homo sapiens transcribed sequences, 81811_at-probeset corresponding to Homo sapiens transcribed sequences; DKFZp547K1113; ET; FLJ10534; FLJ10743; FLJ13171; FLJ14639; FLJ14675; FLJ20195; FLJ20686; FLJ20700; CG005; CG005; MGC5384; IMP-2; INADL; INHBC; KIAA0379; KIAA0676; KIAA0779; KIAA1193; KTN1; HLF5; LRRFIP1; MKRN4 ; MAN1Cl; MVK; MUC20; MPZL1; MYO1A; MRLC2; NFATC3; ODAG; PARVA; PASK; PIK3C2B; PGF; PKP4; PRKX; PRKY; PTPRF; PTMA; PTMA; PHTF2; RAB14; ARHGEF6; RIPX; REC8L1; RIOK3; SEMA3F; SRRM21 MGC709071 SMT3H2; SLC28A3; SAT; SFRS111 SOX2; THOC2; TRIM51 USP7; USP9X; USH1C; AF020591; ZNF131; ZNF160; andZNF264 as compared to a control group, wherein the decrease of at least five of the genes looked at as compared to the control group is indicative of the individual having lung cancer.
45. The method of paragraph 44, wherein one looks at the decrease in expression of at least 25 of the genes as compared to a control group, wherein the decrease at least ten of the genes looked at as compared to the control group is indicative of the individual having lung cancer.
46. The method of paragraph 44, wherein one looks at the decrease in expression of at least 50 of the genes as compared to a control group, wherein the decrease of at least twenty-five of the genes looked at as compared to the control group is indicative of the individual having lung cancer.
47. The method of paragraph 44, wherein one looks at the decrease in expression of at least 75 of the genes as compared to a control group, wherein the decrease of at least twenty-five of the genes looked at as compared to the control group is indicative of the individual having lung cancer.
48. The method of paragraph 44, wherein one looks at the decrease in expression of at least 100 of the genes as compared to a control group, wherein the decrease of at least twenty-five of the genes looked at as compared to the control group is indicative of the individual having lung cancer.
49. A method of diagnosing lung cancer in an individual which comprises looking for an increase in expression of at least 10 genes selected from the group consisting of 217414_x_at- probeset;; 217232_x_at-probeset;; ATF3; ASXL2; ARF4L; APG5L; ATP6V0B; BAG1; BTG2; COMT; CTSZ; CGI-128; C14orf87; CLDN3; CYR61; CKAP1; DAF; DAF; DSIPI; DKFZP564G2022; DNAJB9; DDOST; DUSP1; DUSP6 ; DKC1; EGR1; EIF4EL3; EXT2; GMPPB; GSN; GUK1; HSPA8; Homo sapiens PRO2275 mRNA, complete cds; Homo sapiens transcribed sequence with strong similarity to protein ref:NP_006442.2, polyadenylate binding protein-interacting protein 1; HAX1; DKFZP434K046; IMAGE3455200; HYOU1; IDN3; JUNB; KRT8; KIAA0100; KIAA0102; APH-1A; LSM4; MAGED2; MRPS7 ; MOCS2; MNDA; NDUFA8; NNT; NFIL3; PWP1; NR4A2; NUDT4; ORMDL2; PDAP2; PPIH; PBX3; P4HA2; PPP1R15A; PRG11 P2RX4; SUI1; SUI1; SUI1; RAB5C; ARHB; RNASE4; RNH; RNPC4; SEC23B; SERPINA1; SH3GLB1; SLC35B1; SOX9; SOX9; STCH; SDHC; TINF2; TCF8; E2-EPF; FOS; JUN; ZFP36; ZNF500; and ZDHHC4 as compared to a control group, wherein the increase of at least one of the at least five genes looked at as compared to the control group is indicative of the individual having lung cancer.
50. The method of paragraph 49, wherein one looks at the increase in expression of at least 25 of the genes as compared to a control group, wherein the increase of at least ten of the genes looked at as compared to is indicative of the individual having lung cancer.
51. The method of paragraph 49, wherein one looks at the increase in expression of at least 50 of the genes as compared to a control group, wherein the increase of at least ten of the genes looked at as compared to the control group is indicative of the individual having lung cancer.
52. The method of paragraph 49, wherein one looks at the increase in expression of at least 75 of the genes as compared to a control group, wherein the increase of at least twenty-five of the genes looked at as compared to the control group is indicative of the individual having lung cancer.

## Claims

1. A method of diagnosing a lung disease in an individual comprising examining a biological sample of nucleic acids obtained from epithelial cells of a part of the airway of the individual other than the lungs and analyzing the expression in said sample of at least one gene from a group of genes displaying deviation in expression in an individual with a lung disease as compared to the expression of the same at least one gene in a control, wherein deviation in expression of said at least one gene is indicative that the individual has a lung disease, wherein the lung disease is lung cancer, and wherein the epithelial cells of a part of the airway of the individual other than the lungs were obtained by buccal mucosal scraping or brushing airway surfaces.

2. The method of claim 1, wherein the individual has been exposed to an air pollutant.

3. The method of claim 1, wherein the lung cancer is selected from adenocarcinoma, squamous cell carcinoma, small cell carcinoma, and large cell carcinoma.

4. The method of claims 1-3, wherein expression of at least five genes is analyzed.

5. The method of claims 1-3, wherein expression of at least ten genes is analyzed.

6. The method of claims 1-3, wherein expression of at least twenty genes is analyzed.

7. The method of claims 1-3, wherein expression of at least fifty genes is analyzed.

8. The method of claims 1-7, wherein the individual is a smoker and expression of the at least one gene is analyzed, wherein lower expression of said at least one gene in the smoker compared to expression of said at least one gene in a control smoker is indicative of lung cancer.

9. The method of claims 1-7, wherein the individual is a smoker and expression of the at least one gene is analyzed, wherein higher expression of said at least one gene in the smoker than in a control smoker is indicative of lung cancer.

10. The method of claim 1, wherein the nucleic acid is mRNA.
